# NEW EUROPEAN PATENT SPECIFICATION

(11) **EP 1 220 684 B2**
(45) Date of publication and mention of the opposition decision: **14.07.2010**
(45) Mention of the grant of the patent: 19.07.2006
(21) Application number: 00965477.3
(22) Date of filing: 27.09.2000
(51) Int. Cl.: A61K 38/21, A61K 35/28, C12N 5/00, A61P 35/00, A61K 31/7088, A61K 38/19

(54) **METHODS RELATED TO IMMUNOSTIMULATORY NUCLEIC ACID-INDUCED INTERFERON**
VERFAHREN MITTELS DURCH NUKLEINSÄUREN INDUZIERTES IMMUNOSTIMULATORISCHES INTERFERON
METHODES CONCERNANT L'INTERFERON INDUIT PAR DES ACIDES NUCLEIQUES IMMUNOSTIMULATEURS

(30) Priority: 27.09.1999 US 156147 P
(43) Date of publication of application: 10.07.2002
(60) Divisional application: 05111935.2
(73) Proprietor: Coley Pharmaceutical Group, Inc., Wellesley, MA 02481 (US); UNIVERSITY OF IOWA RESEARCH FOUNDATION, Iowa City, IA 52242 (US); Coley Pharmaceutical GmbH, 40764 Langenfeld (DE)
(72) Inventor: HARTMANN, Gunther Abt. für Klinische Pharmakologie, 53105 Bonn (DE); BRATZLER, Robert L. Coley Pharma. Group, Inc., Wellesley, MA 02481 (US); KRIEG, Arthur Coley Pharmaceutical Group, Inc., Wellesley, MA 02481 (US)
(74) Representative: Jump, Timothy John Simon
(86) International application number: PCT/US2000/026527
(87) International publication number: WO 2001/022990

(56) References cited:
- EP-A- 0 855 184
- WO-A-00/06588
- WO-A-98/18810
- WO-A-98/33517
- WO-A-98/52581
- WO-A-99/51259
- HARTMANN G ET AL: "CPG DNA:A POTENT SIGNAL FOR GROWTH, ACTIVATION, AND MATURATION OF HUMAN DENDRITIC CELLS" PROCEEDINGS OF THE NATIONAL ACADEMY OF SCIENCES OF USA,US,NATIONAL ACADEMY OF SCIENCE. WASHINGTON, vol. 96, August 1999 (1999-08), pages 9305-9310, XP000919153 ISSN: 0027-8424
- CELLA MARINA ET AL: "Plasmacytoid monocytes migrate to inflamed lymph nodes and produce large amounts of type I interferon." NATURE MEDICINE, vol. 5, no. 8, August 1999 (1999-08), pages 919-923, XP002164016 ISSN: 1078-8956
- CELLA MARINA ET AL: "Maturation, activation, and protection of dendritic cells induced by double-stranded RNA." JOURNAL OF EXPERIMENTAL MEDICINE, vol. 189, no. 5, 1 March 1999 (1999-03-01), pages 821-829, XP002164017 ISSN: 0022-1007
- SIEGAL FREDERICK P ET AL: "The nature of the principal type 1 interferon-producing cells in human blood." SCIENCE (WASHINGTON D C), vol. 284, no. 5421, 11 June 1999 (1999-06-11), pages 1835-1837, XP002164018 ISSN: 0036-8075
- BARTHOLOME E J ET AL: "Interferon-beta induce the maturation of IL-12-deficient myeloid dendritic cells able to induce Th2 type cytokine secretion." JOURNAL OF INTERFERON AND CYTOKINE RESEARCH, vol. 19, no. SUPPL. 1, 1999, page S81 XP000990672 Meeting of the International Society for Interferon and Cytokine Research with the participation of the European Cytokine Society;Paris, France; September 5-9, 1999 ISSN: 1079-9907
- JPN. J. CANCER RES. vol. 83, November 1992, pages 1128 - 1131
- ANTISENSE & NUCLEIC ACID DRUG DEVELOPMENT vol. 6, 1996, pages 133 - 139

### SEQUENCE LISTING

<110> Coley Pharmaceutical Group, Inc. University of Iowa Research Foundation
<120> Methods Related to Immunostimulatory Nucleic Acid-Induced Interferon
<130> C1039/7044WO
<150> 60/156,147
   <151> 1999-09-29
<160> 165
<170> FastSEQ for Windows Version 3.0
<210> 1
   <211> 20
   <212> DNA
   <213> Artificial Sequence
<220>
   <223> Synthetic Oligonucleotide
<221> misc_feature
   <222> (1) ... (2)
   <223> Backbone has phosphorothioate linkages.

<221> misc_feature
   <222> (3)...(14)
   <223> Backbone has phosphodiester linkages.
<221> misc_feature
   <222> (15)... (19)
   <223> Backbone has phosphorothioate linkages.
<221> misc_feature
   <222> (20)...(20)
   <223> Backbone has phosphodiester linkages.
<400> 1
   ggggtcaacg ttgagggggg 20
<210> 2
   <211> 24
   <212> DNA
   <213> Artificial Sequence
<220>
   <223> Synthetic Oligonucleotide
<221> misc_feature
   <222> (1)...(24)
   <223> Backbone has phosphorothioate linkages.
<400> 2
   tcgtcgtttt gtcgttttgt cgtt 24
<210> 3
   <211> 20
   <212> DNA
   <213> Artificial Sequence
<220>
   <223> Synthetic Oligonucleotide
<221> misc_feature
   <222> (1)...(20)
   <223> Backbone has phosphorothioate linkages.
<400> 3
   ggggtcgtcg ttttgggggg 20
<210> 4
   <211> 24
   <212> DNA
   <213> Artificial Sequence
<220>
   <223> Synthetic Oligonucleotide
<221> misc_feature
   <222> (1)...(24)
   <223> Backbone has phosphorothioate linkages.
<400> 4
   tcgtcgtttt gtcgttttgg gggg 24
<210> 5
   <211> 20
   <212> DNA
   <213> Artificial Sequence
<220>
   <223> Synthetic Oligonucleotide
<221> misc_feature
   <222> (1)...(20)
   <223> Backbone has phosphorothioate linkages.
<400> 5
   ggggtcgacg tcgagggggg 20
<210> 6
   <211> 20
   <212> DNA
   <213> Artificial Sequence

<220>
   <223> Synthetic Oligonucleotide
<221> misc_feature
   <222> (1)..(20)
   <223> Backbone has phosphorothioate linkages.
<400> 6
   ggggtcatcg atgagggggg 20
<210> 7
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 7
   gggggacgat cgtcgggggg 20
<210> 8
   <211> 20
   <212> DNA
   <213> Artificial Sequence
<220>
   <223> Synthetic Oligonucleotide
<221> misc_feature
   <222> (1)...(20)
   <223> Backbone has phosphorothioate linkages.
<400> 8
   gggggtcgta cgacgggggg 20
<210> 9
   <211> 22
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(16)
   <223> Backbone has phosphodiester linkages.
<221> misc_feature
   <222> (17)...(21)
   <223> Backbone has phosphorothioate linkages.
<221> misc_feature
   <222> (22)...(22)
   <223> Backbone has phosphodiester linkages.
<400> 9
   gggggacgat atcgtcgggg gg 22

<210> 10
   <211> 22
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(16)
   <223> Backbone has phosphodiester linkages.
<221> misc_feature
   <222> (17)...(21)
   <223> Backbone has phosphorothioate linkages.
<221> misc_feature
   <222> (22)...(22)
   <223> Backbone has phosphodiester linkages.
<400> 10
   gggggacgac gtcgtcgggg gg 22
<210> 11
   <211> 22
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(16)
   <223> Backbone has phosphodiester linkages.
<221> misc_feature
   <222> (17)...(21)
   <223> Backbone has phosphorothioate linkages.
<221> misc_feature
   <222> (22)...(22)
   <223> Backbone has phosphodiester linkages.
<400> 11
   gggggacgag ctcgtcgggg gg 22
<210> 12
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.

<400> 12
   gggggacgta cgtcgggggg 20
<210> 13
   <211> 20
   <212> DNA
   <213> Artificial Sequence
<220>
   <223> Synthetic Oligonucleotide
<221> misc_feature
   <222> (1) ... (2)
   <223> Backbone has phosphorothioate linkages.
<221> misc_feature
   <222> (3)...(15)
   <223> Backbone has phosphodiester linkages.
<221> misc_feature
   <222> (16)...(19)
   <223> Backbone has phosphorothioate linkages.
<221> misc_feature
   <222> (20)...(20)
   <223> Backbone has phosphodiester linkages.
<400> 13
   gggggacgat cgttgggggg 20
<210> 14
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(15)
   <223> Backbone has phosphodiester linkages.
<221> misc_feature
   <222> (16)...(19)
   <223> Backbone has phosphorothioate linkages.
<221> misc_feature
   <222> (20)...(20)
   <223> Backbone has phosphodiester linkages.
<400> 14
   ggggaacgat cgtcgggggg 20
<210> 15
   <211> 21
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(15)
   <223> Backbone has phosphodiester linkages.
<221> misc_feature
   <222> (16)...(20)
   <223> Backbone has phosphorothioate linkages.

<221> misc_feature
   <222> (21)...(21)
   <223> Backbone has phosphodiester linkages.
<400> 15
   ggggggacga tcgtcggggg g 21
<210> 16
   <211> 21
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(15)
   <223> Backbone has phosphodiester linkages.
<221> misc_feature
   <222> (16)...(20)
   <223> Backbone has phosphorothioate linkages.
<221> misc_feature
   <222> (21)...(21)
   <223> Backbone has phosphodiester linkages.
<400> 16
   gggggacgat cgtcgggggg g 21
<210> 17
   <211> 21
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(15)
   <223> Backbone has phosphodiester linkages.
<221> misc_feature
   <222> (16) ..(20)
   <223> Backbone has phosphorothioate linkages.
<221> misc_feature
   <222> (21)...(21)
   <223> Backbone has phosphodiester linkages.
<400> 17
   gggggtcatc gatgaggggg g 21
<210> 18
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.

<221> misc_feature
   <222> (15)...(19)
   <223> Backbone has phosphorothioate linkages.
<221> misc_feature
   <222> (20)...(20)
   <223> Backbone has phosphodiester linkages.
<400> 18
   ggggtcgtcg acgagggggg 20
<210> 19
   <211> 22
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(16)
   <223> Backbone has phosphodiester linkages.
<221> misc_feature
   <222> (17)...(21)
   <223> Backbone has phosphorothioate linkages.
<221> misc_feature
   <222> (22)...(22)
   <223> Backbone has phosphodiester linkages.
<400> 19
   ggggtcgttc gaacgagggg gg 22
<210> 20
   <211> 22
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(16)
   <223> Backbone has phosphodiester linkages.
<221> misc_feature
   <222> (17)...(21)
   <223> Backbone has phosphorothioate linkages.
<221> misc_feature
   <222> (22)...(22)
   <223> Backbone has phosphodiester linkages.
<400> 20
   ggggacgttc gaacgtgggg gg 22
<210> 21
   <211> 22
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(16)
   <223> Backbone has phosphodiester linkages.

<221> misc_feature
   <222> (17) ..(21)
   <223> Backbone has phosphorothioate linkages.
<221> misc_feature
   <222> (22)...(22)
   <223> Backbone has phosphodiester linkages.
<400> 21
   ggggaacgac gtcgttgggg gg 22
<210> 22
   <211> 20
   <212> DNA
   <213> Artificial Sequence
<220>
   <223> Synthetic Olignucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 22
   ggggaacgta cgtcgggggg 20
<210> 23
   <211> 24
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(18)
   <223> Backbone has phosphodiester linkages.
<221> misc_feature
   <222> (19)...(23)
   <223> Backbone has phosphorothioate linkages.
<221> misc_feature
   <222> (24)...(24)
   <223> Backbone has phosphodiester linkages.
<400> 23
   ggggaacgta cgtacgttgg gggg 24
<210> 24
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.

<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 24
   ggggtcaccg gtgagggggg 20
<210> 25
   <211> 24
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(18)
   <223> Backbone has phosphodiester linkages.
<221> misc_feature
   <222> (19)...(23)
   <223> Backbone has phosphorothioate linkages.
<221> misc_feature
   <222> (24)... (24)
   <223> Backbone has phosphodiester linkages.
<400> 25
   ggggtcgacg tacgtcgagg gggg 24
<210> 26
   <211> 22
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(16)
   <223> Backbone has phosphodiester linkages.
<221> misc_feature
   <222> (17)...(21)
   <223> Backbone has phosphorothioate linkages.
<221> misc_feature
   <222> (22)...(22)
   <223> Backbone has phosphodiester linkages.
<400> 26
   ggggaccggt accggtgggg gg 22
<210> 27
   <211> 19
   <212> DNA
   <213> Artificial Sequence
<220>
   <223> Synthetic Oligonucleotide

<221> misc_feature
   <222> (1)...(2)
   <223> Backbone has phosphorothioate linkages.
<221> misc_feature
   <222> (3)...(13)
   <223> Backbone has phosphodiester linkages.
<221> misc_feature
   <222> (14)... (18)
   <223> Backbone has phosphorothioate linkages.
<221> misc_feature
   <222> (19)...(19)
   <223> Backbone has phosphodiester linkages.
<400> 27
   gggtcgacgt cgagggggg 19
<210> 28
   <211> 18
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(13)
   <223> Backbone has phosphodiester linkages.
<221> misc_feature
   <222> (14)...(18)
   <223> Backbone has phosphorothioate linkages.
<400> 28
   ggggtcgacg tcgagggg 18
<210> 29
   <211> 22
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(16)
   <223> Backbone has phosphodiester linkages.
<221> misc_feature
   <222> (17)...(21)
   <223> Backbone has phosphorothioate linkages.
<221> misc_feature
   <222> (22)...(22)
   <223> Backbone has phosphodiester linkages.
<400> 29
   ggggaacgtt aacgttgggg gg 22
<210> 30
   <211> 19
   <212> DNA
   <213> Artificial Sequence
<220>
   <223> Synthetic Oligonucleotide
<221> misc_feature
   <222> (1) ... (2)
   <223> Backbone has phosphorothioate linkages.

<221> misc_feature
   <222> (3)...(14)
   <223> Backbone has phosphodiester linkages.
<221> misc_feature
   <222> (15)...(18)
   <223> Backbone has phosphorothioate linkages.
<221> misc_feature
   <222> (19)...(19)
   <223> Backbone has phosphodiester linkages.
<400> 30
   ggggacgtcg acgtggggg 19
<210> 31
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 31
   gggggtcgtt cgttgggggg 20
<210> 32
   <211> 19
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(13)
   <223> Backbone has phosphodiester linkages.
<221> misc_feature
   <222> (14)...(18)
   <223> Backbone has phosphorothioate linkages.
<221> misc_feature
   <222> (19) ... (19)
   <223> Backbone has phosphodiester linkages.
<400> 32
   gggacgatcg tcggggggg 19
<210> 33
   <211> 20
   <212> DNA
   <213> 'Artificial Sequence
<220>
   <223> Synthetic Oligonucleotide

<221> misc_feature
   <222> (1)...(2)
   <223> Backbone has phosphorothioate linkages.
<221> misc_feature
   <222> (3)...(13)
   <223> Backbone has phosphodiester linkages.
<221> misc_feature
   <222> (14)...(19)
   <223> Backbone has phosphorothioate linkages.
<221> misc_feature
   <222> (20)...(20)
   <223> Backbone has phosphodiester linkages.
<400> 33
   gggtcgtcga cgaggggggg 20
<210> 34
   <211> 19
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(13)
   <223> Backbone has phosphodiester linkages.
<221> misc_feature
   <222> (14)...(18)
   <223> Backbone has phosphorothioate linkages.
<221> misc_feature
   <222> (19)...(19)
   <223> Backbone has phosphodiester linkages.
<400> 34
   ggtcgtcgac gaggggggg 19
<210> 35
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15) ..(19)
   <223> Backbone has phosphorothioate linkages.
<221> misc_feature
   <222> (20)...(20)
   <223> Backbone has phosphodiester linkages.
<400> 35
   ggggacgatc gtcggggggg 20
<210> 36
   <211> 27
   <212> DNA
   <213> Artificial Sequence

<220>
   <223> Synthetic Oligonucleotide
<221> misc_feature
   <222> (1)...(2)
   <223> Backbone has phosphorothioate linkages.
<221> misc_feature
   <222> (3)...(21)
   <223> Backbone has phosphodiester linkages.
<221> misc_feature
   <222> (22)...(26)
   <223> Backbone has phosphorothioate linkages.
<221> misc_feature
   <222> (27)... (27)
   <223> Backbone has phosphodiester linkages.
<400> 36
   ggggtcgacg tcgacgtcga ggggggg 27
<210> 37
   <211> 21
   <212> DNA
   <213> Artificial Sequence
<220>
   <223> Synthetic Oligonucleotide
<221> misc_feature
   <222> (1) ... (2)
   <223> Backbone has phosphorothioate linkages.
<221> misc_feature
   <222> (3)...(15)
   <223> Backbone has phosphodiester linkages.
<221> misc_feature
   <222> (16)...(20)
   <223> Backbone has phosphorothioate linkages.
<221> misc_feature
   <222> (21)...(21)
   <223> Backbone has phosphodiester linkages.
<400> 37
   ggggacgacg tcgtgggggg g 21
<210> 38
   <211> 8
   <212> DNA
   <213> Artificial Sequence
<220>
   <223> Synthetic Oligonucleotide
<400> 38
   aacgttct 8
<210> 39
   <211> 24
   <212> DNA
   <213> Artificial Sequence
<220>
   <223> Synthetic Oligonucleotide
<400> 39
   accatggacg aactgtttcc cctc 24
<210> 40
   <211> 24
   <212> DNA
   <213> Artificial Sequence
<220>
   <223> Synthetic Oligonucleotide

<400> 40
   accatggacg acctgtttcc cctc 24
<210> 41
   <211> 24
   <212> DNA
   <213> Artificial Sequence
<220>
   <223> Synthetic Oligonucleotide
<400> 41
   accatggacg agctgtttcc cctc 24
<210> 42
   <211> 24
   <212> DNA
   <213> Artificial Sequence
<220>
   <223> Synthetic Oligonucleotide
<400> 42
   accatggacg atctgtttcc cctc 24
<210> 43
   <211> 24
   <212> DNA
   <213> Artificial Sequence
<220>
   <223> Synthetic Oligonucleotide
<400> 43
   accatggacg gtctgtttcc cctc 24
<210> 44
   <211> 24
   <212> DNA
   <213> Artificial Sequence
<220>
   <223> Synthetic Oligonucleotide
<400> 44
   accatggacg tactgtttcc cctc 24
<210> 45
   <211> 24
   <212> DNA
   <213> Artificial Sequence
<220>
   <223> Synthetic Oligonucleotide
<400> 45
   accatggacg ttctgtttcc cctc 24
<210> 46
   <211> 18
   <212> DNA
   <213> Artificial Sequence
<220>
   <223> Synthetic Oligonucleotide
<400> 46
   agctatgacg ttccaagg 18
<210> 47
   <211> 20
   <212> DNA
   <213> Artificial Sequence
<220>
   <223> Synthetic Oligonucleotide
<400> 47
   ataggaggtc caacgttctc 20

<210> 48
   <211> 20
   <212> DNA
   <213> Artificial Sequence
<220>
   <223> Synthetic Oligonucleotide
<400> 48
   atcgactctc gaacgttctc 20
<210> 49
   <211> 20
   <212> DNA
   <213> Artificial Sequence
<220>
   <223> Synthetic Oligonucleotide
<400> 49
   atcgactctc gagcgttctc 20
<210> 50
   <211> 17
   <212> DNA
   <213> Artificial Sequence
<220>
   <223> Synthetic Oligonucleotide
<400> 50
   atgacgttcc tgacgtt 17
<210> 51
   <211> 20
   <212> DNA
   <213> Artificial Sequence
<220>
   <223> Synthetic Oligonucleotide
<400> 51
   atggaaggtc caacgttctc 20
<210> 52
   <211> 0
   <213> Artificial Sequence
<220>
   <223> Synthetic Oligonucleotide
<400> 52
   ATGGAAGGTCCAGCGTTCTC
<210> 53
   <211> 20
   <212> DNA
   <213> Artificial Sequence
<220>
   <223> Synthetic Oligonucleotide
<400> 53
   atggactctc cagcgttctc 20
<210> 54
   <211> 20
   <212> DNA
   <213> Artificial Sequence
<220>
   <223> Synthetic Oligonucleotide
<400> 54
   atggaggctc catcgttctc 20
<210> 55
   <211> 7
   <212> DNA
   <213> Artificial Sequence

<220>
   <223> Synthetic Oligonucleotide
<400> 55
   caacgtt 7
<210> 56
   <211> 15
   <212> DNA
   <213> Artificial Sequence
<220>
   <223> Synthetic Oligonucleotide
<400> 56
   cacgttgagg ggcat 15
<210> 57
   <211> 8
   <212> DNA
   <213> Artificial Sequence
<220>
   <223> Synthetic Oligonucleotide
<400> 57
   ccaacgtt 8
<210> 58
   <211> 20
   <212> DNA
   <213> Artificial Sequence
<220>
   <223> Synthetic Oligonucleotide
<400> 58
   gagaacgatg gaccttccat 20
<210> 59
   <211> 20
   <212> DNA
   <213> Artificial Sequence
<220>
   <223> Synthetic Oligonucleotide
<400> 59
   gagaacgctc cagcactgat 20
<210> 60
   <211> 20
   <212> DNA
   <213> Artificial Sequence
<220>
   <223> Synthetic Oligonucleotide
<400> 60
   gagaacgctc gaccttccat 20
<210> 61
   <211> 20
   <212> DNA
   <213> Artificial Sequence
<220>
   <223> Synthetic Oligonucleotide
<400> 61
   gagaacgctc gaccttcgat 20
<210> 62
   <211> 20
   <212> DNA
   <213> Artificial Sequence
<220>
   <223> Synthetic Oligonucleotide

<400> 62
   gagaacgctg gaccttccat 20
<210> 63
   <211> 15
   <212> DNA
   <213> Artificial Sequence
<220>
   <223> Synthetic Oligonucleotide
<400> 63
   gcatgacgtt gagct 15
<210> 64
   <211> 21
   <212> DNA
   <213> Artificial Sequence
<220>
   <223> Synthetic Oligonucleotide
<400> 64
   gcgtgcgttg tcgttgtcgt t 21
<210> 65
   <211> 15
   <212> DNA
   <213> Artificial Sequence
<220>
   <223> Synthetic Oligonucleotide
<400> 65
   gctagacgtt agcgt 15
<210> 66
   <211> 15
   <212> DNA
   <213> Artificial Sequence
<220>
   <223> Synthetic Oligonucleotide
<400> 66
   gctagacgtt agtgt 15
<210> 67
   <211> 15
   <212> DNA
   <213> Artificial Sequence
<220>
   <223> Synthetic Oligonucleotide
<400> 67
   gctagatgtt agcgt 15
<210> 68
   <211> 19
   <212> DNA
   <213> Artificial Sequence
<220>
   <223> Synthetic Oligonucleotide
<400> 68
   ggggtcaacg ttgacgggg 19
<210> 69
   <211> 19
   <212> DNA
   <213> Artificial Sequence
<220>
   <223> Synthetic Oligonucleotide

<400> 69
   ggggtcagtc gtgacgggg 19
<210> 70
   <211> 6
   <212> DNA
   <213> Artificial Sequence
<220>
   <223> Synthetic Oligonucleotide
<221> misc_difference
   <222> (5)...(5)
   <223> y = t/u or c
<400> 70
   gtcgyt 6
<210> 71
   <211> 8
   <212> DNA
   <213> Artificial Sequence
<220>
   <223> Synthetic Oligonucleotide
<400> 71
   tcaacgtc 8
<210> 72
   <211> 8
   <212> DNA
   <213> Artificial Sequence
<220>
   <223> Synthetic Oligonucleotide
<400> 72
   tcaacgtt 8
<210> 73
   <211> 8
   <212> DNA
   <213> Artificial Sequence
<220>
   <223> Synthetic Oligonucleotide
<400> 73
   tcagcgct 8
<210> 74
   <211> 12
   <212> DNA
   <213> Artificial Sequence
<220>
   <223> Synthetic Oligonucleotide
<400> 74
   tcagcgtgcg cc 12
<210> 75
   <211> 8
   <212> DNA
   <213> Artificial Sequence
<220>
   <223> Synthetic Oligonucleotide
<400> 75
   tcatcgat 8
<210> 76
   <211> 20
   <212> DNA
   <213> Artificial Sequence
<220>
   <223> Synthetic Oligonucleotide

<400> 76
   tccacgacgt tttcgacgtt 20
<210> 77
   <211> 20
   <212> DNA
   <213> Artificial Sequence
<220>
   <223> Synthetic Oligonucleotide
<400> 77
   tccataacgt tcctgatgct 20
<210> 78
   <211> 20
   <212> DNA
   <213> Artificial Sequence
<220>
   <223> Synthetic Oligonucleotide
<400> 78
   tccatagcgt tcctagcgtt 20
<210> 79
   <211> 20
   <212> DNA
   <213> Artificial Sequence
<220>
   <223> Synthetic Oligonucleotide
<400> 79
   tccatcacgt gcctgatgct 20
<210> 80
   <211> 20
   <212> DNA
   <213> Artificial Sequence
<220>
   <223> Synthetic Oligonucleotide
<400> 80
   tccatgacgg tcctgatgct 20
<210> 81
   <211> 20
   <212> DNA
   <213> Artificial Sequence
<220>
   <223> Synthetic Oligonucleotide
<400> 81
   tccatgacgt ccctgatgct 20
<210> 82
   <211> 20
   <212> DNA
   <213> Artificial Sequence
<220>
   <223> Synthetic Oligonucleotide
<400> 82
   tccatgacgt gcctgatgct 20
<210> 83
   <211> 20
   <212> DNA
   <213> Artificial Sequence
<220>
   <223> Synthetic Oligonucleotide

<400> 83
   tccatgacgt tcctgacgtt 20
<210> 84
   <211> 20
   <212> DNA
   <213> Artificial Sequence
<220>
   <223> Synthetic Oligonucleotide
<400> 84
   tccatgacgt tcctgatgct 20
<210> 85
   <211> 20
   <212> DNA
   <213> Artificial Sequence
<220>
   <223> Synthetic Oligonucleotide
<400> 85
   tccatgccgg tcctgatgct 20
<210> 86
   <211> 20
   <212> DNA
   <213> Artificial Sequence
<220>
   <223> Synthetic Oligonucleotide
<400> 86
   tccatgcgtg cgtgcgtttt 20
<210> 87
   <211> 20
   <212> DNA
   <213> Artificial Sequence
<220>
   <223> Synthetic Oligonucleotide
<400> 87
   tccatgcgtt gcgttgcgtt 20
<210> 88
   <211> 20
   <212> DNA
   <213> Artificial Sequence
<220>
   <223> Synthetic Oligonucleotide
<400> 88
   tccatggcgg tcctgatgct 20
<210> 89
   <211> 20
   <212> DNA
   <213> Artificial Sequence
<220>
   <223> Synthetic Oligonucleotide
<400> 89
   tccatgtcga tcctgatgct 20
<210> 90
   <211> 20
   <212> DNA
   <213> Artificial Sequence
<220>
   <223> Synthetic Oligonucleotide
<400> 90
   tccatgtcgc tcctgatgct 20

<210> 91
   <211> 20
   <212> DNA
   <213> Artificial Sequence
<220>
   <223> Synthetic Oligonucleotide
<400> 91
   tccatgtcgg tcctgacgca 20
<210> 92
   <211> 20
   <212> DNA
   <213> Artificial Sequence
<220>
   <223> Synthetic Oligonucleotide
<400> 92
   tccatgtcgg tcctgatgct 20
<210> 93
   <211> 20
   <212> DNA
   <213> Artificial Sequence
<220>
   <223> Synthetic Oligonucleotide
<400> 93
   tccatgtcgg tcctgctgat 20
<210> 94
   <211> 20
   <212> DNA
   <213> Artificial Sequence
<220>
   <223> Synthetic Oligonucleotide
<400> 94
   tccatgtcgt ccctgatgct 20
<210> 95
   <211> 20
   <212> DNA
   <213> Artificial Sequence
<220>
   <223> Synthetic Oligonucleotide
<400> 95
   tccatgtcgt tcctgtcgtt 20
<210> 96
   <211> 20
   <212> DNA
   <213> Artificial Sequence
<220>
   <223> Synthetic Oligonucleotide
<400> 96
   tccatgtcgt ttttgtcgtt 20
<210> 97
   <211> 19
   <212> DNA
   <213> Artificial Sequence
<220>
   <223> Synthetic Oligonucleotide
<400> 97
   tcctgacgtt cctgacgtt 19
<210> 98
   <211> 19
   <212> DNA
   <213> Artificial Sequence

<220>
   <223> Synthetic Oligonucleotide
<400> 98
   tcctgtcgtt cctgtcgtt 19
<210> 99
   <211> 20
   <212> DNA
   <213> Artificial Sequence
<220>
   <223> Synthetic Oligonucleotide
<400> 99
   tcctgtcgtt ccttgtcgtt 20
<210> 100
   <211> 20
   <212> DNA
   <213> Artificial Sequence
<220>
   <223> Synthetic Oligonucleotide
<400> 100
   tcctgtcgtt ttttgtcgtt 20
<210> 101
   <211> 20
   <212> DNA
   <213> Artificial Sequence
<220>
   <223> Synthetic Oligonucleotide
<400> 101
   tccttgtcgt tcctgtcgtt 20
<210> 102
   <211> 21
   <212> DNA
   <213> Artificial Sequence
<220>
   <223> Synthetic oligonucleotide
<400> 102
   tcgtcgctgt ctccccttct t 21
<210> 103
   <211> 21
   <212> DNA
   <213> Artificial Sequence
<220>
   <223> Synthetic Oligonucleotide
<400> 103
   tcgtcgctgt ctgcccttct t 21
<210> 104
   <211> 21
   <212> DNA
   <213> Artificial Sequence
<220>
   <223> Synthetic Oligonucleotide
<400> 104
   tcgtcgctgt tgtcgtttct t 21
<210> 105
   <211> 14
   <212> DNA
   <213> Artificial Sequence
<220>
   <223> Synthetic Oligonucleotide

<400> 105
   tcgtcgtcgt cgtt 14
<210> 106
   <211> 20
   <212> DNA
   <213> Artificial Sequence
<220>
   <223> Synthetic Oligonucleotide
<400> 106
   tcgtcgttgt cgttgtcgtt 20
<210> 107
   <211> 22
   <212> DNA
   <213> Artificial Sequence
<220>
   <223> Synthetic Oligonucleotide
<400> 107
   tcgtcgttgt cgttttgtcg tt 22
<210> 108
   <211> 24
   <212> DNA
   <213> Artificial Sequence
<220>
   <223> Synthetic Oligonucleotide
<400> 108
   tcgtcgtttt gtcgttttgt cgtt 24
<210> 109
   <211> 17
   <212> DNA
   <213> Artificial Sequence
<220>
   <223> Synthetic Oligonucleotide
<400> 109
   tctcccagcg ggcgcat 17
<210> 110
   <211> 18
   <212> DNA
   <213> Artificial Sequence
<220>
   <223> Synthetic Oligonucleotide
<400> 110
   tctcccagcg tgcgccat 18
<210> 111
   <211> 8
   <212> DNA
   <213> Artificial Sequence
<220>
   <223> Synthetic Oligonucleotide
<400> 111
   tcttcgaa 8
<210> 112
   <211> 8
   <212> DNA
   <213> Artificial Sequence
<220>
   <223> Synthetic Oligonucleotide
<400> 112
   tcttcgat 8

<210> 113
   <211> 13
   <212> DNA
   <213> Artificial Sequence
<220>
   <223> Synthetic Oligonucleotide
<400> 113
   tgtcgttgtc gtt 13
<210> 114
   <211> 19
   <212> DNA
   <213> Artificial Sequence
<220>
   <223> Synthetic Oligonucleotide
<400> 114
   tgtcgttgtc gttgtcgtt 19
<210> 115
   <211> 25
   <212> DNA
   <213> Artificial Sequence
<220>
   <223> Synthetic Oligonucleotide
<400> 115
   tgtcgttgtc gttgtcgttg tcgtt 25
<210> 116
   <211> 21
   <212> DNA
   <213> Artificial Sequence
<220>
   <223> Synthetic Oligonucleotide
<400> 116
   tgtcgtttgt cgtttgtcgt t 21
<210> 117
   <211> 7
   <212> DNA
   <213> Artificial Sequence
<220>
   <223> Synthetic Oligonucleotide
<221> misc_difference
   <222> (6) ... (6)
   <223> y = t/u or c
<400> 117
   tgtcgyt 7
<210> 118
   <211> 20
   <212> DNA
   <213> Artificial Sequence
<220>
   <223> Synthetic Oligonucleotide
<400> 118
   atggaaggtc caaggggctc 20
<210> 119
   <211> 20
   <212> DNA
   <213> Artificial Sequence
<220>
   <223> Synthetic Oligonucleotide
<400> 119
   atggaaggtc cagggggctc 20

<210> 120
   <211> 20
   <212> DNA
   <213> Artificial Sequence
<220>
   <223> Synthetic Oligonucleotide
<400> 120
   atggaaggtc cggggttctc 20
<210> 121
   <211> 20
   <212> DNA
   <213> Artificial Sequence
<220>
   <223> Synthetic Oligonucleotide
<400> 121
   atggactctc cggggttctc 20
<210> 122
   <211> 20
   <212> DNA
   <213> Artificial Sequence
<220>
   <223> Synthetic Oligonucleotide
<400> 122
   atggactctg gagggggctc 20
<210> 123
   <211> 20
   <212> DNA
   <213> Artificial Sequence
<220>
   <223> Synthetic Oligonucleotide
<400> 123
   atggactctg gaggggtctc 20
<210> 124
   <211> 20
   <212> DNA
   <213> Artificial Sequence
<220>
   <223> Synthetic Oligonucleotide
<400> 124
   atggactctg gggggttctc 20
<210> 125
   <211> 20
   <212> DNA
   <213> Artificial Sequence
<220>
   <223> Synthetic Oligonucleotide
<400> 125
   atggaggctc catggggctc 20
<210> 126
   <211> 20
   <212> DNA
   <213> Artificial Sequence
<220>
   <223> Synthetic Oligonucleotide
<400> 126
   gagaaggggc cagcactgat 20

<210> 127
   <211> 20
   <212> DNA
   <213> Artificial Sequence
<220>
   <223> Synthetic Oligonucleotide
<400> 127
   gagaaggggg gaccttccat 20
<210> 128
   <211> 20
   <212> DNA
   <213> Artificial Sequence
<220>
   <223> Synthetic Oligonucleotide
<400> 128
   gagaaggggg gaccttggat 20
<210> 129
   <211> 15
   <212> DNA
   <213> Artificial Sequence
<220>
   <223> Synthetic Oligonucleotide
<400> 129
   gcatgagggg gagct 15
<210> 130
   <211> 14
   <212> DNA
   <213> Artificial Sequence
<220>
   <223> Synthetic Oligonucleotide
<400> 130
   gctagaggga gtgt 14
<210> 131
   <211> 15
   <212> DNA
   <213> Artificial Sequence
<220>
   <223> Synthetic Oligonucleotide
<400> 131
   gctagagggg agggt 15
<210> 132
   <211> 15
   <212> DNA
   <213> Artificial Sequence
<220>
   <223> Synthetic Oligonucleotide
<400> 132
   gctagatgtt agggg 15
<210> 133
   <211> 20
   <212> DNA
   <213> Artificial Sequence
<220>
   <223> Synthetic Oligonucleotide
<400> 133
   gggggacgat cgtcgggggg 20
<210> 134
   <211> 20
   <212> DNA
   <213> Artificial Sequence

<220>
   <223> Synthetic Oligonucleotide
<400> 134
   gggggggggg gggggggggg 20
<210> 135
   <211> 20
   <212> DNA
   <213> Artificial Sequence
<220>
   <223> Synthetic Oligonucleotide
<400> 135
   ggggtcaacg ttgagggggg 20
<210> 136
   <211> 20
   <212> DNA
   <213> Artificial Sequence
<220>
   <223> Synthetic Oligonucleotide
<400> 136
   ggggtcgacg tcgagggggg 20
<210> 137
   <211> 20
   <212> DNA
   <213> Artificial Sequence
<220>
   <223> Synthetic Oligonucleotide
<400> 137
   tccatcgggg gcctgatgct 20
<210> 138
   <211> 20
   <212> DNA
   <213> Artificial Sequence
<220>
   <223> Synthetic Olignucleotide
<400> 138
   tccatgaggg gcctgatgct 20
<210> 139
   <211> 20
   <212> DNA
   <213> Artificial Sequence
<220>
   <223> Synthetic Oligonucleotide
<400> 139
   tccatgcggg tggggatgct 20
<210> 140
   <211> 20
   <212> DNA
   <213> Artificial Sequence
<220>
   <223> Synthetic Oligonucleotide
<400> 140
   tccatggggg tcctgatgct 20
<210> 141
   <211> 20
   <212> DNA
   <213> Artificial Sequence
<220>
   <223> Synthetic Oligonucleotide

<400> 141
   tccatggggt ccctgatgct 20
<210> 142
   <211> 20
   <212> DNA
   <213> Artificial Sequence
<220>
   <223> Synthetic Oligonucleotide
<400> 142
   tccatggggt gcctgatgct 20
<210> 143
   <211> 20
   <212> DNA
   <213> Artificial Sequence
<220>
   <223> Synthetic Oligonucleotide
<400> 143
   tccatggggt tcctgatgct 20
<210> 144
   <211> 20
   <212> DNA
   <213> Artificial Sequence
<220>
   <223> Synthetic Oligonucleotide
<400> 144
   tccatgtggg gcctgatgct 20
<210> 145
   <211> 20
   <212> DNA
   <213> Artificial Sequence
<220>
   <223> Synthetic Oligonucleotide
<400> 145
   tccatgtggg gcctgctgat 20
<210> 146
   <211> 20
   <212> DNA
   <213> Artificial Sequence
<220>
   <223> Synthetic Oligonucleotide
<400> 146
   tccatgtggg tggggatgct 20
<210> 147
   <211> 24
   <212> DNA
   <213> Artificial Sequence
<220>
   <223> Synthetic Oligonucleotide
<221> misc_feature
   <222> (1)...(24)
   <223> Backbone has phosphorothioate linkages.
<400> 147
   tcgtcgtttt gtcgttttgt cgtt 24
<210> 148
   <211> 21
   <212> DNA
   <213> Artificial Sequence
<220>
   <223> Synthetic Oligonucleotide

<221> misc_feature
   <222> (1) ... (2)
   <223> Backbone has phosphorothioate linkages.
<221> misc_feature
   <222> (3)...(14)
   <223> Backbone has phosodiester linkages.
<221> misc_feature
   <222> (15)...(20)
   <223> Backbone has phosphorothioate linkages.
<221> misc_feature
   <222> (21)...(21)
   <223> Backbone has phosodiester linkages.
<221> misc_difference
   <222> (2)...(2)
   <223> m = a or c
<221> misc_difference
   <222> (18)...(18)
   <223> m = a or c
<400> 148
   gmggtcaacg ttgagggmgg g 21
<210> 149
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 149
   ggggagttcg ttgagggggg 20
<210> 150
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.

<221> misc_feature
   <222> (20)...(20)
   <223> Backbone has phosphodiester linkages.

<400> 150
   gggggagcat gctcgggggg 20
<210> 151
   <211> 20
   <212> DNA
   <213> Artificial Sequence
<220>
   <223> Synthetic Oligonucleotide
<221> misc_feature
   <222> (1) ... (2)
   <223> Backbone has phosphorothioate linkages.
<221> misc_feature
   <222> (3)...(14)
   <223> Backbone has phosphodiester linkages.
<221> misc_feature
   <222> (15)... (19)
   <223> Backbone has phosphorothioate linkages.
<221> misc_feature
   <222> (20) ..(20)
   <223> Backbone has phosphodiester linkages.
<400> 151
   ggggtcaagc ttgagggggg 20
<210> 152
   <211> 20
   <212> DNA
   <213> Artificial Sequence
<220>
   <223> Synthetic Oligonucleotide
<221> misc_feature
   <222> (1)..(20)
   <223> Backbone has phosphorothioate linkages.
<400> 152
   ggggacgtcg acgtgggggg 20
<210> 153
   <211> 22
   <212> DNA
   <213> Artificial Sequence
<220>
   <223> Synthetic Oligonucleotide
<221> misc_feature
   <222> (1)...(22)
   <223> Backbone has phosphorothioate linkages.
<400> 153
   ggggtcgttc gaacgagggg gg 22
<210> 154
   <211> 22
   <212> DNA
   <213> Artificial Sequence
<220>
   <223> Synthetic Oligonucleotide
<221> misc_feature
   <222> (1)...(22)
   <223> Backbone has phosphorothioate linkages.
<400> 154
   ggggacgttc gaacgtgggg gg 22

<210> 155
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphorothioate linkages.
<400> 155
   gggggagcat gctggggggg 20
<210> 156
   <211> 21
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(15)
   <223> Backbone has phosphodiester linkages.
<221> misc_feature
   <222> (16)...(20)
   <223> Backbone has phosphorothioate linkages.
<221> misc_feature
   <222> (21)...(21)
   <223> Backbone has phosphodiester linkages.
<400> 156
   gggggtcaac gttgaggggg g 21
<210> 157
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 157
   gggggatgat tgttgggggg 20

<210> 158
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<221> modified_base
   <222> (7)...(7)
   <223> n = 5- methylcytidine
<221> modified_base
   <222> (10)...(10)
   <223> n = 5-methylcytidine
<400> 158
   gggggangan tgttgggggg 20
<210> 159
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 159
   gggggagcta gcttgggggg 20
<210> 160
   <211> 20
   <212> DNA
   <213> Artificial Sequence
<220>
   <223> Synthetic Oligonucleotide
<221> misc_feature
   <222> (1) ... (2)
   <223> Backbone has phosphorothioate linkages.
<221> misc_feature
   <222> (3)...(14)
   <223> Backbone has phosphodiester linkages.

<221> misc_feature
   <222> (15)...(19)
   <223> Backbone has phosphorothioate linkages.
<221> misc_feature
   <222> (20)...(20)
   <223> Backbone has phosphodiester linkages.
<400> 160
   gggtcgtcgt cgtggggggg 20
<210> 161
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 161
   ggggacgtcg tcgtgggggg 20
<210> 162
   <211> 20
   <212> DNA
   <213> Artificial Sequence
<220>
   <223> Synthetic Oligonucleotide
<221> misc_feature
   <222> (1)...(2)
   <223> Backbone has phosphorothioate linkages.
<221> misc_feature
   <222> (3)...(14)
   <223> Backbone has phosphodiester linkages.
<221> misc_feature
   <222> (15)...(19)
   <223> Backbone has phosphorothioate linkages.
<221> misc_feature
   <222> (20)...(20)
   <223> Backbone has phosphodiester linkages.
<400> 162
   ggggaaccgc ggttgggggg 20
<210> 163
   <211> 45
   <212> DNA
   <213> Artificial Sequence
<220>
   <223> Synthetic Oligonucleotide
<400> 163
   accgatgacg tcgccggtga cggcaccacg acggccaccg tgctg 45
<210> 164
   <211> 30
   <212> DNA
   <213> Artificial Sequence
<220>
   <223> Synthetic Oligonucleotide
<400> 164
   accgatgacg tcgccggtga cggcaccacg 30
<210> 165
   <211> 30
   <212> DNA
   <213> Artificial Sequence
<220>
   <223> Synthetic Oligonucleotide
<400> 165
   gggggggggg ggaacgttgg gggggggggg 30

## Description

### Background of the Invention

Human interferon alpha (IFN-α), also known as leukocyte interferon and α interferon, comprises a family of extracellular signaling proteins with antiviral, antiproliferative, and immunomodulatory activities. The first type of interferon to be identified and commercialized, IFN-α remains the most widely used interferon for clinical applications.

IFN-α is a member of the family of Type I interferons, which also includes IFN-β, omega (leukocyte (II)) interferon and tau (trophoblast) interferon. Omega and tau interferons are not clinically used. IFN-β, also known as fibroblast interferon, is well characterized but less utilized than IFN-α in the clinic. Fibroblasts are the predominant cellular producers of IFN-β. IFN-β has been approved in the United States for the treatment of relapsing forms of multiple sclerosis. Interferon gamma (IFN-γ), also known as gamma interferon, is the only known type II interferon. IFN-γ is produced by activated T lymphocytes and plays an important role in the establishment of a Th1 immune response. Its therapeutic use is limited. In the United States, human IFN-γ has been approved for reducing the frequency and severity of infections with chronic granulomatous disease.

IFN-α itself represents a family of more than a dozen related, homologous proteins (isoforms, Table 1), each encoded by a unique gene and each exhibiting a unique activity profile. The activities of the different α interferon species on viruses can vary as much as twenty-fold or more.

IFN-α products in clinical use are recombinant proteins or highly purified natural proteins of a single isoform. Recombinant IFN-α has been approved for use in the treatment of a variety of tumors and viral diseases (Table 2).

Until recently, B lymphocytes were believed to be the predominant producers of IFN-α. Recently a new cell type has been identified in the peripheral blood as the major source of Type I interferon production. These previously unidentified "natural interferon producing cells" (IPC) had been described for many years as a rare CD4⁺/MHC class II⁺ population (1:1000 within peripheral blood mononuclear cells (PBMC)) capable of synthesizing extremely large amounts of type I IFN upon viral infection. Cella M et al. Nat Med 5:919 (1999); Galy A et al. Blood 95:128 (2000); Siegal FP et al. Science 284:1835 (1999). After isolation of IPCs from the peripheral blood, IL-3 is required for survival of this cell type.

**Table 1. Family of Human IFN-α**

| | |
|---|---|
| IFN-αA | (IFN-α2a) |
| IFN-α2 | (IFN-α2b) |
| IFN-α4b | (IFN-α4) |
| IFN-αB2 | (IFN-α8) |
| IFN-αC | (IFN-α10) |
| IFN-αD | (IFN-α1) |
| IFN-αF | (IFN-α21) |
| IFN-αG | (IFN-α5) |
| IFN-αH2 | (IFN-α14) |
| IFN-αI | (IFN-α17) |
| IFN-αJ1 | (IFN-α7) |
| IFN-αK | (IFN-α6) |
| IFN-αM1 | |
| IFN-αN | |
| IFN-αWA | (IFN-α16) |

**Table 2. Current Clinical Approval of IFN-α**

| Approved in the United States | Approved Outside the United States |
|---|---|
| Chronic hepatitis B | Multiple myeloma |
| Chronic hepatitis C | Renal cell carcinoma |
| Hairy cell leukemia | Bladder cell carcinoma |
| Cutaneous T-cell leukemia | Colon carcinoma |
| Chronic myeloid leukemia | Cervical dysplasia |
| Non-Hodgkin's lymphoma | Laryngeal papillomatosis |
| Adjuvant therapy for malignant melanoma | |
| Kaposi's Sarcoma (AIDS-related) | |
| Condylomata acuminata (venereal warts) | |

Dendritic cells (DC) are thought to play a key role in the priming of immune responses against neoantigens. Banchereau J et al., Nature 392:245 (1998). Recent evidence suggests the presence of several distinct DC subtypes in human peripheral blood. Zhong RK et al. J Immunol 163:1254 (1999). These subtypes of DC include myeloid DC (mDC) and plasmacytoid DC (pDC, also known as DC2 cells). Precursor dendritic cells contain two subsets, a CD 11c⁺/CD123^{+/-} population (precursor of mDC) and a CD1 1c⁻/CD123⁺⁺ population (precursor of pDC). The latter has recently attracted major attention since it was reported to be identical with the natural type I IFN producing cell (IPC). O'Doherty U et al. J Exp Med 178:1067 (1993); Grouard G et al. J Exp Med 185:1101 (1997); Thomas R et al. J Immunol 153:4016 (1994). Upon maturation this cell type develops characteristic features of DC. O'Doherty U et al. J Exp Med 178:1067 (1993); Thomas R et al. J Immunol 153:4016 (1994); Galy A et al. Blood 95:128 (2000); Chehimi J et al. Immunology 68:488. (1989).

The frequency of IPCs in PBMC in normal individuals varies between 0.2 and 0.6 percent. They are characterized by the absence of lineage markers CD3 (T cells), CD 14 (monocytes), CD19 (B cells) and CD56 (NK cells), by the absence of CD11c, and by their expression of CD4, CD123 (IL-3 receptor α, IL-3Rα) and MHC class II. Grouard G et al. J Exp Med 185:1101-11 (1997); Rissoan M-C et al. Science 283:1183-6 (1999); Siegal FP et al. Science 284:1835-7 (1999); Cella M et al. Nat Med 5:919-23 (1999). Morphologically IPCs resemble lymphocytes. IPCs can be isolated from PBMC by a combination of magnetic bead activated cell sorting (MACS) and fluorescence-activated cell sorting (flow cytometry, FACS). Without addition of IL-3, most of the IPCs die within 3 days of cell culture. Infection of IPCs with herpes simplex virus (HSV, Siegal FP et al. Science 284:1835-7 (1999)) or influenza virus (Cella M et al. Nat Med 5:919-23 (1999)) leads to production of large amounts of type I interferons as measured by a bioassay (protection of fibroblasts against vesicular stomatitis virus).

Aside from its role in carrying the genetic code, DNA has recently been shown to function as a signaling molecule (Krieg AM, 1998, Biodrugs). The immune systems of higher eukaryotes appear to have evolved a mechanism to detect prokaryotic nucleic acids based on their content of unmethylated CpG dinucleotides in particular base contexts. Krieg AM et al. Nature 374:546-9 (1995). Unmethylated CpG dinucleotides are common in bacterial DNA, but are underrepresented ("CpG suppression") and are methylated in vertebrate DNA. Bird AP *Trends in Genetics* 3:342 (1987). DNA containing these unmethylated CpG dinucleotides in immune stimulatory base contexts ("CpG motifs") triggers humoral immunity by inducing B cell activation, resistance to activation-induced apoptosis, and IL-6 and IgM secretion. Krieg AM et al. Nature 374:546-9 (1995); Yi AK et al. J Immunol 157:5394 (1996); and Klinman D et al. Proc Natl Acad Sci USA 93:2879 (1996). Such CpG DNA also directly activates monocytes and macrophage to secrete Th1-like cytokines. Ballas ZK et al. J Immunol 157:1840 (1996); Cowdery JS et al. J Immunol 156:4570 (1996); and Halpern MD et al. Cell Immunol 167:72 (1996). This leads to the activation of natural killer (NK) cell lytic activity and IFN-γ secretion. Ballas ZK et al. J Immunol 157:1840 (1996); Cowdery JS et al. J Immunol 156:4570 (1996); and Chace JH Clin Immunol Immunopath 84:185-93 (1997).

Yamamoto et al. reported in 1988 their findings that a nucleic acid fraction, designated MY-1, extracted from *Mycobacterium bovis* (BCG) induced type I interferon in vitro. Yamamoto S et al. Jpn J Cancer Res 79:866-73 (1988). Subsequently Tokunaga et al. subsequently synthesized a panel of 45-mer oligonucleotides with sequence present in cDNA encoding three randomly selected known BCG proteins and found that one sequence, BCG-A4, was a strong inducer of type I IFN in mouse spleen cell suspensions. Tokunaga T et al. Microbiol Immunol 36:55-66 (1992). A 5' 30-mer fragment, BCG-A4a, was reported to be as potent an inducer of IFN as the intact 45-mer BCG-A4.
BCG-A4 ACCGATGACGTCGCCGGTGACGGCACCACGACGGCCACCGTGCTG (SEQ ID NO:163)
BCG-A4a ACCGATGACGTCGCCGGTGACGGCACCACG (SEQ ID NO:164)
These workers went on to report that all oligonucleotides that induced IFN included a hexamer palindromic sequence GACGTC (present in BCG-A4 and BCG-A4a), AGCGCT, and AACGTT, but not ACCGGT. Yamamoto S et al. J Immunol 148:4072-6 (1992). Kimura et al. then found that among 30-mer phosophodiester oligodeoxynucleotides (ODNs) containing the hexamer palindrome AACGTT and oligoA, oligoC, oligoT, and oligoG ends, the latter (GGGGGGGGGGGGAACGTTGGGGGGGGGGGG; SEQ ID NO:165) was the strongest inducer of type I IFN in mouse spleen cell suspensions. Kimura Y et al. J Biochem (Tokyo) 116:991-4 (1994).

Recently it was surprisingly discovered that CpG ODN sequences with the strongest activity on human B cells did not induce detectable levels of type I IFN in PBMC. Hartmann G et al. J Immunol 164:1617-24 (2000).

### Summary of the Invention

It was discovered according to the invention that certain immunostimulatory nucleic acids (ISNAs) are especially suited as single agents to promote both survival and stimulation of IPCs. It was also discovered according to the invention that certain ISNAs obviate the requirement of IL-3 for IPC survival and the requirement of viral infection for IPC activation.

In addition, it was surprisingly discovered according to the invention that certain CpG ISNA induce the production of large amounts of type I IFN but have minimal effects activating B cells, while certain other CpG ISNA strongly activate human B cells and IPCs but have minimal effects inducing type I IFN. Surprisingly, it was discovered that the CpG ISNA that are strong inducers of type I IFN do not necessarily contain a hexamer palindrome GACGTC, AGCGCT, or AACGTT described by Yamamoto and colleagues. Yamamoto S et al. J Immunol 148:4072-6 (1992).

These discoveries open avenues for the use of ISNA, and especially certain CpG ISNA, as a therapeutic agent for clinical applications which call for the use of IFN-α. Clinical strategies comprise local and systemic *in vivo* administration of ISNA as well as *ex vivo* strategies in which *in vitro* ISNA-activated isolated IPCs are reinfused into the patient locally or systemically. These therapeutic strategies include the combination with other growth factors (IL-3, GM-CSF, flt3-ligand, etc.) as well as with other stimuli (superantigens, viral products). CpG ISNA of the invention that are inducers of type I IFN also allow the *in vitro* production of natural interferons using a permanent cell line derived from IPCs. Since natural IFN-α is a family of more than a dozen separate gene products, the individual products of which have unique activity profiles, the clinical use of natural interferon may be preferable compared to recombinant IFN-α derived from a single recombinant IFN-α gene.

It was also surprisingly discovered according to the invention that type I IFN activates a subset of T lymphocytes called γδ T cells. In addition, it was further discovered according to the invention that CpG ODN that are strong inducers of type I IFN, but not CpG ODN that are strong activators of B cells and pDCs without being strong inducers of type I IFN, can activate γδ T cells present within a population of peripheral blood mononuclear cells (PBMCs). Without meaning to be held to a particular theory, it appears likely that type I IFN-inducing CpG ODN can activate γδ T cells present within the PDMC by their ability to induce secretion of type I IFN by IPCs also present in the PBMC.

In addition to the ability to activate γδ T cells, it was also surprisingly discovered according to the invention that type I IFN-inducing CpG ODN, but not CpG ODN that are strong activators of B cells and pDCs without being strong inducers of type I IFN, can enhance proliferation of antigen-activated γδ T cells present within a population of PBMCs. In particular, proliferation is enhanced in connection with the presence of specific nonpeptide antigen, for example, the phosphoantigen isopentenyl pyrophosphate (IPP).

It was also surprisingly discovered according to the invention that certain CpG ODN in combination with IPP synergistically induce the production of IFN-γ and perforin in γδ T cells.

In another surprising discovery according to the invention, it was found that type I IFN-inducing CpG ODN, but not CpG ODN that are strong activators of B cells and pDCs without being strong inducers of type I IFN, can block CD40-stimulated IL-12 production in PBMC. It was surprisingly found, in addition, that CpG ODN that are strong activators of B cells and pDCs without being strong inducers of type I IFN had the opposite effect, i.e., these ODN actually enhanced CD40-stimulated IL-12 production in PBMC.

It was further discovered according to the invention that that CpG ODN that are strong activators of B cells and pDCs without being strong inducers of type I IFN are better promoters of antigen-specific priming and recall of human cytotoxic T lymphocytes (CTLs) than are CpG ODN that are potent inducers of type I IFN.

According to one aspect of the invention, an improvement is provided for therapies involving administration of IFN-α to subjects. The improvement involves co-administering an effective amount of an isolated ISNA. In one embodiment, the IFN-α is to be administered at the clinically established effective dose for IFN-α alone. In another embodiment, the IFN-α is to be administered at a dosage below the clinically established effective dose for IFN-α alone. The IFN-α also can be administered at the maximum tolerated dose for IFN-α in the absence of the oligonucleotide. In other embodiments, the IFN-α is to be administered at 20 percent below, 30 percent below, 40 percent below, or even 50 percent below the maximum tolerated dose of IFN-α or the clinically established effective dose for IFN-α alone.

The invention, in one aspect, therefore relates to the use of an isolated nucleic acid for the manufacture of a medicament for use in the treatment of a proliferative disorder or viral infection which calls for IFN-α treatment, wherein said nucleic acid has a sequence selected from the groups consisting of:

| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, and |
| ggGGACGACGTCGTGgggggG | ODN 2336 | SEQ ID NO:37, |

wherein each lower case letter represents phosphorothioate linkage and each upper case letter indicates phosphodiester linkage.

In certain more preferred embodiments the ISNA has a sequence corresponding to ggGGGACGAGCTCGTCgggggG (ODN 2247; SEQ ID NO:11), ggGGGACGATCGTCGgggggG (ODN 2255; SEQ ID NO:16), ggGGACGTTCGAACGTgggggG (ODN 2295; SEQ ID NO:20), ggGGTCGACGTCGACGTCGAGgggggG (ODN 2334; SEQ ID NO:36), or ggGGACGACGTCGTGgggggG (ODN 2336; SEQ ID NO:37), wherein each lower case letter represents phosphorothioate linkage and each upper case letter indicates phosphodiester linkage.

In one embodiment, the improvement further involves co-administering granulocyte-monocyte colony-stimulating factor (GM-CSF) to the subject.

In one embodiment, the subject has a proliferative disorder such as hairy cell leukemia, chronic myelogenous leukemia, cutaneous T-cell leukemia, multiple myeloma, follicular lymphoma, malignant melanoma, squamous cell carcinoma, AIDS-related Kaposi's sarcoma, renal cell carcinoma, prostate carcinoma, bladder cell carcinoma, cervical dysplasia, and colon carcinoma. In another embodiment the subject has a viral infection such as hepatitis B, hepatitis C, condyloma acuminatum, human immunodeficiency virus, herpes, cytomegalovirus, Epstein-Barr virus, and papillomavirus.

According to another aspect of the invention, the treatment involves supplementing IFN-α treatment of a subject. This aspect of the invention involves administering to a subject in need of IFN-α treatment an effective amount of IFN-α and an ISNA of the invention. The IFN-α doses, ISNAs, concurrent therapy, and conditions calling for treatment with IFN-α according to this aspect of the invention are the same as those described above.

According to another aspect of the invention, the treatment involves treating a subject to activate IPCs of the subject. This involves isolating IPCs from the subject in need of such treatment, culturing the isolated IPCs *in vitro,* contacting the IPCs *in vitro* with an effective amount of an isolated ISNA, and returning the contacted cells to the subject. The cells can also be contacted *in vitro* with a growth factor or with a cytokine. In one embodiment, the method further involves contacting the IPC cells *in vitro* with IL-3 or GM-CSF. In another embodiment, the cells are cultured *in vitro* in the absence of IL-3 and/or GM-CSF. The ISNAs and conditions calling for treatment with IFN-α according to this aspect of the invention are as described above.

According to another aspect of the invention, the treatment involves increasing the efficacy of IFN-α treatment of a subject. This involves administering to a subject in need of treatment with IFN-α a pharmaceutical composition including IFN-α and co-administering to the subject a pharmaceutical composition including an ISNA in an amount which, together with the administered IFN-α, is an effective IFN-α treatment. The efficacy of the IFN-α treatment is greater than the efficacy of administering the same amount of IFN-α in the absence of co-administering the ISNA. The ISNAs and conditions calling for treatment with IFN-α according to this aspect of the invention are as described above. In one embodiment, the pharmaceutical compositions are administered locally.

According to another aspect of the invention, the treatment involves decreasing the dose of IFN-α needed for effective treatment of a subject. This involves administering to a subject in need of treatment with IFN-α a pharmaceutical composition comprising IFN-α and co-administering to the subject a pharmaceutical composition including an ISNA. The amount of administered IFN-α is less than an amount of IFN-α required to achieve the same therapeutic benefit in the absence of co-administering the ISNA. In certain embodiments, the amount of administered IFN-α is at least 20 percent, at least 30 percent, at least 40 percent, or even at least 50 percent below the amount of IFN-α required in the absence of coadministering the immunostimulatory nucleic acid. The pharmaceutical composition including the ISNA can be administered locally. The ISNAs and conditions calling for treatment with IFN-α according to this aspect of the invention are as described above.

According to another aspect of the invention, the treatment involves preventing an IFN-α treatment-related side effect in a subject receiving or in need of treatment with IFN-α. This involves administering to a subject in need of the treatment an IFN-α pharmaceutical composition and a pharmaceutical composition comprising an immunostimulatory nucleic acid in an amount which, together with the administered IFN-α, is an effective IFN-α treatment. The IFN-α treatment-related side effect is reduced in comparison to the side effect when IFN-α is administered in the absence of co-administering ISNA. The IFN-α treatment-related side effect may be systemic. The IFN-α treatment-related side effect prevented by the method can include any one of flu-like syndrome, fever, headache, chills, myalgia, fatigue, anorexia, nausea, vomiting, diarrhea, and depression. The pharmaceutical composition including the ISNA can be administered locally. The ISNAs and conditions calling for treatment with IFN-α according to this aspect of the invention are as described above.

According to another aspect of the invention, the treatment involves enhancing the efficacy of IFN-α treatment in a subject in need of such treatment. This involves administering to a subject in need of such treatment an effective amount of a pharmaceutical composition containing IFN-α for treating the condition, isolating natural IFN-producing cells from a donor, contacting the isolated IFN-producing cells *ex vivo* with an amount of an ISNA effective for inducing the IFN-producing cells to release IFN-α, and administering the contacted cells to the subject. The donor can be, but does not have to be, the subject. The treatment further can comprise contacting the isolated cells with an antigen. The administration of the cells can be accomplished in any manner suitable for the purposes of the method, and can include local injection. The local injection can be via a blood vessel supplying a target tissue. The blood vessel can be selected from, among others, hepatic artery, portal vein, celiac artery, and splenic artery. The ISNAs and conditions calling for treatment with IFN-α according to this aspect of the invention are as described above.

According to another aspect of the invention, the treatment involves supporting survival of IPCs *in vitro.* This involves isolating such cells from a subject, culturing the cells in a sterile medium suitable for tissue culture, and contacting the cells *in vitro* with an amount of ISNA effective to support the growth of the cells in the absence of IL-3. In a preferred embodiment the cells can be precursor type 2 dendritic cells. The culture conditions also can be selected to be free of IL-3 and/or free of GM-CSF, or they can include IL-3, GM-CSF, or other growth factors and cytokines. The ISNAs, including oligonucleotides, sequences, modifications and the like according to this aspect of the invention, are as described above.

According to another aspect of the invention, the treatment involves stimulating isolated IPCs *in vitro.* This involves isolating such cells from a subject, culturing the cells in a sterile medium suitable for tissue culture, and contacting the cells *in vitro* with an amount of ISNA effective to induce secretion of at least one type I interferon or the expression of CD 80. The culture conditions can be in the presence or absence of interleukin-3, GM-CSF, or other growth factors and cytokines. The IPCs can be precursor type 2 dendritic cells. The ISNAs, including oligonucleotides, sequences, modifications and the like according to this aspect of the invention, are as described above.

According to another aspect of the invention, the treatment involves stimulating the production of an array of at least 3, 4, 5, 6, 7 or even 8 or more interferon sub-types. This involves contacting IFN-producing cells with an ISNA. The cells may or may not be isolated. The contacting may be *in vivo* or *in vitro.* The ISNAs, including oligonucleotides, sequences, modifications and the like according to this aspect of the invention, are as described herein.

According to another aspect of the invention, a method is provided for inhibiting IL-12 production. The method involves contacting IL-12-producing cells, in the presence of interferon-producing cells under conditions in which the IL-12-producing cells normally produce IL-12, with an immunostimulatory nucleic acid in an amount effective for inducing secretion of type I interferon.

In another aspect the invention provides an isolated nucleic acid having a sequence selected from the group which includes:

| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO: 13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO: 16 |
| ggGGGTCATCGATGAgggggG | ODN 2260 | SEQ ID NO: 17 |
| ggGGTCGTCGACGAgggggG | ODN 2293 | SEQ ID NO: 18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, and |
| ggGGACGACGTCGTGgggggG | ODN 2336 | SEQ ID NO:37, |

wherein each lower case letter represents phosphorothioate linkage and each upper case letter indicates phosphodiester linkage.

In yet another aspect the invention provides a pharmaceutical composition containing an isolated nucleic acid having a sequence selected from the group which includes:

| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, and |
| ggGGACGACGTCGTGgggggG | ODN 2336 | SEQ ID NO:37, |

wherein each lower case letter represents phosphorothioate linkage and each upper case letter indicates phosphodiester linkage, plus a pharmaceutically acceptable carrier. In some embodiments the pharmaceutical composition also contains IFN-α.

According to another aspect of the invention, an interferon composition for administration to a subject is provided. The composition includes interferon in a container for administration to a subject. The amount of the interferon in the container is at least about 10 percent less than the maximum tolerated dose (MTD). Preferably the amount of interferon in the container is at least about 20 percent below the MTD, at least 30 percent below the MTD, at least 40 percent below the MTD, or even at least 50 percent below the MTD. The container also can include an ISNA.

In still another aspect of the invention, kits for administration of interferon and an ISNA to a subject are provided. The kits include a container containing a composition which includes IFN-α and instructions for administering the interferon to a subject in need of such treatment in an amount which is at least about 10 percent less than the MTD, 20 percent less than the MTD, 30 percent less than the MTD, 40 percent less than the MTD, or 50 percent less than the MTD. The kit can include, in the same container or in a separate container, an ISNA. The kit also can include instructions for treating a subject with a condition susceptible to treatment with IFN-α.

These and other aspects of the invention are described in greater detail below.

### Brief Description of the Figures

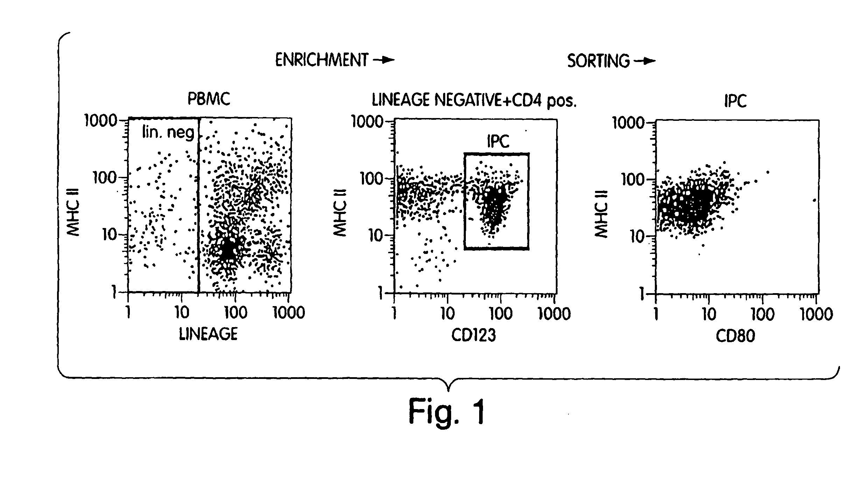

Figure 1 depicts FACS analyses of cell populations during the isolation and characterization of IPCs performed with magnetic beads and flow cytometry. From left to right are shown: selection of lin-/MHC class II+ cells from PBMCs; further selection of CD123+/ MHC class II+ cells from lin-/CD4+/MHC class II+ cells; and characterization of freshly isolated lin-/CD4+/MHC class II+/CD123+ IPCs as CD80-.

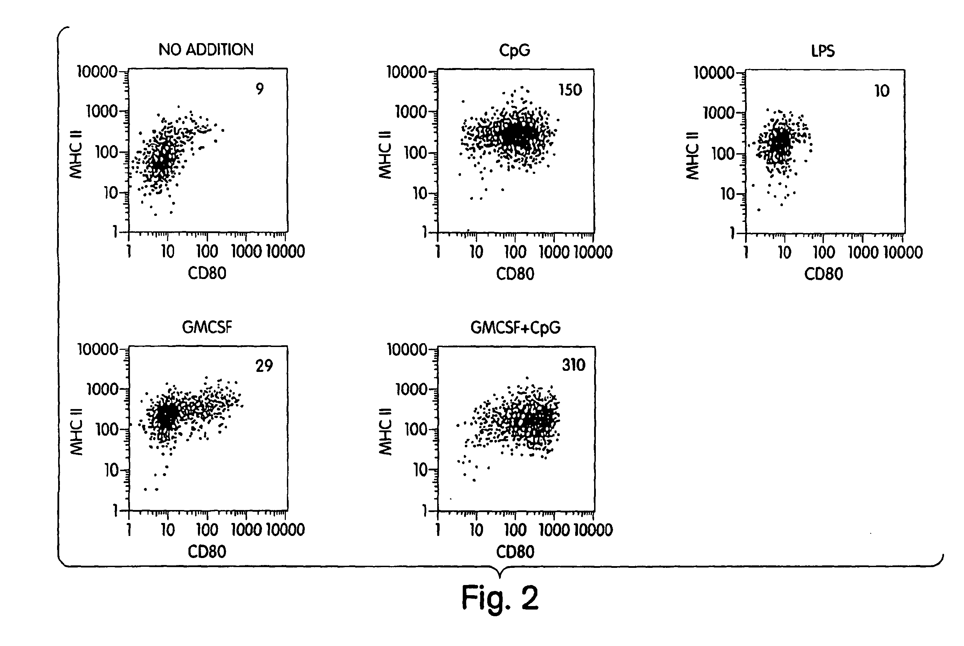

Figure 2 depicts FACS analyses of survival and activation (CD80) of freshly isolated IPCs after incubation for two days in the presence of selected growth factors and stimuli. Growth factor (GM-CSF) and/or stimulus (CpG oligonucleotide or LPS) for each panel: top left, none; top middle, CpG oligonucleotide; top right, LPS; bottom left, GM-CSF; and bottom middle, GM-CSF plus CpG oligonucleotide. The number in the upper right corner of each panel is the mean fluorescence intensity (MFI) for CD80. Results are representative of five independent experiments.

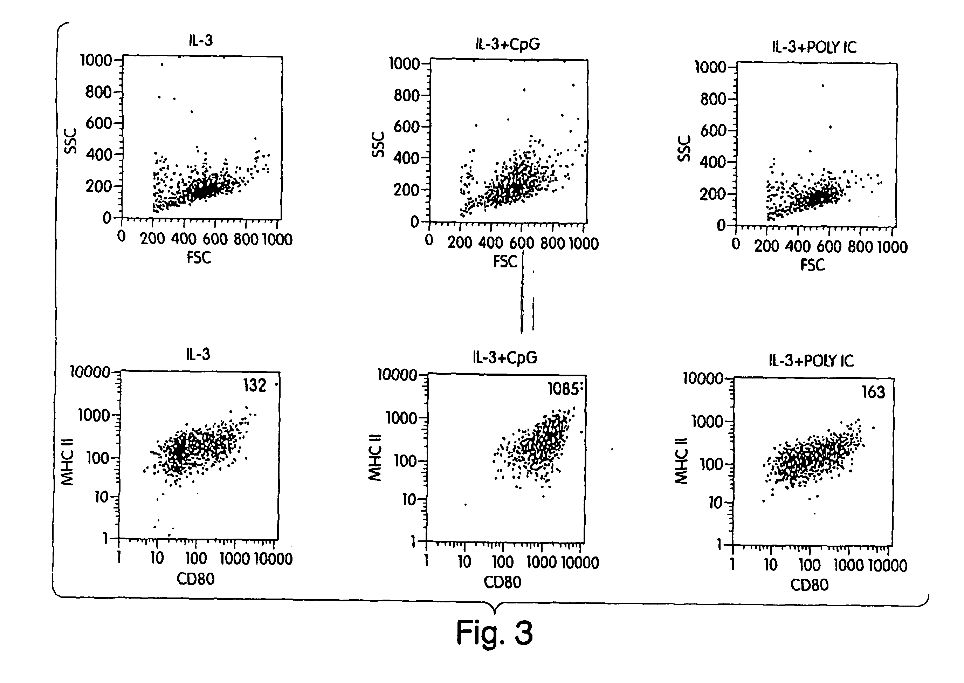

Figure 3 depicts FACS analyses showing the different effects CpG and poly IC have on the survival and activation of freshly isolated IPCs. All cells were cultured for three days in the presence of IL-3. Cells were then cultured for an additional 24 hours with the addition of: nothing (left panels); CpG (middle panels); or poly IC (right panels). MFI for CD80 is shown in the top right of each of the bottom panels. Results are representative of three independent experiments.

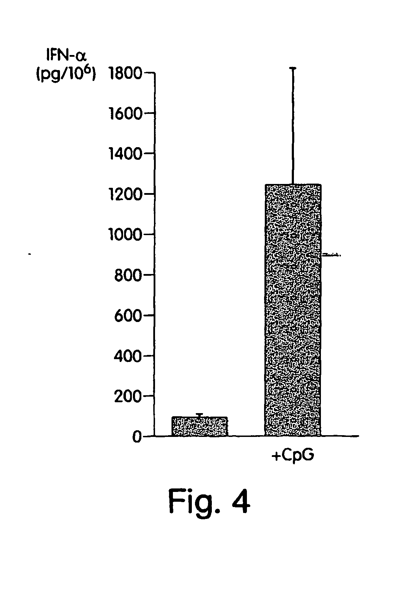

Figure 4 is a graph depicting the concentration of IFN-α (determined by IFN-α-specific ELISA) present in the supernatant of IPCs cultured for two days in the presence of IL-3 and GM-CSF, either with CpG oligonucleotide (solid bar) or without CpG oligonucleotide (open bar). Results are representative of three independent experiments.

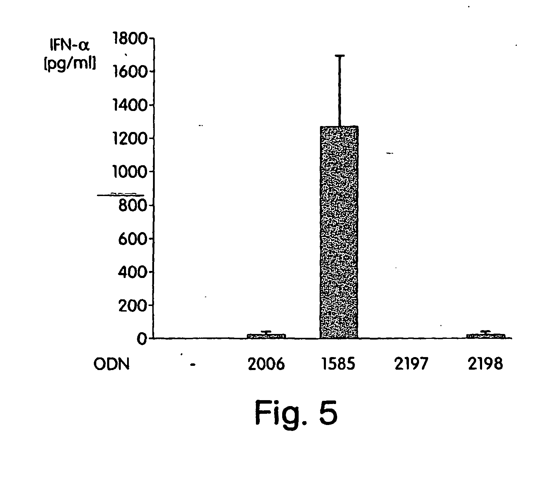

Figure 5 is a graph depicting the concentration of IFN-α induced in the supernatants of PBMC from different donors following incubation for 48 hours in the presence of 3 µM ODN 2006 (n=7), 1585 (n=7), 2197 (n=6), 2198 (n=5), or media without added ODN (n=7). Error bars indicate SEM.

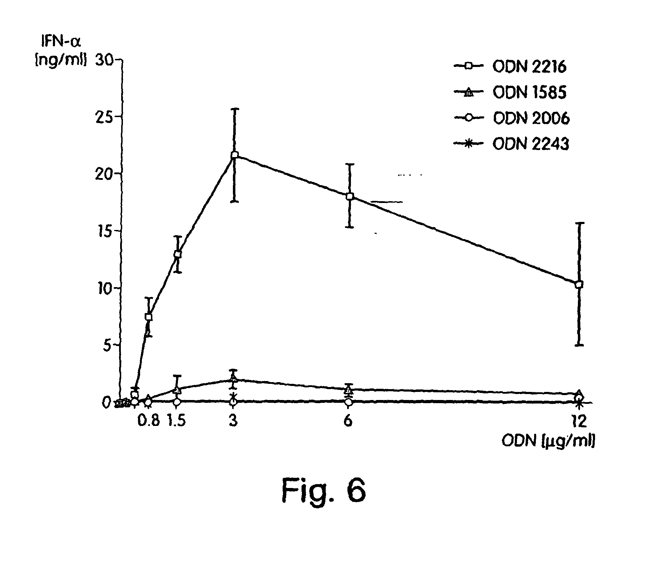

Figure 6 is a graph depicting the dose-response of CpG ODN-induced IFN-α synthesis by PBMC cultured for 48 hours in the presence of ODN 2216, 1585, 2006, and 2243 at concentrations ranging from 0.2 to 12 µg/ml.

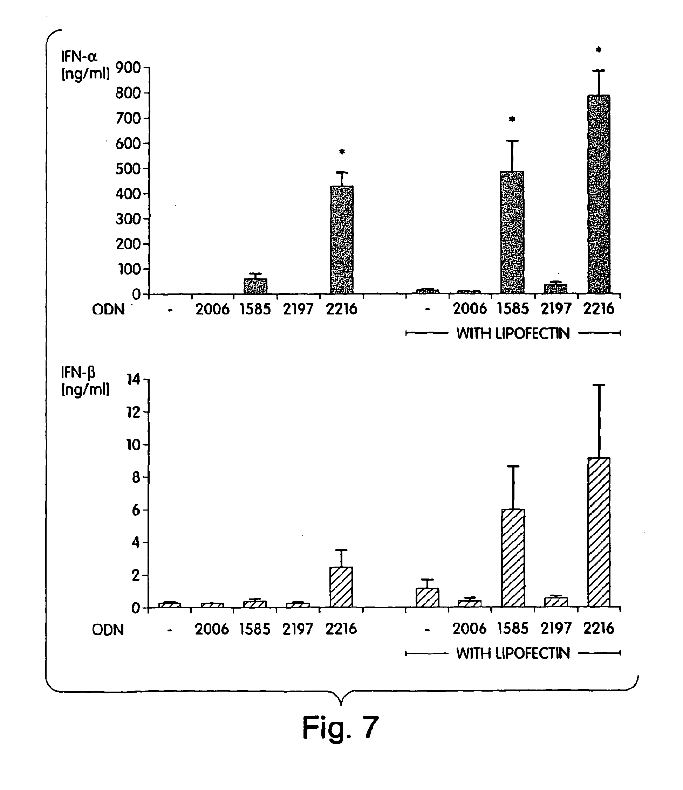

Figure 7 is a graph depicting CpG ODN-mediated stimulation of IFN-α and IFN-β production in PBMC enriched for plasmacytoid dendritic cells, with (n=3) and without (n=4) addition of lipofectin (10 µg/ml). PBMC were cultured for 48 hours in the presence of IL-3 alone (-) or IL-3 with the addition of ODN 2006, 1585, 2197, or 2216. Results are presented as means of 3 or 4 independent experiments with different donors, each performed in duplicate. Error bars indicate SEM. *p<0.0018 (Bonferroni-Dunn correction).

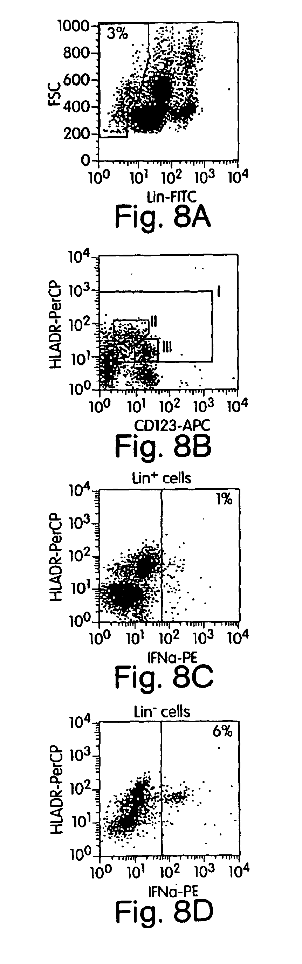

Figure 8 is a series of four graphs depicting results of four FACS experiments examining intracellular IFN-α. Panel A, identification of lin+ and lin⁻ cells. Panel B, identification of CD123^{+/-}/HLA DR⁺⁺ mDC (gate II) and CD123⁺⁺/HLA DR⁺pDC (gate III) in lin⁻ cells. Panel C, lack of staining for intracellular IFN-α in lin⁺ cells. Panel D, staining for intracellular IFN-α in lin⁻ cells.

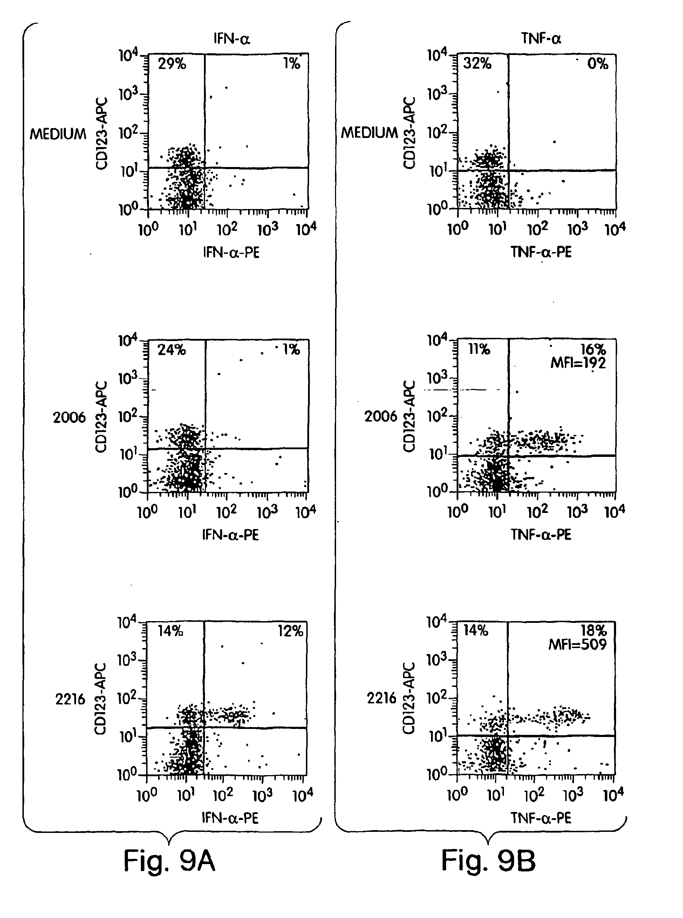

Figure 9 is a series of six graphs depicting results of six FACS experiments examining intracellular IFN-α (Panels A) and TNF-α (Panels B) in lin⁻/HLA DR⁺ plasmacytoid dendritic cell precursor cells after stimulation with different CpG oligonucleotides (2006, 2216, both at 3 µg/ml). Brefeldin A was added during incubation for TNF-α. MFI, mean fluorescence intensity.

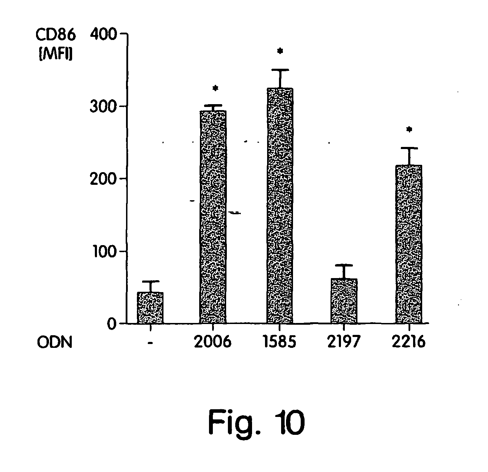

Figure 10 is a graph depicting CD86 expression on plasmacytoid dendritic cells in response to IL-3 alone (-) or to IL-3 with various CpG ODN (2006, 1585, 2197, or 2216, each at 3 µg/ml). Results are presented as means of three independent experiments with cells from different donors. Error bars indicate SEM. *p<0.0018 (Bonferroni-Dunn correction).

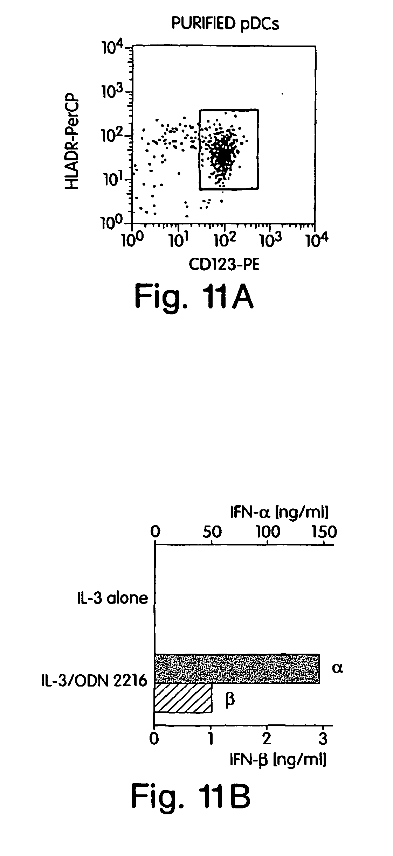

Figure 11 depicts a graph representing a FACS purification of plasmacytoid dendritic cells (Panel A) and the secretion of IFN-α and IFN-β by purified plasmacytoid dendritic cells in response to IL-3 with and without ODN 2216 (Panel B).

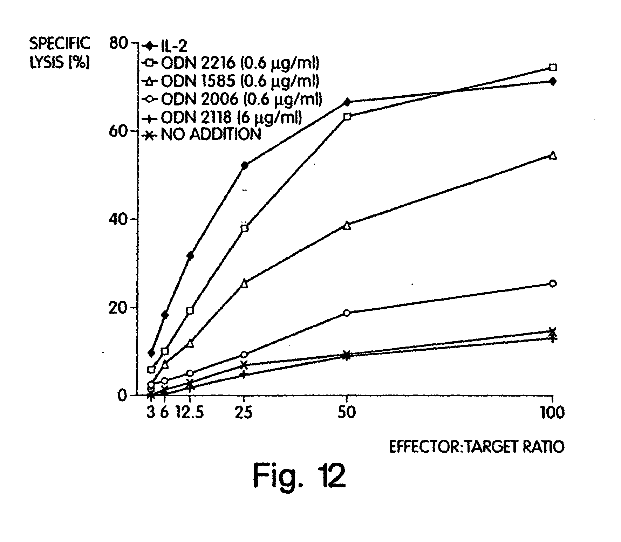

Figure 12 depicts NK cell-mediated lysis of K562 cells following exposure of PBMC to ODN 2216, 1585, 2006, 2118, IL-2, or media alone.

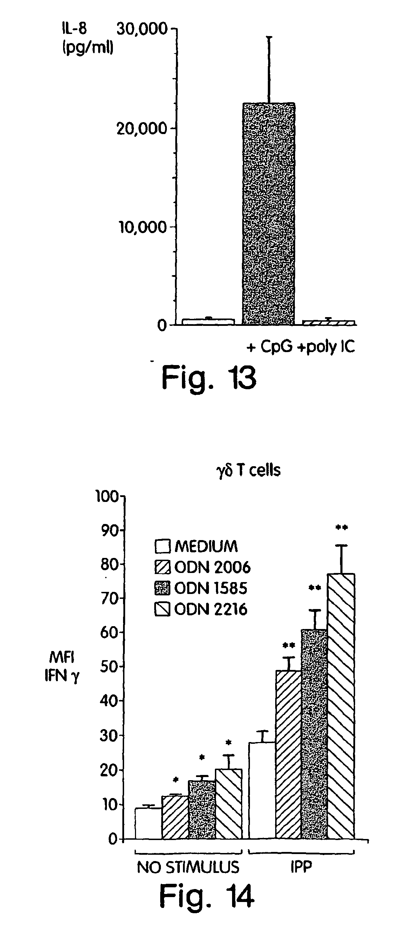

Figure 13 is a graph depicting the concentration of IL-8 (determined by an IL-8-specific ELISA) present in the supernatant of IPCs cultured for two days in the presence of IL-3 alone (left), IL-3 supplemented with CpG oligonucleotide (middle), or IL-3 supplemented with poly IC (right). Results are representative of three independent experiments.
Figure 14 is a graph depicting IFN-γ production by γδ T cells in response to CpG ODN 2006,1585, or 2216 in the presence or absence of nonpeptide antigen isopentenyl pyrophosphate (IPP). Results are shown in terms of mean fluorescence intensity (MFI) for intracellular staining for IFN-γ, with medium alone as negative control. Data are presented as mean + SEM; * (p < 0,01) and ** (p <0,001) indicate p values calculated by Student's t-test for paired samples comparing medium control to CpG ODN and IPP alone to IPP + CpG ODN.

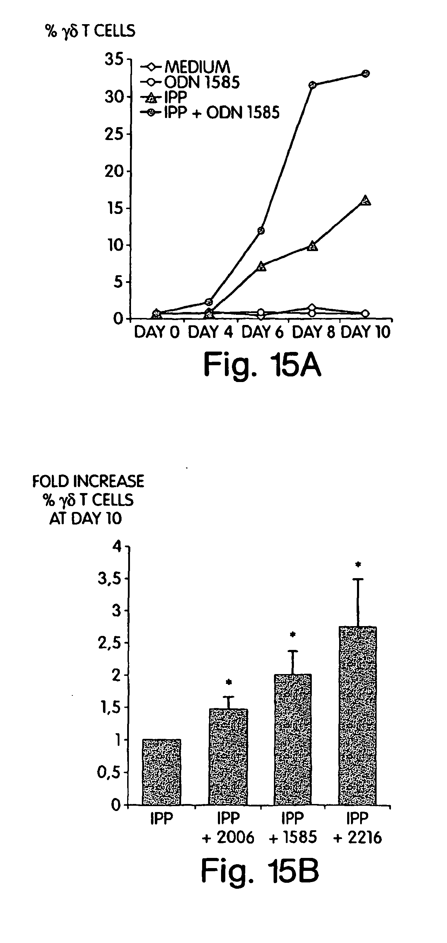

Figure 15 is a pair of graphs depicting proliferation of γδ T cells in response to CpG ODN 2006, 1585, or 2216 in the presence or absence of nonpeptide antigen isopentenyl pyrophosphate (IPP). Panel A depicts the kinetics of γδ T cell expansion, over 10 days, from one representative experiment. Panel B depicts the expansion of γδ T cells 10 days after stimulation with IPP alone or in combination with different CpG ODN. Between 9 and 16 donors were analyzed for each ODN. Data are presented as x-fold increase compared to IPP alone (mean + SEM); * indicates p < 0,05 (IPP versus IPP + CpG ODN.

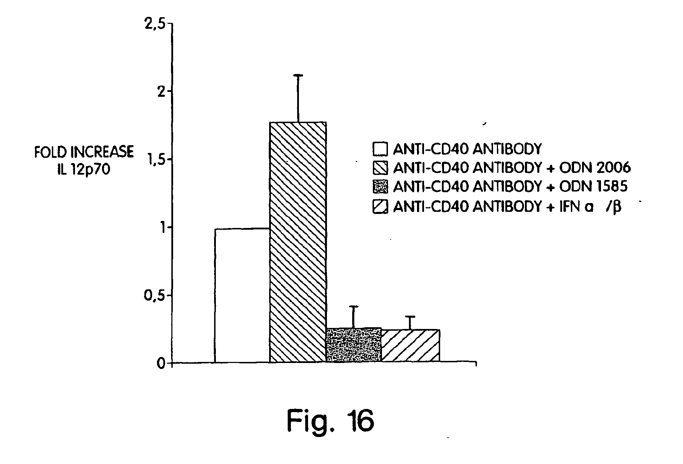

Figure 16 is a graph depicting regulation of CD40-induced IL-12p70 production by type I IFN and by various CpG ODN. Data are shown as x-fold of IL-12p70 production by anti-CD40 alone (mean = 143 pg/ml) and represent the mean + SEM of three different donors.

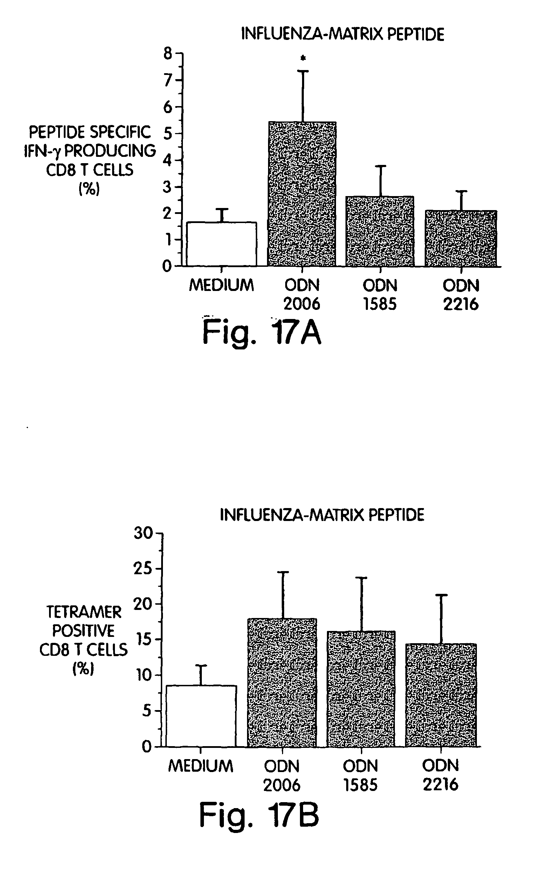

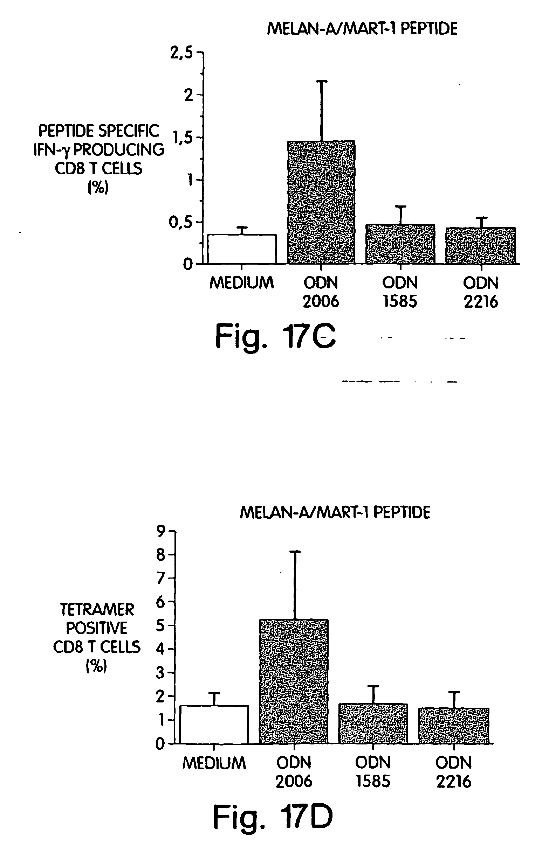

Figure 17 is a series of graphs depicting the effects of CpG ODN 2006, 1585, and 2216 on recall and primary peptide-specific human CTL responses. Panels A and C, peptide-specific IFN-γ producing CTL as a percentage of all CD8⁺ T cells for the recall antigen influenza-matrix peptide and for the primary antigen melan-A/mart-1 peptide, respectively. Panels B and D, antigen-specific tetramer-positive staining CD8⁺ T cells for the recall antigen influenza-matrix peptide and for the primary antigen melan-A/mart-1 peptide, respectively.

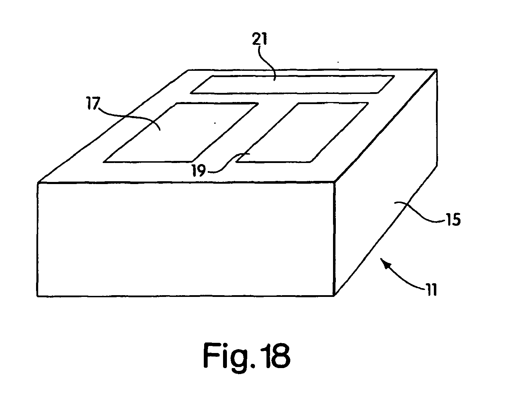

Figure 18 is a schematic representation of a kit which includes a container containing a composition which includes IFN-α in an amount which is at least about 10 percent less than the maximum tolerated dose (MTD) and, in the same container or in a separate container, an ISNA. The kit also can include instructions for treating a subject with a condition susceptible to treatment with IFN-α.

### Detailed Description of the Invention

The invention involves the discovery that a particular subset of blood cells, natural IFN-producing cells (IPCs), are stimulated by ISNAs to produce IFN-α. This discovery was surprising because it was previously unknown what component of UV-irradiated virus or of heat-killed bacteria was responsible for inducing IFN-α production by IPCs. Siegal FP et al. Science 284:1835-7 (1999). The discovery was also surprising because mature DC2s, which arise from the IPCs, are not strong producers of IFN-α. Furthermore, it was also known that monocyte-derived dendritic cells (DC1s) do not produce IFN-α in response to CpG nucleic acids. It also was surprising that a broad array of IFN-α molecules is stimulated. In addition, the invention involves the local induction of IFN-α at the site of ISNA administration, thus avoiding toxic effects associated with systemic administration of IFN-α in doses necessary to achieve similar local concentration of IFN-α. The invention also involves the unexpected discovery that ISNAs can stimulate IFN-producing cells to activate them to express the costimulatory molecule CD80 (B7-1). Another unexpected discovery is that ISNAs can support the survival of IFN-producing cells even in the absence of interleukin-3. These various discoveries have led to the *in vivo, ex vivo* and *in vitro* inventions described herein.

An ISNA is a nucleic acid molecule which, upon contacting cells of the immune system, is itself capable of inducing contacted cells of the immune system to proliferate and/or to become activated. The contacting can be direct or indirect, e.g., the ISNA may directly stimulate a first type of immune cell to express a product which may in turn stimulate a second type of immune cell which has not been exposed to, or is not responsive to, the ISNA. The immunostimulatory effect of the ISNA is separate from any product that might happen to be encoded by the sequence of the ISNA. Similarly, the immunostimulatory effect of an ISNA is distinct from and does not rely upon any antisense mechanism. Only certain nucleic acids are ISNAs. Originally it was believed that certain palindromic sequences were immunostimulatory. Tokunaga T et al. Microbiol Immunol 36:55-66 (1992); Yamamoto T et al. Antisense Res Dev 4:119-22 (1994). Further work demonstrated that non-palindromic sequences are also immunostimulatory provided they contained CpG dinucleotides within particular sequence contexts (CpG motifs). Krieg AM et al. Nature 374:546-9 (1995). The ISNAs can be single-stranded or double-stranded. Generally, double-stranded nucleic acid molecules are more stable *in vivo,* while single-stranded nucleic acid molecules have increased immune activity. Thus in some aspects of the invention it is preferred that the ISNA be single-stranded and in other aspects it is preferred that the ISNA be double-stranded.

The terms "nucleic acid" and "oligonucleotide" are used interchangeably to mean multiple, covalently linked nucleotides, wherein each nucleotide comprises a sugar (e.g., ribose or deoxyribose) linked to a phosphate group and to an exchangeable organic base, which is either a substituted pyrimidine (e.g., cytosine (C), thymine (T) or uracil (U)) or a substituted purine (e.g., adenine (A) or guanine (G).

As used herein, the terms "nucleic acid" and "oligonucleotide" refer to oligoribonucleotides as well as oligodeoxyribonucleotides. The terms shall also include polynucleosides (i.e., a polynucleotide minus the phosphate) and any other organic base-containing polymer. Nucleic acid molecules can be obtained from existing nucleic acid sources (e.g., genomic DNA or cDNA), but are preferably synthetic (e.g., produced by oligonucleotide synthesis).

The terms "nucleic acid" and "oligonucleotide" also encompass nucleic acids or oligonucleotides with a covalently modified base and/or sugar. For example, they include nucleic acids having backbone sugars which are covalently attached to low molecular weight organic groups other than a hydroxyl group at the 3' position and other than a phosphate group at the 5' position. Thus modified nucleic acids may include a 2'-O-alkylated ribose group. In addition, modified nucleic acids may include sugars such as arabinose instead of ribose. Thus the nucleic acids may be heterogeneous in backbone composition thereby containing any possible combination of polymer units linked together such as peptide nucleic acids (which have an amino acid backbone with nucleic acid bases). In some embodiments the nucleic acids are homogeneous in backbone composition.

Nucleic acids also can include base analogs such as C-5 propyne modified bases. Wagner et al. Nature Biotechnology 14:840-844 (1996). Purines and pyrimidines include but are not limited to adenine, cytosine, guanine, thymine, 5-methylcytosine, 2-aminopurine, 2-amino-6-chloropurine, 2,6-diaminopurine, hypoxanthine, and other naturally and non-naturally occurring nucleobases, substituted and unsubstituted aromatic moieties.

The nucleic acid is a linked polymer of bases, nucleobase analogs, or nucleotides. As used herein with respect to linked units of a nucleic acid, "linked" or "linkage" means two entities are bound to one another by any physicochemical means. Any linkage known to those of ordinary skill in the art, covalent or non-covalent, is embraced. Such linkages are well known to those of ordinary skill in the art. Natural linkages, which are those ordinarily found in nature connecting the individual units of a nucleic acid , are most common. The individual units of a nucleic acid may be linked, however, by synthetic or modified linkages.

The nucleic acids in accordance with the invention include at least one unmethylated CpG dinucleotide. An oligonucleotide containing at least one unmethylated CpG dinucleotide is a nucleic acid molecule which contains an unmethylated cytosine-guanine dinucleotide sequence (i.e., "CpG DNA" or DNA containing a 5' cytosine followed by 3' guanine and linked by a phosphate bond) and activates the immune system. The entire CpG oligonucleotide can be unmethylated or portions may be unmethylated but at least the C of the 5' CG 3' must be unmethylated. The CpG oligonucleotides can be double-stranded or single-stranded. The terms CpG oligonucleotide or CpG nucleic acid as used herein refer to an immunostimulatory CpG oligonucleotide or a nucleic acid unless otherwise indicated.

For use in the instant invention, the nucleic acids can be synthesized *de novo* using any of a number of procedures well known in the art. For example, the nucleic acids can be synthesized using the β-cyanoethyl phosphoramidite method (Beaucage SL and Caruthers MH Tetrahedron Lett 22:1859 (1981)) or the nucleoside H-phosphonate method (Garegg et al. Tetrahedron Lett 27:4051 (1986); Froehler et al. Nucl Acid Res 14:5399 (1986); Garegg et al. Tetrahedron Lett 27:4055 (1986); Gaffney et al. Tetrahedron Lett 29:2619 (1988)). These chemistries can be performed by a variety of automated oligonucleotide synthesizers available in the market. These oligonucleotides are referred to as synthetic oligonucleotides. Alternatively, ISNAs can be produced on a large scale in plasmids, (see Sambrook, T., et al., "Molecular Cloning: A Laboratory Manual", Cold Spring Harbor Laboratory Press, New York, 1989) and separated into smaller pieces or administered whole. Oligonucleotides can be prepared from existing nucleic acid sequences (e.g., genomic DNA or cDNA) using known techniques, such as those employing restriction enzymes, exonucleases or endonucleases. Oligonucleotides prepared in this manner are referred to as isolated oligonucleotides. The term ISNA encompasses both synthetic and isolated immunostimulatory nucleic acids.

For use *in vivo,* ISNAs are preferably relatively resistant to degradation (e.g., are stabilized). A "stabilized nucleic acid molecule" shall mean a nucleic acid molecule that is relatively resistant to *in vivo* degradation (e.g., via an exo- or endo-nuclease). For example, if the 3' end of an oligonucleotide has self-complementarity to an upstream region, so that it can fold back and form a sort of stem loop structure, then the oligonucleotide becomes stabilized and therefore exhibits more activity.

Alternatively, nucleic acid stabilization can be accomplished via phosphate backbone modifications. Preferred stabilized oligonucleotides of the instant invention have a modified backbone. It has been demonstrated that modification of the oligonucleotide backbone provides enhanced activity of the ISNAs when administered *in vivo.* These stabilized structures are preferred because the ISNAs of the invention have at least a partial modified backbone. For example, CpG oligonucleotides of a given sequence which include at least two phosphorothioate linkages at the 5' end of the oligonucleotide and multiple phosphorothioate linkages at the 3' end, preferably five, provide maximal activity and protect the oligonucleotide from degradation by intracellular exo- and endo-nucleases. Other modified oligonucleotides include phosphodiester modified oligonucleotides, combinations of phosphodiester and phosphorothioate oligonucleotide, methylphosphonate, methylphosphorothioate, phosphorodithioate, and combinations thereof. Each of these combinations and their particular effects on immune cells is discussed in more detail in PCT Published Patent Applications PCT/US95101570 and PCTIUS97/19791 claiming priority to U.S. Serial Nos. 08/386,063 and 08/960,774, filed on February 7, 1995 and October 30, 1997, respectively. It is believed that these modified backbone oligonucleotides may show more stimulatory activity due to enhanced nuclease resistance, increased cellular uptake, increased protein binding, and/or altered intracellular localization.

Modified backbones such as phosphorothioates may be synthesized using automated techniques employing either phosphoramidate or H-phosphonate chemistries. Aryl- and alkyl-phosphonates can be made, e.g., as described in U.S. Patent No. 4,469,863; and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described. Uhlmann E and Peyman A Chem Rev 90:544 (1990); Goodchild J Bioconjugate Chem 1:,165 (1990).

Other stabilized oligonucleotides include: nonionic DNA analogs, such as alkyl- and aryl-phosphates (in which the charged phosphonate oxygen is replaced by an alkyl or aryl group), and alkylphosphodiester and alkylphosphotriesters (in which the charged oxygen moiety is alkylated). Oligonucleotides which contain diol, such as tetraethyleneglycol or hexaethyleneglycol, at either or both termini have also been shown to be substantially resistant to nuclease degradation.

In some embodiments the ISNAs useful according to the invention are S and R chiral ISNAs. An "S chiral ISNA" as used herein is an ISNA wherein at least two nucleotides have a backbone modification forming a chiral center and wherein a plurality of the chiral centers have S chirality. An "R chiral ISNA" as used herein is an ISNA wherein at least two nucleotides have a backbone modification forming a chiral center and wherein a plurality of the chiral centers have R chirality. The backbone modification may be any type of modification that forms a chiral center. The modifications include but are not limited to phosphorothioate, phosphorodithioate, methylphosphonate, methylphosphorothioate, and combinations thereof.

The chiral ISNAs must have at least two nucleotides within the oligonucleotide that have a backbone modification. All or less than all of the nucleotides in the oligonucleotides, however, may have a modified backbone. Of the nucleotides having a modified backbone (referred to as chiral centers), a plurality have a single chirality, S or R. A "plurality" as used herein refers to an amount greater than 50 percent. Thus, less than all of the chiral centers may have S or R chirality as long as a plurality of the chiral centers have S or R chirality. In some embodiments at least 55 percent, 60 percent, 65 percent, 70 percent, 75 percent, 80 percent, 85 percent, 90 percent, 95 percent, or 100 percent of the chiral centers have S or R chirality. In other embodiments at least 55 percent, 60 percent, 65 percent, 70 percent, 75 percent, 80 percent, 85 percent, 90 percent, 95 percent, or 100 percent of the nucleotides have backbone modifications.

The S and R chiral ISNAs may be prepared by any method known in the art for producing chirally pure oligonucleotides. Many references teach methods for producing stereopure phosphorothioate oligodeoxynucleotides using an oxathiaphospholane method have been published. Stec WJ et al. J Am Chem Soc 117:12019 (1995). Other methods for making chirally pure oligonucleotides have been described by companies such as ISIS Pharmaceuticals. U.S. Patents have also described these methods. For instance, U.S. Patent Nos. 5,883,237; 5,837,856; 5,599,797; 5,512,668; 5,856,465; 5,359,052; 5,506,212; 5,521,302; and 5,212,295 disclose methods for generating stereopure oligonucleotides.

A "subject" shall mean a human or vertebrate animal including but not limited to a dog, cat, horse, cow, pig, sheep, goat, chicken, non-human primate (e.g., monkey), fish (aquaculture species, e.g., salmon), rabbit, rat, and mouse.

A "subject having a proliferative disorder" is a subject that has detectable and unwanted proliferating cells. The unwanted proliferating cell can be cancerous cells in a subject with cancer. The cancer may be a malignant or non-malignant cancer. Cancers or tumors include but are not limited to biliary tract cancer; bladder cancer; brain cancer; breast cancer; cervical cancer; choriocarcinoma; colon cancer; endometrial cancer; esophageal cancer; gastric cancer; intraepithelial neoplasms; leukemia; liver cancer; lung cancer (e.g., small cell and non-small cell); lymphoma; melanoma; multiple myeloma; neuroblastomas; oral cancer; ovarian cancer; pancreas cancer; prostate cancer; rectal cancer; renal cancer, sarcomas; skin cancer; stomach cancer; testicular cancer; and thyroid cancer; as well as other carcinomas and sarcomas. In other embodiments the unwanted proliferating cells can be non-cancerous, e.g., cells associated with an autoimmune condition or inflammatory condition.

A "subject having a viral infection" is a subject that has been exposed to a virus and has acute or chronic manifestations or detectable levels of the virus in the body.

Examples of viruses that have been found in humans include but are not limited to: *Retroviridae* (e.g., human immunodeficiency viruses, such as HIV- (also referred to as HTLV-III, LAV or HTLV-III/LAV, or HIV-III; and other isolates, such as HIV-LP; *Picornaviridae* (e.g., polio viruses, hepatitis A virus; enteroviruses, human Coxsackie viruses, rhinoviruses, echoviruses); *Calciviridae* (e.g., strains that cause gastroenteritis); *Togaviridae* (e.g., equine encephalitis viruses, rubella viruses); *Flaviridae* (e.g., hepatitis C virus (HCV), dengue viruses, encephalitis viruses, yellow fever viruses); *Coronoviridae* (e.g., coronaviruses); *Rhabdoviridae* (e.g., vesicular stomatitis viruses, rabies viruses); *Filoviridae* (e.g., ebola viruses); *Paramyxoviridae* (e.g., parainfluenza viruses, mumps virus, measles virus, respiratory syncytial virus); *Orthomyxoviridae* (e.g., influenza viruses); *Bungaviridae* (e.g., Hantaan viruses, bunga viruses, phleboviruses and Nairo viruses); Arena viridae (hemorrhagic fever viruses); *Reoviridae* (e.g., reoviruses, orbiviurses and rotaviruses); *Birnaviridae; Hepadnaviridae* (Hepatitis B virus); *Parvovirida* (parvoviruses); *Papovaviridae* (papilloma viruses, polyoma viruses); *Adenoviridae* (most adenoviruses); *Herpesviridae* (herpes simplex virus (HSV) 1 and 2, varicella zoster virus, cytomegalovirus (CMV), herpes virus; *Poxviridae* (variola viruses, vaccinia viruses, pox viruses); *Iridoviridae* (e.g., African swine fever virus); and unclassified viruses (e.g., the etiological agents of spongiform encephalopathies, the agent of delta hepatitis (thought to be a defective satellite of hepatitis B virus), the unclassified agents of non-A, non-B hepatitis (class 1 = internally transmitted; class 2 = parenterally transmitted); Norwalk and related viruses, and astroviruses).

Although many of the viruses described above relate to human disorders, the invention is also useful for treating nonhuman vertebrates. Nonhuman vertebrates are also capable of developing infections which can be prevented or treated with the ISNAs disclosed herein. For instance, in addition to the treatment of infectious human diseases, the methods of the invention are useful for treating infections of animals.

Infectious virus of both human and non-human vertebrates include retroviruses, RNA viruses and DNA viruses. This group of retroviruses includes both simple retroviruses and complex retroviruses. The simple retroviruses include the subgroups of B-type retroviruses, C-type retroviruses and D-type retroviruses. An example of a B-type retrovirus is mouse mammary tumor virus (MMTV). The C-type retroviruses include subgroups C-type group A (including Rous sarcoma virus (RSV), avian leukemia virus (ALV), and avian myeloblastosis virus (AMV)) and C-type group B (including murine leukemia virus (MLV), feline leukemia virus (FeLV), murine sarcoma virus (MSV), gibbon ape leukemia virus (GALV), spleen necrosis virus (SNV), reticuloendotheliosis virus (RV) and simian sarcoma virus (SSV)). The D-type retroviruses include Mason-Pfizer monkey virus (MPMV) and simian retrovirus type 1 (SRV-1). The complex retroviruses include the subgroups of lentiviruses, T-cell leukemia viruses and the foamy viruses. Lentiviruses include HIV-1, but also include HIV-2, SIV, Visna virus, feline immunodeficiency virus (FIV), and equine infectious anemia virus (EIAV). The T-cell leukemia viruses include HTLV-1, HTLV-2, simian T-cell leukemia virus (STLV), and bovine leukemia virus (BLV). The foamy viruses include human foamy virus (HFV), simian foamy virus (SFV) and bovine foamy virus (BFV).

Examples of other RNA viruses that are antigens in vertebrate animals include, but are not limited to, the following: members of the family Reoviridae, including the genus Orthoreovirus (multiple serotypes of both mammalian and avian retroviruses), the genus Orbivirus (Bluetongue virus, Eugenangee virus, Kemerovo virus, African horse sickness virus, and Colorado Tick Fever virus), the genus Rotavirus (human rotavirus, Nebraska calf diarrhea virus, murine rotavirus, simian rotavirus, bovine or ovine rotavirus, avian rotavirus); the family Picornaviridae, including the genus Enterovirus (poliovirus, Coxsackie virus A and B, enteric cytopathic human orphan (ECHO) viruses, hepatitis A virus, Simian enteroviruses, Murine encephalomyelitis (ME) viruses, Poliovirus muris, Bovine enteroviruses, Porcine enteroviruses , the genus Cardiovirus (encephalomyocarditis virus (EMC), Mengovirus), the genus Rhinovirus (Human rhinoviruses including at least 113 subtypes; other rhinoviruses), the genus Apthovirus (Foot and Mouth disease (FMDV); the family Calciviridae, including Vesicular exanthema of swine virus, San Miguel sea lion virus, Feline picornavirus and Norwalk virus; the family Togaviridae, including the genus Alphavirus (Eastern equine encephalitis virus, Semliki forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); forest virus, Sindbis virus, Chikungunya virus, O'Nyong-Nyong virus, Ross river virus, Venezuelan equine encephalitis virus, Western equine encephalitis virus), the genus Flavirius (Mosquito borne yellow fever virus, Dengue virus, Japanese encephalitis virus, St. Louis encephalitis virus, Murray Valley encephalitis virus, West Nile virus, Kunjin virus, Central European tick borne virus, Far Eastern tick borne virus, Kyasanur forest virus, Louping III virus, Powassan virus, Omsk hemorrhagic fever virus), the genus Rubivirus (Rubella virus), the genus Pestivirus (Mucosal disease virus, Hog cholera virus, Border disease virus); the family Bunyaviridae, including the genus Bunyvirus (Bunyamwera and related viruses, California encephalitis group viruses), the genus Phlebovirus (Sandfly fever Sicilian virus, Rift Valley fever virus), the genus Nairovirus (Crimean-Congo hemorrhagic fever virus, Nairobi sheep disease virus), and the genus Uukuvirus (Uukuniemi and related viruses); the family Orthomyxoviridae, including the genus Influenza virus (Influenza virus type A, many human subtypes); Swine influenza virus, and Avian and Equine Influenza viruses; influenza type B (many human subtypes), and influenza type C (possible separate genus); the family paramyxoviridae, including the genus Paramyxovirus (Parainfluenza virus type 1, Sendai virus, Hemadsorption virus, Parainfluenza viruses types 2 to 5, Newcastle Disease Virus, Mumps virus), the genus Morbillivirus (Measles virus, subacute sclerosing panencephalitis virus, distemper virus, Rinderpest virus), the genus Pneumovirus (respiratory syncytial virus (RSV), Bovine respiratory syncytial virus and Pneumonia virus of mice); the family Rhabdoviridae, including the genus Vesiculovirus (VSV), Chandipura virus, Flanders-Hart Park virus), the genus Lyssavirus (Rabies virus), fish Rhabdoviruses, and two probable Rhabdoviruses (Marburg virus and Ebola virus); the family Arenaviridae, including Lymphocytic choriomeningitis virus (LCM), Tacaribe virus complex, and Lassa virus; the family Coronoaviridae, including Infectious Bronchitis Virus (IBV), Mouse Hepatitis virus, Human enteric corona virus, and Feline infectious peritonitis (Feline coronavirus).

Illustrative DNA viruses that are antigens in vertebrate animals include, but are not limited to: the family Poxviridae, including the genus Orthopoxvirus (Variola major, Variola minor, Monkey pox Vaccinia, Cowpox, Buffalopox, Rabbitpox, Ectromelia), the genus Leporipoxvirus (Myxoma, Fibroma), the genus Avipoxvirus (Fowlpox, other avian poxvirus), the genus Capripoxvirus (sheeppox, goatpox), the genus Suipoxvirus (Swinepox), the genus Parapoxvirus (contagious postular dermatitis virus, pseudocowpox, bovine papular stomatitis virus); the family Iridoviridae (African swine fever virus, Frog viruses 2 and 3, Lymphocystis virus of fish); the family Herpesviridae, including the α-Herpesviruses (Herpes Simplex Types 1 and 2, Varicella-Zoster, Equine abortion virus, Equine herpes virus 2 and 3, pseudorabies virus, infectious bovine keratoconjunctivitis virus, infectious bovine rhinotracheitis virus, feline rhinotracheitis virus, infectious laryngotracheitis virus) the Beta-herpesviruses (Human cytomegalovirus and cytomegaloviruses of swine, monkeys and rodents); the gamma-herpesviruses (Epstein-Barr virus (EBV), Marek's disease virus, Herpes saimiri, Herpesvirus ateles, Herpesvirus sylvilagus, guinea pig herpes virus, Lucke tumor virus); the family Adenoviridae, including the genus Mastadenovirus (Human subgroups A,B,C,D,E and ungrouped; simian adenoviruses (at least 23 serotypes), infectious canine hepatitis, and adenoviruses of cattle, pigs, sheep, frogs and many other species, the genus Aviadenovirus (Avian adenoviruses); and non-cultivatable adenoviruses; the family Papoviridae, including the genus Papillomavirus (Human papilloma viruses, bovine papilloma viruses, Shope rabbit papilloma virus, and various pathogenic papilloma viruses of other species), the genus Polyomavirus (polyomavirus, Simian vacuolating agent (SV-40), Rabbit vacuolating agent (RKV), K virus, BK virus, JC virus, and other primate polyoma viruses such as Lymphotrophic papilloma virus); the family Parvoviridae including the genus Adeno-associated viruses, the genus Parvovirus (Feline panleukopenia virus, bovine parvovirus, canine parvovirus, Aleutian mink disease virus, etc.). Finally, DNA viruses may include viruses which do not fit into the above families such as Kuru and Creutzfeldt-Jacob disease viruses and chronic infectious neuropathic agents (CHINA virus).

Each of the foregoing lists is illustrative, and is not intended to be limiting. In addition, these viruses, either in intact form or as fragments thereof, can be used as antigens in immunization procedures. An antigen is a substance recognized by the immune system as foreign and which induces specific immunity. Antigens can be carbohydrates (including, e.g., polysaccharides, glycolipids, and glycoproteins), proteins and polypeptides, as well as other oligomers, polymers, and small molecules which can bind to antigen receptors on immune cells. Specific immunity to an antigen can involve antigen recognition by T cells and/or B cells.

Nucleic acids containing an appropriate ISNA can be effective in any vertebrate. Different nucleic acids containing an ISNA can cause optimal immune stimulation depending on the mammalian species. Thus an oligonucleotide causing optimal stimulation or inhibition in humans may not cause optimal stimulation or inhibition in a mouse, and vice versa. One of skill in the art can identify the optimal oligonucleotides useful for a particular mammalian species of interest using routine assays described herein and/or known in the art, using the guidance supplied herein.

The ISNA may be directly administered to the subject or may be administered in conjunction with a nucleic acid delivery complex. A "nucleic acid delivery complex" shall mean a nucleic acid molecule associated with (e.g., ionically or covalently bound to; or encapsulated within) a targeting means (e.g., a molecule that results in higher affinity binding to target cell (e.g., B cell surfaces and/or increased cellular uptake by target cells). Examples of nucleic acid delivery complexes include nucleic acids associated with: a sterol (e.g., cholesterol), a lipid (e.g., a cationic lipid, virosome or liposome), or a target cell specific binding agent (e.g., a ligand recognized by target cell specific receptor). Preferred complexes may be sufficiently stable *in vivo* to prevent significant uncoupling prior to internalization by the target cell. However, the complex can be cleavable under appropriate conditions within the cell so that the nucleic acid is released in a functional form.

The ISNA or other therapeutics may be administered alone (e.g., in saline or buffer) or using any delivery vehicles known in the art. For instance the following delivery vehicles have been described: cochleates (Gould-Fogerite et al., 1994, 1996); emulsomes (Vancott et al., 1998, Lowell et al., 1997); ISCOMs (Mowat et al., 1993, Carlsson et al., 1991, Hu et., 1998, Morein et al., 1999); liposomes (Childers et al., 1999, Michalek et al., 1989, 1992, de Haan 1995a, 1995b); live bacterial vectors (e.g., *Salmonella, Escherichia coli, Bacillus Calmette-Guerin, Shigella, Lactobacillus*) (Hone et al., 1996, Pouwels et al., 1998, Chatfield et al., 1993, Stover et al., 1991, Nugent et al., 1998); live viral vectors (e.g., Vaccinia, adenovirus, Herpes Simplex) (Gallichan et al., 1993, 1995, Moss et al., 1996, Nugent et al., 1998, Flexner et al., 1988, Morrow et al., 1999); microspheres (Gupta et al., 1998, Jones et al., 1996, Maloy et al., 1994, Moore et al., 1995, O'Hagan et al., 1994, Eldridge et al., 1989); nucleic acid vaccines (Fynan et al., 1993, Kuklin et al., 1997, Sasaki et al., 1998, Okada et al., 1997, Ishii et al., 1997); polymers (e.g., carboxymethylcellulose, chitosan) (Hamajima et al., 1998, Jabbal-Gill et al., 1998); polymer rings (Wyatt et al., 1998); Proteosomes (Vancott et al., 1998, Lowell et al., 1988, 1996, 1997); sodium fluoride (Hashi et al., 1998); transgenic plants (Tacket et al., 1998, Mason et al., 1998, Haq et al., 1995); virosomes (Gluck et al., 1992, Mengiardi et al., 1995, Cryz et al., 1998); virus-like particles (Jiang et al., 1999, Leibl et al., 1998). Those skilled in the art will recognize that other delivery vehicles that are known in the art may also be used.

Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial toxicity and yet is entirely effective to treat the particular subject. The effective amount for any particular application can vary depending on such factors as the disease or condition being treated, the particular ISNA being administered (e.g., the number of unmethylated CpG motifs or their location in the nucleic acid, the degree of chirality to the oligonucleotide), the antigen, the size of the subject, or the severity of the disease or condition. One of ordinary skill in the art can empirically determine the effective amount of a particular ISNA and/or antigen and/or other therapeutic agent without necessitating undue experimentation.

For adult human subjects, doses of the ISNA compounds described herein typically range from about 50 µg/dose to 20 mg/dose, more typically from about 80 µg/dose to 8 mg/dose, and most typically from about 800 µg/dose to 4 mg/dose. Stated in terms of subject body weight, typical dosages range from about 0.5 to 500 µg/kg/dose, more typically from about 1 to 100 µg/kg/dose, and most typically from about 10 to 50 µg/kg/dose. Doses will depend on factors including the route of administration, e.g., oral administration may require a substantially larger dose than subcutaneous administration.

The formulations of the invention are administered in pharmaceutically acceptable solutions, which may routinely contain pharmaceutically acceptable concentrations of salt, buffering agents, preservatives, compatible carriers, adjuvants, and optionally other therapeutic ingredients.

The ISNA can be given in conjunction with other agents known in the art to be useful in combination with IFN-α to treat viral and proliferative disorders. Examples of such other agents currently used or under investigation for use in combination with IFN-α include ribavirin, amantadine, chemotherapeutic agents (e.g., 5-fluorouracil and BCNU), radiation therapy, phototherapy, and cytokines, including IL-2, IL-12, and IFN-γ.

For use in therapy, an effective amount of the ISNA can be administered to a subject by any mode that delivers the ISNA to the desired site, e.g., mucosal, systemic. "Administering" the pharmaceutical composition of the present invention may be accomplished by any means known to the skilled artisan. Preferred routes of administration include but are not limited to oral, parenteral, intralesional, topical, transdermal, intramuscular, intranasal, intratracheal, inhalational, ocular, vaginal, and rectal.

For oral administration, the compounds (i.e., ISNA, antigen, other therapeutic agent) can be formulated readily by combining the active compound(s) with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers for neutralizing internal acid conditions or may be administered without any carriers.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

For administration by inhalation, the compounds for use according to the present invention may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the compound and a suitable powder base such as lactose or starch.

The compounds, when it is desirable to deliver them systemically, may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, e.g., sterile pyrogen-free water, before use.

The compounds may also be formulated in rectal or vaginal compositions such as suppositories or retention enemas, e.g., containing conventional suppository bases such as cocoa butter or other glycerides.

In addition to the formulations described previously, the compounds may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer Science 249:1527 (1990), which is incorporated herein by reference.

The ISNAs may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulfuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2 percent w/v); citric acid and a salt (1-3 percent w/v); boric acid and a salt (0.5-2.5 percent w/v); and phosphoric acid and a salt (0.8-2 percent w/v). Suitable preservatives include benzalkonium chloride (0.003-0.03 percent w/v); chlorobutanol (0.3-0.9 percent w/v); parabens (0.01-0.25 percent w/v) and thimerosal (0.004-0.02 percent w/v).

The pharmaceutical compositions of the invention contain an effective amount of an ISNA and optionally antigens and/or other therapeutic agents optionally included in a pharmaceutically-acceptable carrier. The term "pharmaceutically-acceptable carrier" means one or more compatible solid or liquid filler, diluants or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

A variety of administration routes are available. The particular mode selected will depend, of course, upon the particular adjuvants or antigen selected, the particular condition being treated and the dosage required for therapeutic efficacy. The methods of this invention, generally speaking, may be practiced using any mode of administration that is medically acceptable, meaning any mode that produces effective levels of an immune response without causing clinically unacceptable adverse effects. Preferred modes of administration are discussed above.

The compositions may conveniently be presented in unit dosage form and may be prepared by any of the methods well known in the art of pharmacy. All methods include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product. Liquid dose units are vials or ampoules. Solid dose units are tablets, capsules and suppositories. For treatment of a patient, depending on activity of the compound, manner of administration, purpose of the immunization (i.e., prophylactic or therapeutic), nature and severity of the disorder, age and body weight of the patient, different doses may be necessary. The administration of a given dose can be carried out both by single administration in the form of an individual dose unit or else several smaller dose units.

Other delivery systems can include time-release, delayed release or sustained release delivery systems. Such systems can avoid repeated administrations of the compounds, increasing convenience to the subject and the physician. Many types of release delivery systems are available and known to those of ordinary skill in the art. They include polymer-based systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono-, di- and tri-glycerides; hydrogel release systems; silastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

As used herein, a method which calls for administration of IFN-α refers to a clinical method for treating a subject by administration of IFN-α with the aim of achieving a desired therapeutic result. There are a number of clinical indications for which IFN-α is an established therapeutic agent. A subject in need of IFN-α treatment has a clinical indication for which IFN-α is an established therapeutic agent. These clinical indications include certain viral infections and certain proliferative disorders, notably cancers and pre-cancerous conditions. The viral infections for which IFN-α has current approval for use in the United States are hepatitis B, hepatitis C, and condylomata acuminata (venereal or anogenital warts). The neoplasms for which IFN-α has current approval for use in the United States are hairy cell leukemia, cutaneous T-cell leukemia, chronic myelogenous leukemia (CML), non-Hodgkin's lymphoma, malignant melanoma, and AIDS-related Kaposi's sarcoma. Outside of the United States, IFN-α is also in clinical use for bladder cell carcinoma, colon carcinoma, renal cell carcinoma, multiple myeloma, cervical dysplasia, and laryngeal papillomatosis. Other indications under investigation for IFN-α treatment include other viral infections and other cancers, including, for example, Behcet's disease, HIV, prostate cancer, small cell lung cancer, pancreatic cancer, squamous cell carcinoma, glioma, and malignant pleural mesothelioma, among others.

As used herein, a pharmaceutical composition comprising IFN-α refers to a preparation of recombinant or natural IFN-α suitable for pharmaceutical use. IFN-α may be derived from natural material (e.g., leukocytes, myeloblasts, lymphocytes) or material derived therefrom (e.g., cell lines), or those prepared with recombinant DNA technology. Details of the cloning of IFN-α and the direct expression thereof, especially in *Escherichia coli,* have been the subject of many publications. The preparation of recombinant IFNs is known, for example, from Gray et al. Nature 295:503-8 (1982), Goeddel et al. Nature 284:316-20 (1980), Goeddel et al. Nature 290:20-26 (1981), and EP 174143. In the United States IFN-α is available as recombinant human IFN-α2a (ROFERON-A), recombinant human IFN-α2b (INTRON A), and as purified natural IFN-αn3 (ALFERON N). Outside the United States, IFN-α is also available as purified natural IFN-αn1 (WELLFERON).

As used herein, a clinically established effective dose for IFN-α alone refers to a dose of recombinant or natural IFN-α, administered in the absence of another agent that increases IFN-α bioavailability, that is the standard recommended dose for a particular clinical indication. A clinically established effective dose for IFN-α alone can encompass, however, the use of IFN-α in combination with other agents and treatment modalities, such as conventional chemotherapy, radiation therapy, antiviral agents, and surgery. In a majority of subjects the standard recommended dose of IFN-α for a particular clinical indication would be expected to exert a desired clinical effect. In a given clinical application in a given subject, a clinically established effective dose for IFN-α alone can also refer to a dose of IFN that is, or has been, or would be expected to be effective in that subject for treating a condition of the subject. For example, a subject might be responsive to a dose of IFN-α alone that is less than the standard recommended dose. Conversely, a subject might be unable to tolerate a clinically established effective dose due to actual or anticipated side effects of the IFN-α treatment.

The maximum tolerated dose (MTD) for any therapeutic compound is identified as part of its clinical evaluation. For example, phase I trials can include a determination of the maximum tolerated dose, dose-limiting toxicities (DLT) and pharmacokinetics of a test compound. Thus, the MTD for any Food and Drug Administration (FDA) approved therapeutic compound is known to those of ordinary skill in the art as a matter of the public record. The MTD for any particular therapeutic compound may vary according to its formulation (e.g., injectable formulation, implantable bioerodible polymer formulation, oral formulation), route of delivery (e.g., intravenous, oral, intratumoral), manner of delivery (e.g., infusion, bolus injection), dosing schedule (e.g., hourly, daily, weekly) and the like. MTD frequently is defined as the highest dose level at which 50 percent of subjects administered with the drug develop a dose-limiting toxicity. Other definitions which are clinically relevant and generally accepted will be known to one of ordinary skill in the art.

Examples of MTD for various types of IFN-a have been published in studies involving various routes of administration, indications, combinations with other agents, and clinical settings. In one study the MTD of recombinant IFN-α2a was 18 million international units (IU) when given intramuscularly three times weekly in combination with phototherapy for treatment of cutaneous T-cell lymphoma (mycosis fungoides and Sézary syndrome). Kuzel TM et al. J Natl Cancer Inst 82:203-7 (1990). In an independent study, the MTD of IFN-α2b for the treatment of cutaneous T-cell lymphoma was found to be 18 million IU given intramuscularly three times a week. Qiu B and Chen M Chin Med J (Engl) 109:404-6 (1996). The MTD for IFN-α2a was lower, 3 million IU subcutaneously three times a week, for patients undergoing high dose pelvic radiation for rectal cancer. Perera F et al. Int J Radiat Oncol Biol Phys 37:297-303 (1997). The MTD for IFN-α2b was 10 million IU daily for patients with AIDS-related Kaposi's sarcoma following cytotoxic chemotherapy. Gill PS et al. J Biol Response Mod 9:512-6 (1990). In yet another study the MTD of IFN-α2b was 18 million IU/m² given weekly as a 24-hour infusion in combination with 5-fluorouracil and leucovorin to patients with metastatic colorectal cancer. Cascinu S et al. Anticancer Drugs 7:520-4 (1996).

Measurement of maximum tolerated dose may be expressed as weight of drug per weight of subject, weight of drug per body surface area, etc. The MTD of anticancer compounds is frequently expressed as weight per square meters (mg/m²) of body surface area. MTD also may be expressed as a dose relative to a time component, such as weight of drug per body surface area per day.

For therapeutics which have not yet been subjected to human clinical trials, or subjected to any determination of the MTD in humans (e.g., experimental or highly toxic compounds), one of skill in the art can estimate the MTD by using animal models. Calculation of MTD in animals may be based on a number of physiological parameters, such as death, particular toxicities, and drug-induced weight loss. Using death as an endpoint, the MTD may be the highest dose given test animals in which each member of the test group survived. Using toxicity as an endpoint, the MTD may be the dose at which moderate but not severe toxicity is observed. Using weight loss as an endpoint, the MTD may be the dose above which a certain percent change in body weight is induced. Other methods for determining MTDs using animal models and various endpoints are known to one of ordinary skill in the art. Correlation of animal MTDs to human MTDs for a therapeutic compound is an accepted practice in the pharmaceutical arts.

Thus the invention in one aspect provides compositions and formulations for administration to a subject, preferably a human subject, containing an amount of interferon which is below the maximum tolerated dose for interferon.

In one aspect the invention, involves treating a subject in need of treatment with IFN-α which entails coadministration of an effective amount of an isolated ISNA in conjunction with administration of IFN-α. As used herein, an effective amount of an isolated ISNA refers to that amount of an isolated ISNA that causes cells to produce IFN-α. In one preferred embodiment an effective amount of an isolated ISNA refers to that amount of an isolated ISNA that causes cells *in vivo* to produce IFN-α. In another preferred embodiment an effective amount of an isolated ISNA refers to that amount of an isolated ISNA corresponding to an amount that causes cells *in vitro* to produce IFN-α. In another preferred embodiment an effective amount of an isolated ISNA refers to that amount of an isolated ISNA that causes an increase in the local or circulating amount of IFN-α above the corresponding level which would occur only from the administration of exogenous IFN-α. In another preferred embodiment an effective amount of an isolated ISNA refers to that amount of an isolated ISNA that increases the therapeutic benefit of exogenous IFN-α above that which would be obtained without the ISNA. In yet another embodiment an effective amount of an isolated ISNA refers to that amount of an isolated ISNA that would allow the same therapeutic effect to be achieved for a given amount of IFN-α when the oligonucleotide is coadministered with a lower dose of IFN-α.

As used herein, the term coadminister refers to administering at least two agents in clinical association with one another. Coadministration can include administering the at least two agents together or sequentially. In a preferred embodiment, ISNA is administered either before, at the same time as, or after administering IFN-α, provided the local or systemic concentration of IFN-α is increased over the corresponding concentration of IFN-α that would be achieved by administering the same amount of IFN-α alone. Coadminister means the administration of interferon-α close enough in time to the administration of the ISNA such that their effects are more than the effects that would be achieved if administering either one on its own at the same dose. Preferably, the effects are at least additive. They also may be administered via different modes, for example such as administering the interferon systemically and administering the ISNA locally.

In certain embodiments involving simultaneous coadministration, the IFN-α and the ISNA may be prepared as a single formulation. In other embodiments involving simultaneous coadministration, the IFN-α and the ISNA may be prepared and administered separately. In this latter instance individual IFN-α and ISNA formulations may be packaged together as a kit with instructions for simultaneous administration. Simlarly, in embodiments calling for consecutive coadministration, individual IFN-α and ISNA formulations may be packaged together as a kit with instructions for their sequential administration.

As used herein, administered locally refers to administration by a route that achieves a local concentration of IFN-α that exceeds the systemic concentration of IFN-α. For example, local administration to a particular lesion or organ could be accomplished by direct injection into the lesion or organ or by direct injection into an afferent blood vessel associated with and supplying the lesion or organ to be treated. In the example local administration to the liver, local administration can be accomplished by injection or infusion into the hepatic artery, the celiac artery, or the portal vein.

In another aspect, the invention involves supplementing IFN-α treatment of a subject in need of IFN-α treatment wherein an effective amount of IFN-α and an isolated ISNA are both administered to the subject. The IFN-α induced by the ISNA supplements the IFN-α directly administered to the subject, thus extending the clinical efficacy of a given dose of IFN-α. Furthermore, because the ISNA-induced IFN-α typically includes a plurality of subtypes, while the directly administered IFN-α typically includes only a single subtype, the range of biological effects afforded by IFN-α treatment is also expanded by the coadministration of the ISNA and the IFN-α.

The invention also involves increasing the efficacy of IFN-α treatment of a subject. This aspect of the invention involves administering to a subject in need of treatment with IFN-α a pharmaceutical composition comprising IFN-α and coadministering to the subject in need of such treatment a pharmaceutical composition comprising an ISNA in an amount which, together with the administered IFN-α, is an effective IFN-α treatment, wherein the efficacy of the IFN-α treatment is greater than the efficacy of administering the same amount of IFN-α in the absence of coadministering the ISNA.

As used herein, a method of enhancing efficacy or increasing efficacy of IFN-α treatment of a subject refers to a method in which the effect of administering a given dose of IFN-α to a subject results in a greater clinical effect than expected or previously observed when using that same dose of IFN-α. In a preferred embodiment the method entails coadministering an amount of ISNA in an amount effective for inducing the production of IFN-α by IPCs. The amount of IFN-α achieved locally or systemically in this method reflects contributions both from administered IFN-α and from induced IFN-α, thereby achieving an enhanced efficacy of IFN-α treatment for a given dose of administered IFN-α. The increased efficacy could be manifested as, for example, a greater degree of response to treatment, a more rapid course of response to treatment, or improved compliance with the treatment regimen.

According to another aspect the invention involves decreasing a dose of IFN-α effective for treating a subject in need of treatment with IFN-α. The method involves administering to a subject in need of treatment with IFN-α a pharmaceutical composition comprising IFN-α, and coadministering to the subject in need of such treatment a pharmaceutical composition comprising an immunostimulatory nucleic acid in an amount which, together with the administered IFN-α; is an effective IFN-α treatment, and wherein the amount of administered IFN-α is less than an amount of IFN-α required in the absence of coadministering the immunostimulatory nucleic acid.

As used herein, a method of decreasing a dose of IFN-α effective for treating a subject refers to a method in which IFN-α is administered to a subject in an amount or with a frequency that is reduced compared to a previously established amount or frequency, while achieving a desired clinical effect in treating a condition of the subject. In a preferred embodiment the dose amount can be reduced by a clinically determined extent to an amount that is, for example, at least 10 percent below the customary or maximum tolerated dose of IFN-α alone. In other more preferred embodiments, the IFN-α dose amount can be reduced by a clinically determined extent to an amount that is at least 20 percent, at least 30 percent, or at least 40 percent below the customary or maximum tolerated dose of IFN-α alone. In a most preferred embodiment, the IFN-α dose amount can be reduced by a clinically determined extent to an amount that is at least 50 percent below the customary or maximum tolerated dose of IFN-α alone. In another preferred embodiment the dose frequency can be reduced by a clinically determined extent to a frequency that is, for example, at least 10 percent below the customary or maximum tolerated dose of IFN-α alone. In other more preferred embodiments, the IFN-α dose frequency can be reduced by a clinically determined extent to a frequency that is at least 20 percent, at least 30 percent, or at least 40 percent below the customary or maximum tolerated dose of IFN-α alone. In a most preferred embodiment, the IFN-α dose frequency can be reduced by a clinically determined extent to a frequency that is at least 50 percent below the customary or maximum tolerated dose of IFN-α alone.

Yet another aspect of the invention involves preventing an IFN-α treatment-related side effect in a subject receiving or in need of treatment with IFN-α. The method entails administering to a subject in need of treatment with iFN-α a pharmaceutical composition comprising IFN-α and coadministering to the subject in need of such treatment a pharmaceutical composition comprising an immunostimulatory nucleic acid in an amount which, together with the administered IFN-α, is an effective IFN-α treatment, and wherein an IFN-α treatment-related side effect is reduced in comparison to the side effect when IFN-α is administered in the absence of coadministering the immunostimulatory nucleic acid.

Assays for IFN-α are well known in the art. These include direct tests, e.g., enzyme-linked immunosorbent assay (ELISA) specific for at least one IFN-α, and indirect tests, e.g., functional tests including NK cell activation/cytotoxicity (Trinchieri G Adv Immunol 47:187-376 (1989) and phenotyping by fluorescence-activated cell sorting (FACS) analysis for class I MHC. Additional specific assay methods well known in the art can be particularly useful in settings where local concentration or local presence of IFN-α is of interest; these methods include, for example, immunohistochemistry, nucleic acid hybridization (e.g., Northern blotting), Western blotting, reverse transcriptase/polymerase chain reaction (RT/PCR), and in situ RT/PCR. A further method, involving detection of intracellular IFN-α by flow cytometry, is disclosed below in Example 6.

As used herein, a method of preventing an IFN-α treatment-related side effect in a subject refers to a method of reducing the incidence or severity of an IFN-α treatment-related side effect experienced by a subject receiving IFN-α treatment. As used herein, an IFN-α treatment-related side effect is a clinical side effect that is induced in a subject as a result of administration of IFN-α to the subject. A number of such side effects have been well documented through clinical experience and clinical trials. Such side effects are frequently dose-limiting in a subject. Systemic IFN-α treatment-related side effects most commonly encountered include: influenza-like syndrome, fever, headache, chills, myalgia, fatigue, anorexia, nausea, vomiting, diarrhea, depression, hypothyroidism, neutropenia, and anemia. In a preferred embodiment the IFN-α treatment-related side effect is reduced sufficiently to promote greater compliance with IFN-α treatment. In another preferred embodiment the IFN-α treatment-related side effect is reduced sufficiently to permit resumption of IFN-α treatment otherwise precluded by the side effect. In another preferred embodiment the IFN-α treatment-related side effect is reduced sufficiently to permit an intensification of IFN-α treatment.

In another aspect of the invention, a second method is provided for enhancing efficacy of IFN-α treatment in a subject in need of such treatment. This involves the steps of administering to a subject in need of such treatment an amount of a pharmaceutical composition comprising IFN-α effective for treating a condition of the subject, isolating natural interferon-producing cells (IPCs) from a donor, contacting the isolated IPCs *ex vivo* with an amount of a pharmaceutical composition comprising an immunostimulatory nucleic acid effective for inducing the IPCs to release IFN-α and administering the contacted cells to the subject. The donor and the subject can be a single individual or they can be different individuals. In certain embodiments the contacted cells are administered to the subject in a local fashion, e.g., via injection or infusion into a blood vessel supplying a target tissue to be treated. The method according to this aspect of the invention can optionally include contacting the isolated IPCs with an antigen. In certain embodiments the method may also include contacting the isolated IPCs with a growth factor which the IPCs do not produce themselves. Such a growth factor not produced by IPCs can include, for example, IL-3 or GM-CSF, and would exclude IL-8 and TNF-α.

As used herein, the term growth factor refers to a soluble signaling factor that induces a responsive cell type to undergo maturation and mitosis. Categories of growth factors include a number of cytokines, growth factors *per se,* and hormones. Specific examples of growth factors include, without limitation, IL-1, IL-2, IL-3, IL-6, GM-CSF, G-CSF, PDGF, TGF-β, NGF, IGFs, growth hormone, erythropoietin, thrombopoietin, and the like. In addition to naturally occurring growth factors, growth factor analogs and growth factor derivatives such as fusion proteins can be used for the purposes of the invention.

As used herein, the term natural interferon-producing cell (IPC) refers to a specialized type of leukocyte that is the chief producer of IFN-α in response to enveloped viruses, bacteria, and tumors. IPCs are lineage negative (lin-)/CD4+/MHC class II+ cells that are present in low frequency in peripheral blood mononuclear cells (PBMCs) and in tonsillar tissue. Siegal FP et al. Science 284:1835-7 (1999); Grouard G et al. J Exp Med 185:1101-11 (1997). The frequency of IPCs in PBMCs in normal individuals varies between 0.2 and 0.6 percent. They are characterized by the absence of lineage markers CD3 (T cells), CD14 (monocytes), CD19 (B cells) and CD56 (NK cells), by the absence of CD11c, and by their expression of CD4, CD123 (IL-3 receptor α, IL-3Rα) and MHC class II. Grouard G et al. J Exp Med 185:1101-11 (1997); Rissoan M-C et al. Science 283:1183-86 (1999); Siegal FP et al. Science 284:1835-7 (1999); Cella M et al. Nat Med 5:919-23 (1999).

As used herein, isolating IPCs from a subject refers to a process of removing from the subject a body fluid or tissue containing IPCs and enriching for the IPCs from the body fluid or tissue to an extent that at least 1 percent of the cells are IPCs. In a most preferred embodiment at least 99 percent of cells are IPCs. In another preferred embodiment at least 95 percent of cells are IPCs. In another preferred embodiment at least 90 percent of cells are IPCs. In another preferred embodiment at least 80 percent of cells are IPCs. In another preferred embodiment at least 70 percent of cells are IPCs. In another preferred embodiment at least 60 percent of cells are IPCs. In another preferred embodiment at least 50 percent of cells are IPCs. In another preferred embodiment at least 40 percent of cells are IPCs. In another preferred embodiment at least 30 percent of cells are IPCs. In another preferred embodiment at least 20 percent of cells are IPCs. In another preferred embodiment at least 10 percent of cells are IPCs. In another preferred embodiment at least 5 percent of cells are IPCs. The enriching can be achieved by a series of selecting steps which can include, for example, a negative selection of lineage-positive cells by contacting cells with magnetic beads conjugated with antibodies specific for lineage markers (i.e., anti-CD3, anti-CD11c, anti-CD14, anti-CD16, anti-CD19, anti-CD56) and then passing the contacted cells over a depletion column in the presence of a strong magnetic field; a positive selection step involving contacting the cells passing through the depletion column with microbead-conjugated anti-CD4 and passing the contacted cells over a positive selection column; and further enhancement for IPCs by fluorescence-activated cell sorting (FACS) using anti-CD123 and anti-MHC class II. Other methods can be employed to equal effect, as will be appreciated by those skilled in the art, provided they result in the isolation or enrichment of (lin-)/CD4+/CD123+/MHC class II+ interferon producing cells to an extent that at least 1 percent of the viable cells are IPCs.

In yet another aspect of the invention, a method is provided for supporting the survival of natural interferon-producing cells (IPCs) *in vitro.* The method involves isolating IPCs from a subject (as described above), culturing the IPCs in a sterile medium suitable for tissue culture, and contacting the IPCs *in vitro* with an amount of immunostimulatory nucleic acid effective to support the growth of the IPCs in the absence of interleukin 3 (IL-3). In a preferred embodiment, the IPCs are precursor type 2 dendritic cells (pDC2s; plasmacytoid monocytes). Siegal FP et al. Science 284:1835-7 (1999). The IPCs can be cultured under suitable tissue culture conditions either with or, more notably, without exogenous IL-3 and/or GM-CSF.

As used herein, a method of supporting survival of interferon-producing cells (IPCs) *in vitro* refers to providing a factor or inducing a signal that promotes the viability of IPCs placed in *in vitro* culture. For example, in the absence of IL-3, normally most IPCs die within three days of being placed into cell culture. Addition of IL-3 to IPCs will support the survival of IPCs in culture. According to this aspect of the invention, IL-3 is not required for IPC survival *in vitro* if the IPCs are contacted with an effective amount of ISNA.

The invention in another aspect provides a method for stimulating isolated interferon-producing cells (IPCs) *in vitro.* The method includes the steps of isolating IPCs from a subject (described above), culturing the IPCs in a sterile medium suitable for tissue culture, and contacting the IPCs *in vitro* with an amount of immunostimulatory nucleic acid effective to induce secretion of at least one type I interferon. In a preferred embodiment the type I interferon induced by the method is IFN-α. As described above, preferred IPCs are precursor type 2 dendritic cells (pDC2s; plasmacytoid monocytes). Siegal FP et al. Science 284:1835-7 (1999). Importantly, the IPCs can be stimulated in culture in the absence of GM-CSF and without viral infection by, e.g., Sendai virus, HSV or influenza virus. Activation can be assayed using methods well known in the art, including FACS analysis of the cell surface activation marker CD80 and ELISA or bioassay (e.g., protection of fibroblasts against vesicular stomatitis virus) for type I IFN.

As used herein, a method of stimulating isolated interferon-producing cells (IPCs) *in vitro* refers to providing a factor or inducing a signal that results in a change in IPC size, morphology, or expression of a cell surface antigen, transcript, or a secreted product that is not characteristic of IPCs in the absence of the factor or signal. Freshly isolated IPCs, in the absence of a signal provided by viral infection, CD40L ligation, or GM-CSF, display a smooth round lymphoid morphology with a diameter of 8-10 µm and do not express CD80 or CD86 on their cell surface. Grouard G et al. J Exp Med 185:1101-11 (1999). Similarly, freshly isolated IPCs do not secrete IFN-α in large amounts. Siegal FP et al. Science 284:1835-7 (1999). In contrast, IPCs exposed to IL-3 in vitro develop pseudopods and a veiled morphology, express CD80 and CD86 on their surface, and secrete large amounts of type I IFN (IFN-α and IFN-β) when exposed to ultraviolet-irradiated herpes simplex virus, Sendai virus, or heat-killed *Staphylococcus aureus.* Grouard G et al. J Exp Med 185:1101-11 (1999); Siegal FP et al. Science 284:1835-7 (1999). According to this aspect of the invention, ISNA can be used in place of viral infection, CD40L ligation, or GM-CSF to induce a signal effective for stimulating isolated IPCs in vitro.

The invention further involves treating a subject to activate interferon-producing cells (IPCs) of the subject. The method involves isolating IPCs from a subject in need of such treatment, culturing the IPCs *in vitro*, contacting the IPCs *in vitro* with an effective amount of an isolated immunostimulatory nucleic acid, and returning the contacted IPCs to the subject. IPCs are isolated from a subject as described above and placed into culture under suitable in vitro cell culture conditions. Such culture conditions may optionally include provision of exogenous growth factor, including IL-3 or GM-CSF. However, IL-3 or GM-CSF may not be required for the purposes of the method. According to this method, the subject may be treated without direct administration of a pharmaceutical preparation of IFN-α. Activation of the IPCs can be assayed as described above, with reference made to IPCs similarly obtained and cultured but not contacted with ISNA.

In yet another aspect, the invention involves stimulating production of a plurality of type I IFN subtypes. The method involves contacting IPCs with an amount of immunostimulatory nucleic acid effective to induce secretion of at least two type I interferons. In one embodiment the IPCs are brought into contact with ISNA in vivo. In another embodiments the IPCs are isolated and/or are contacted with ISNA in vitro under suitable cell culture conditions. Various other embodiments result in the induction of at least three, at least four, at least five, at least six, at least seven, and at least eight subtypes of type I IFN. The various subtypes can be determined using methods well described in the art and known to those of skill in the art, e.g., subtype-specific ELISA, amino-terminal sequencing, and mass spectrometry (MS).

Matrix-assisted laser desorption/ionization time-of-flight (MALDI TOF)-MS and electrospray ionization (ESI)-MS are now standard methods used for identifying peptides available in femtomole quantities. Mann M and Talbo G Curr Opin Biotechnol 7:11-19 (1996); Mann M and Wilm M Trends Biochem Sci 20:219-24 (1995); Mann M et al. Anal Chem 61:1702-8 (1989). Individual bands were cut out of the polyacrylamide gel, cleaved with trypsin and then eluted to yield peptide fragments that are subjected to MALDI TOF-MS or ESI-MS analysis. The combination of mass/charge data from the MS, cleavage site specificity of the trypsin digest, and peptide sequence data permitted identification of individual proteins and peptides. MALDI TOF-MS analysis gives a mass fingerprint of the cleaved and analyzed proteins. The fingerprint is useful only for scanning against a database of calculated peptide masses corresponding to fully sequenced proteins. The ESI-MS analysis is more difficult, but it permits identification based on comparison to either complete or partial sequence data. Mass accuracies for either method can exceed 0.01 percent, i.e., 1 Da per 10 kDa.

In another aspect the invention relates to the discovery that type I IFN induces activation and proliferation of γδ T cells. The γδ T cells are antigen-specific T cells in a preactivated stage which respond to common phosphate-containing non-peptide antigens. Examples of γδ T cell antigens include phosphate-containing non-peptide molecules from heat-killed mycobacteria; isopentenyl pyrophosphate (IPP) and related prenyl pyrophosphate derivatives; monoethyl phosphate; and γ-monoethyl derivatives of nucleoside and deoxynucleoside triphosphates. Tanaka Y et al. Nature 375:155-8 (1995). Previous studies showed that γδ T cells can secrete a variety of lymphockines and mount cytolytic responses. For example, exposure of γδ T cells to these phosphate-containing non-peptide antigens stimulates IFN-γ production in the absence of APC. While clearly belonging to the T-cell lineage, human γδ T cells are distinctly different from α/β T cells, and they share several features with NK cells. The observations that γδ cells accumulate in lesions caused by mycobacterial infections, respond to virally infected cells in a virus-nonspecific manner, require neither antigen-processing nor antigen-presenting cells, and that they are preferentially located in various epithelia, together suggest that γδ cells may be responsive to pattern recognition and responsible for a first line of defense.

It was discovered according to this aspect of the invention that CpG ODN in combination with IPP synergistically induced activation of human γδ T cells present within PBMC, as measured by the production of IFN-γ and perforin. In addition, it was also discovered according to this aspect of the invention that CpG ODN in combination with IPP synergistically induced proliferation of human γδ T cells present within PBMC. These effects were abrogated by isolating γδ T cells from PBMC or by addition of neutralizing antibodies to type I IFN, and they were reproduced by the addition of recombinant type I IFN. Notably, ODN 2216 and 1585, both strong inducers of type I IFN, were more potent in their effects on γδ T cells than ODN 2006.

In humans Th1 responses are driven by IL-12 and/or IFN-γ. IL-12 and IFN-α/β both promote IFN-γ synthesis in T cells and NK cells. It was previously known that IL-12 promotes γδ T cells to secrete IFN-γ. Since IFN-β has been described to downregulate IL-12 production, experiments were performed to study the effect on IL-12 production exerted by ISNA which induce type I IFN. The results of these experiments (Example 13) demonstrate that certain CpG ODN suppress CD40-dependent IL-12p70 production by an IFN-α/β-mediated negative feedback mechanism on IL-12p40 mRNA production. Thus the interaction of T cells and antigen-presenting cells via CD40L leads to a cytokine milieu dominated by IL-12 or IFN-α/β. Although both promote Th1 responses, CpG ODN which are better inducers of B cell activation than of type I IFN may be superior for priming naive T cells, and conversely CpG ODN which are strong inducers of type I IFN may have higher activity to support preactivated and memory T cells.

According to another aspect of the invention, an interferon composition for administration to a subject is provided. The composition includes recombinant or natural interferon in a container for administration to a subject. The amount of the interferon in the container is at least about 10 percent less than the maximum tolerated dose (MTD). Preferably the amount of interferon in the container is at least about 20 percent below the MTD, at least 30 percent below the MTD, at least 40 percent below the MTD, or even at least 50 percent below the MTD. In other embodiments, the amount of interferon in the container is at least about 20 percent below, 30 percent below, 40 percent below, or even 50 percent below the clinically established effective dose. The container also can include an ISNA.

In still another aspect of the invention, kits for administration of interferon and an ISNA to a subject are provided. Referring to **Figure 18** depicting a kit **11,** the kits include a container **19** containing a composition **17** which includes IFN-α and instructions **21** for administering the interferon to a subject in need of such treatment in an amount which is at least about 10 percent less than the maximum tolerated dose (MTD), 20 percent less than the MTD, 30 percent less than the MTD, 40 percent less than the MTD, or 50 percent less than the MTD. The kit **11** can include, in the same container or in a separate container **19,** an ISNA. The kit also can include instructions **21** for treating a subject with a condition susceptible to treatment with IFN-α. Examples of such conditions, proliferative and viral, are as described above. Kit **11** also includes a box-like package **15.**

The present invention is further illustrated by the following Examples, which in no way should be construed as further limiting.

### EXAMPLES

### Example 1. Isolation and characterization of IPCs.

Peripheral blood mononuclear cells (PBMCs) contain a total of 0.2 to 0.4 percent IPCs, which are characterized by the lack of lineage markers (CD3, CD14, CD16, CD19, CD20, CD56) and can be distinguished from other lineage-negative cells by the expression of CD4, CD123 (IL-3Rα), and MHC class II.

IPCs were isolated from peripheral blood by using the VARIOMACS technique (Milteny Biotec, Auburn, CA) and the technique previously described. O'Doherty U et al. J Exp Med 178:1067-76 (1993). PBMCs were obtained from buffy coats of healthy blood donors by Ficoll-Paque density gradient centrifugation (Histopaque-1077, Sigma) as previously described. Hartmann G et al. Antisense Nucleic Acid Drug Dev 6:291-9 (1996). Monoclonal antibodies directed to CD3 (UCHT1), CD14 (M5E2), and CD19 (B43) were purchased from PharMingen (San Diego). PBMCs were incubated with anti-CD3, CD14, CD16, CD19, and CD56 antibodies conjugated to colloidal superparamagnetic microbeads and passed over a depletion column in a strong magnetic field. Resulting lineage-negative (lin-) cells in the flow-through were incubated with a microbead-conjugated antibody to CD4 and passed over a positive selection column. Further purification of IPCs to >99 percent from lin-/CD4+ cells was achieved by fluorescence-activated cell sorting (FACS) using phycoerythrin (PE)-labeled anti-CD123 and FITC-labeled anti-MHC class II.

Surface antigen staining was performed as previously described. Hartmann G et al. J Pharmacol Exp Ther 285:920-8 (1998). Monoclonal antibodies to MHC class II (HLA-DR, Immun-357) and CD80 (MAB104) were purchased from Immunotech (Marseilles, France). All other antibodies were purchased from PharMingen (San Diego): mAbs to CD3 (UCHT1), CD14 (M5E2), CD19 (B43), and CD86 (2331 (FUN-1)). FITC-labeled IgG1,κ (MOPC-21) and phycoerythrin-labeled IgG2b,κ were used to control for specific staining. Lyons AB and Parish CR J Immunol Methods 171:131-7 (1994).

Flow cytometric data were acquired on a FACScan (Becton Dickinson Immunocytometry Systems, San Jose, CA). Spectral overlap was corrected by appropriate compensation. Analysis was performed on viable cells within a morphologic gate (forward scattering (FSC), side scattering (SSC), >94 percent of cells MHC class II positive and lineage marker negative). Data were analyzed with the computer program FLOWJO (version 2.5.1, Tree Star, Stanford, CA).

*Results.* Viability as determined by trypan blue exclusion was >95 percent. Freshly isolated IPCs are negative for costimulatory molecules CD80 and CD86. **Figure 1** depicts FACS analyses of IPCs isolated from PBMCs with magnetic beads and flow cytometry. From left to right are shown: selection of lin-/MHC class II+ cells from PBMCs; further selection of CD123+/MHC class II+ cells from lin-/MHC class II+ cells; and characterization of freshly isolated lin-/MHC class II+/CD123+ IPCs as CD80-.

### Example 2. CpG oligonucleotide supports the survival and activation of IPCs in vitro.

The majority of freshly isolated IPCs die within 3 days if not incubated in the presence of IL-3 or GM-CSF. Remaining live cells are not activated or are only weakly activated. If CpG oligonucleotide but no other growth factors are added to the cell culture of IPCs, IPCs survive and become highly activated as shown by their increased expression of costimulatory molecules (e.g., CD80, **Figure 2**).

Freshly isolated IPCs (see Example 1) were suspended in RPMI 1640 culture medium supplemented with 10 percent (vol/vol) heat-inactivated (56°C, 1 h) FCS (HyClone), 1.5 mM L-glutamine, 100 units/ml penicillin, and 100 µg/ml streptomycin (all from GIBCO/BRL) (complete medium). All compounds were purchased endotoxin-tested. Freshly prepared IPCs (final concentration 5x10⁵ cells per ml) were cultured for two days in complete medium alone or complete medium supplemented with 6 µg/ml phosphorothioate CpG ODN 2006 (5'-tcgtcgttttgtcgttttgtcgtt-3'; SEQ ID NO: 147), 100 ng/ml LPS (from *Salmonella typhimurium,* Sigma catalog no. L2262), 800 units/ml GM-CSF (1.25 x 10⁴ units/mg, Genzyme), or CpG oligonucleotide in combination with GM-CSF. Expression of CD80 and MHC class II on IPCs was examined by flow cytometry (see Example 1).

*Results.* Representative results of five independent experiments are depicted in Figure 2. A single addition of CpG ODN 2006 (SEQ ID N0:147, 2 µg/ml) to freshly prepared IPCs was superior to GM-CSF (800 units/ml) in promoting cell survival (74.3 percent ± 5.2 percent vs. 57.1 percent ± 2.3 percent). The combination of GM-CSF and CpG ODN 2006 (SEQ ID NO:147) further increased the number of viable cells (81.0 percent ± 6.7 percent). Freshly isolated IPCs placed into culture for two days without IL-3 or GM-CSF remained unactivated, as indicated by the lack of staining for CD80, even when LPS was added to the media. Addition of GM-CSF induced CD80. Addition of CpG ODN 2006 (SEQ ID NO:147, 6 µg/ml) activated IPCs to an even greater degree than GM-CSF (800 units/ml). Further activation of IPCs occurred when GM-CSF and CpG oligonucleotide were present together. This demonstrates that CpG substitutes for IL-3 and GM-CSF in supporting the survival of IPCs. LPS did not contribute either to survival or activation of IPCs (**Figure 2**).

### Example 3. CpG oligonucleotide, but not poly IC, activates IPCs in vitro.

IL-3 provides excellent survival of IPCs but does not activate IPCs. When IL-3 was combined with CpG oligonucleotide, expression of CD80 increased by 5 to 20-fold (**Figure 3**). Poly IC, another polynucleotide with well known immunostimulatory functions on myeloid cells (dendritic cells, macrophages), did not stimulate IPCs.

Freshly prepared IPCs (see Example 1, final concentration 3x10⁵ cells per ml) were cultured for three days in complete medium (see Example 2) supplemented with 10 ng/ml IL-3. Cultures of IPCs were then continued for a further 24 hours (a) without any additional supplements, (b) following the addition of 6 µg/ml CpG ODN 2006 (SEQ ID NO: 147), and (c) following the addition of 10 µg/ml poly IC. Forward scattering (FSC), side scattering (SSC), and expression of CD80 and MHC class II on IPCs were examined by flow cytometry (see Example 1).

*Results.* Representative results of three independent experiments are shown in **Figure 3**. IPCs cultured in complete media supplemented with IL-3 and CpG ODN 2006 (SEQ ID NO:147, 6 µg/ml) were larger and more granular than IPCs cultured in complete media containing IL-3 alone or in complete media supplemented with IL-3 and poly IC. In addition, IPCs cultured in complete media supplemented with IL-3 and CpG ODN 2006 (SEQ ID NO:147, 6 µg/ml) were more highly activated than IPCs cultured in complete media containing IL-3 alone or in complete media supplemented with IL-3 and poly IC. This demonstrates that CpG oligonucleotide activates IPCs *in vitro.*

### Example 4. CpG oligonucleotide induces IFN-α, production by IPCs.

Induction of type I interferons by CpG in whole PBMC has been previously demonstrated. Sun S et al. J Exp Med 188:2335-42 (1998). Here is shown for the first time that IFN-α is induced in IPCs by CpG oligonucleotide within 48 hours using an ELISA specific for 9 out of 10 subspecies of IFN-α tested.

Freshly prepared IPCs (see Example 1, final concentration 3x10⁵ cells per ml) were cultured for two days in complete medium (see Example 2) supplemented with 10 ng/ml IL-3 and 800 units/ml GM-CSF (1.25 x 10⁴ units/mg, Genzyme). Half of the cultures were supplemented with 6 µg/ml CpG ODN 2006 (SEQ ID NO:147). IFN-α was measured in the supernatant using a combination of separate ELISAs specific for IFN-α (human IFN-α multispecies ELISA, PBL Biomedical Laboratories, New Brunswick, NJ) and for IFN-β (PBL Biomedical Laboratories, New Brunswick, NJ) performed according to the supplier's instructions. The multispecies IFN-α ELISA has a range from 100 to 5000 pg/ml, detects all of the human IFN-α subtypes except IFN-αF, and does not detect IFN-β or IFN-γ. The IFN-β ELISA has a range of 250-10,000 pg/ml.

*Results.* Representative results of three independent experiments appear in **Figure 4**. IPCs cultured for 48 hours in complete media supplemented with 10 ng/ml IL-3, 800 units/ml GM-CSF and 6 µg/ml CpG ODN 2006 (SEQ ID NO:147) were strongly induced to secrete IFN-α compared to similar cultures without added CpG oligonucleotide. This result demonstrates CpG oligonucleotide induces human IPCs to secrete multiple subspecies of IFN-α. This result also indicates CpG oligonucleotide would permit the *in vitro* production of natural interferons using a permanent cell line derived from IPCs.

### Example 5. Identification of CpG ODN with IFN-α and IFN-β inducing activity.

The 24mer CpG ODN 2006 (SEQ ID NO:147) which contains three consecutive "human" CpG motifs (5' GTCGTT 3') is one of the most potent CpG sequences to activate human B cells. Hartmann G et al. J Immunol 164:944-53 (2000); Hartmann G et al. J Immunol 164:1617-24 (2000). In contrast to other microbial stimuli such as LPS and poly (I:C), ODN 2006 strongly promotes survival and activation of pDC precursors. However, compared to its strong ability to activate NK cells, the ability of ODN 2006 to induce type I IFN in pDC is relatively poor.

In order to test the hypothesis that other CpG ODN may activate NK cells by inducing type I IFN in pDC, a panel of CpG ODN with known NK cell activity were tested for their capability to stimulate IFN-α production in PBMC. The panel of CpG ODN included the following, where lower case letters signify phosphorothioate linkages, upper case letters signify phosphodiester linkages, and m signifies 7-deaza-guanosine:

| | | |
|---|---|---|
| tcgtcgttttgtcgttttgtcgtt | ODN 2006 | (SEQ ID NO:147) |
| ggGGTCAACGTTGAgggggG | ODN 1585 | (SEQ ID NO:1) |
| gmGGTCAACGTTGAgggmggG | ODN 2197 | (SEQ ID NO:148) |
| ggGGAGTTCGTTGAgggggG | ODN 2198 | (SEQ ID NO:149) |

All ODN were dissolved in TE buffer (10 mM Tris-HCI, 1 mM EDTA, pH 8) at a concentration of 20 mg/ml. Aliquots diluted in PBS (0.4 mg/ml) were stored at -20 °C and thawed prior to use. Pyrogen-free reagents were used for all dilutions. ODN were tested for endotoxin using the LAL assay (BioWhittaker, Walkersville, MD; lower detection limit 0.1 EU/ml).

Freshly isolated PBMC were incubated with CpG ODN (3 µg/ml) for 48 hours. IFN-α was measured in the supernatant by an ELISA which detects 10 of 13 isoforms of IFN-α. Among all sequences initially examined, CpG ODN 1585 (SEQ ID NO:1) showed the highest activity to induce IFN-α in PBMC. ODN 1585 is a chimeric ODN (mixed phosphorothioate-phosphodiester backbone) with poly G on both ends and a central CpG-dinucleotide within a 10mer palindrome. Hartmann G et al. *J Pharmacol Exp Ther* 285:920 (1998). ODN 1585 stimulated IFN-α in the nanogram range (1.3 ± 0.4 ng/ml; n=7) as compared to ODN 2006 which did not induce significant amounts of IFN-α in PBMC (0.021 ± 0.015 ng/ml; n=8) (**Figure 5**). The control ODN 2197 (7-deaza-guanosine substitutions in poly G ends, unable to form G tetrads) and ODN 2198 (CG and poly G ends but no palindrome) were essentially inactive (**Figure 5**). Based on the sequence of ODN 1585, a new panel of CpG ODN were then designed. ODN 2216 (ggGGGACGATCGTCgggggG; SEQ ID NO:7), which contains poly G ends and three CG dinucleotides within a palindrome, is one example among several sequences with pronounced IFN-α-inducing activity in PBMC (23.7 ± 5.2 ng/ml; n=7).

CpG ODN stimulated IFN-α production in a concentration-dependent manner (**Figure 6**). The activities of ODN 2216 and ODN 1585 were tested for concentrations up to 12 µg/ml, confirming that the higher potency of ODN 2216 was not a concentration-dependent effect. As little as 0.4 µg/ml ODN 2216 induced considerable amounts of IFN-α (0.7 ng/ml) in PBMC, whereas ODN 2006 and the GC control to ODN 2216 (ggGGGAGCATGCTCgggggG; ODN 2243; SEQ ID NO:150) had no effect even at higher concentrations. Maximum activity was reached at 3 µg/ml. Production of IFN-α could already be detected after 6 hours of incubation (0.2 ng/ml) and reached a plateau after 48 hours.

The natural interferon-producing cell in PBMC upon virus infection is identical to the pDC precursor with a frequency of less than 0.5 %. PBMC were enriched 10- to 70-fold for pDC precursors by depletion of T-cells, NK-cells and monocytes (2 - 18 % CD123⁺⁺ pDC; 3 - 10 % CD11c⁺ myeloid DC (mDC); 50 - 90 % B-cells; n=4). To increase the viability of pDC, IL-3 was added to all samples. This procedure resulted in a 30- to 60-fold increase in IFN-α production (up to 428.3 ± 56.8 ng/ml with ODN 2216; n=4; **Figure 7**, upper panel, left side). The most active CpG ODN in PBMC were also the most active in the samples enriched for pDC. ODN 2006, ODN 2197, or IL-3 alone induced only little IFN-α (means: 0.8, 0.4 and 0.6 ng/ml respectively, n=4). Poly (I:C) (7 µg/ml), which mimics double-stranded RNA and which is known to induce IFN-α in macrophages, was an even weaker stimulus of IFN-α in cells enriched for pDC (0.3 ng/ml, not in figure). The same CpG ODN which induced high amounts of IFN-α also stimulated IFN-β production (up to 2.8 ± 0.8 ng/ml, n=4; **Figure 7**, lower panel, left side). Considering that IFN-β represents a single isoform and that IFN-α consists of at least 13 isoforms, remarkable amounts of IFN-β are produced.

To determine whether cellular uptake of CpG ODN was critical for the induction of IFN-α and IFN-β by CpG ODN, the effects of the cationic lipid lipofectin were examined (**Figure 7**, upper and lower panel, right side). Positively charged cationic lipids form complexes with negatively charged ODN, which increase cellular uptake of ODN. Lipofectin enhanced the production of IFN-α and IFN-β induced by CpG ODN (up to 786 ng/ml IFN-α, n=3; and up to 9 ng/ml IFN-β, n=3). The increase was seen for all CpG ODN examined but was most prominent for ODN 1585 (20-fold).

### Example 6. CpG ODN-induced IFN-α is exclusively produced by plasmacytoid dendritic precursor cells.

To examine which cell type within PBMC produces IFN-α in response to CpG ODN, a protocol was developed which allowed the detection of intracellular IFN-α on a single cell basis by flow cytometry. PBMC were incubated with ODN 2216 (SEQ ID NO:7) or ODN 2006 (SEQ ID NO: 147) at 3 µg/ml. After five hours, cells were harvested and intracellular staining of IFN-α was performed.
For the analysis of intracellular IFN-α, no brefeldin A was added during the incubation period to block protein secretion. PBMC were harvested (approximately 600,000 cells/tube), incubated with anti-CD123-biotin (Pharmingen), washed in PBS (400 g, 5 minutes, 4°C) and stained with Streptavidin-APC (Pharmingen), FITC-conjugated anti-lineage cocktail (consisting of anti-CD3, -CD14, -CD16, -CD19, -CD20, and -CD56; Becton Dickinson), and anti-HLA DR-PerCP (Becton Dickinson). Then cells were washed in PBS, resuspended in 100 µl of fixation buffer A (Fix and Perm Kit, Caltag Laboratories, Burlingame, CA) and incubated at room temperature for 15 min. Cells were washed in 2 ml PBS again and then resuspended in 100 µl permeabilization buffer B (Fix and Perm Kit). 4 µg/ml mouse anti-human IFN-α mAb (MMHA-11, PBL Biomedical Laboratories) was added. PE-labeled mouse IgGl (MOPC-21, Pharmingen) was used as control antibody. After 15 min incubation at room temperature in the dark, cells were washed in 2 ml PBS. For detection of intracellular IFN-α cells were again resuspended in 100 µl permeabilization buffer B (Fix and Perm Kit) and stained with PE-labeled rat anti-mouse Ig κ light chain (R8-140, Pharmingen) as secondary antibody. After washing in PBS, cells were analyzed by four-color flow cytometry on a Becton Dickinson FACS Calibur equipped with two lasers (excitation at 488 nm and 635 nm). Spectral overlap was corrected by appropriate compensation and gates were set using isotype control antibodies. Analysis was performed on viable cells within a morphologic gate (FSC, SSC, > 97 % of viable cells as confirmed by propidium iodide staining). Data were analysed using CELLQUEST (Becton Dickinson) or FLOWJO software (version 2.5.1, Tree Star, Inc., Stanford, CA).

*Results.* As shown in **Figure 8A**, lineage⁺ and lineage⁻ (lin⁺ and lin⁻) cells were defined by lineage marker expression and forward scatter characteristics. After stimulation with ODN 2216 intracellular IFN-α was not detectable in lin⁺ cells which contain monocytes and macrophages as potential IFN-α producing cells **(****Figure 8C****).** Within the lin⁻ cells, which contain mainly pDC and mDC, a distinct population with intermediate HLA DR (MHC class II) expression stained positive for IFN-α **(****Figure 8D****).**

Within the lin population, mDC and pDC can be distinguished by their HLA DR/CD123 phenotype **(****Figure 8B****)**. The mDC are CD123^{+/-} and HLA DR⁺⁺ (gate II); pDC are CD123⁺⁺ and HLA DR⁺ (gate III). The CD123⁺⁺/HLA DR⁻ population are basophils. **Figure 9A** shows intracellular staining for IFN-α in lin⁻/HLA DR⁺ cells. IFN-α was exclusively produced by pDC in response to ODN 2216 but not in response to ODN 2006. Among pDC 46 % stained positive for IFN-α, corresponding to a frequency of 0.25 % cells within PBMC which produced IFN-α at this particular time point of staining. In three other experiments frequencies of IFN-α producing cells in response to ODN 2216 were 0.08 %, 0.05 % and 0.22 % of PBMC (16 %, 8 % and 63 % of pDC).

In contrast to the results with pDC, in mDC no IFN-α synthesis was detected after stimulation with ODN 2006 or ODN 2216.

Thus, pDC were the only cells within PBMC which produced IFN-α in response to CpG ODN. Of note, intracellular IFN-α staining was performed without brefeldin A. Thus the amount of IFN-α detected represented the actual IFN-α production at the time point of harvest and not the cumulative amount of IFN-α over several hours. When brefeldin A was added during incubation to block protein secretion (standard protocol for intracellular cytokine staining), no IFN-α-producing cells could be detected.

### Example 7. Both IFN-α-inducing and non-IFN-α-inducing CpG ODN stimulate early TNF production in plasmacytoid dendritic cells.

It has been reported that pDC produce TNF-α in response to IL-3 and thus promote their own maturation in an autocrine fashion. Hartmann G et al. Antisense Nucleic Acid Drug Dev 6:291-9 (1996). The intracellular accumulation of TNF-α in pDC therefore was examined in response to different CpG ODN (**Figure 9B**). PBMC were incubated for five hours with with ODN 2216 (SEQ ID NO:7) or ODN 2006 (SEQ ID NO:147) at 3 µg/ml in the absence of IL-3. Brefeldin A (1 µg/ml, Sigma) was added during the five hour stimulation period to block cytokine secretion. PBMC were harvested (approximately 600,000 cells/tube), incubated with anti-CD 123-biotin, washed in PBS (400 g, 5 minutes, 4°C) and stained with Streptavidin-APC (Pharmingen), FITC-conjugated anti-lineage cocktail and anti-HLA DR-PerCP (Becton Dickinson). Then cells were washed in PBS, resuspended in 100 µl of fixation buffer A (Fix and Perm Kit, Caltag Laboratories, Burlingame, CA) and incubated at room temperature for 15 min. Cells were washed in 2 ml PBS again and then resuspended in 100 µl permeabilization buffer B (Fix and Perm Kit). 5 µg/ml PE-labeled mouse anti-human TNF-α mAb (MAb11, Pharmingen) was added as primary antibody. PE-labeled mouse IgG1 (MOPC-21, Pharmingen) was used as control antibody. After 15 min incubation at room temperature in the dark, cells were washed in 2 ml PBS and then analyzed by four-color flow cytometry as described above.

*Results.* In contrast to IFN-α, the percentage of TNF-α producing pDC in response to ODN 2006 and ODN 2216 was similar (59 % versus 56 %). Two other experiments showed comparable results (26 vs 22 % and 8 vs 6 % TNF-α⁺ pDC with ODN 2006 and ODN 2216, respectively). The production of TNF-α per cell (MFI, mean fluorescence intensity) was consistently higher with ODN 2216 than with ODN 2006 (**Figure 9B**). No TNF-α was detected in mDC. Lineage⁺ cells did not produce significant amounts of TNF-α in response to either ODN 2006 or ODN 2216.

### Example 8. Upregulation of costimulatory molecules on pDC in response to IFN-α-inducing CpG ODN.

In previous studies ODN 2006 was reported to stimulate the expression of costimulatory molecules on CD4⁺ peripheral blood DC. Zhong RK et al. J Immunol 163:1354 (1999). In order to examine the capacity of different CpG ODN to upregulate CD80 and CD86 on pDC, PBMC were depleted of monocytes, T cells and NK cells, and the remaining cells were stimulated with different CpG ODN in the presence of IL-3. After 48 hours, expression of CD80 and CD86 was examined on pDC (CD123⁺⁺/HLA DR⁺) by three-color flow cytometry. B-cells (CD 123⁻/HLA DR⁺) and mDC (CD123^{+/-}/HLA DR⁺⁺) were excluded from the analysis. As shown in **Figure 10****,** expression of CD86 on pDC was increased by ODN 2006 (SEQ ID NO: 147) as well as by ODN 1585 (SEQ ID NO: 1) and ODN 2216 (SEQ ID NO:7). The effect of ODN 1585 was abolished by substitution of the poly G tails with 7-deaza-guanosine (ODN 2197; SEQ ID NO:148). The non-palindrome CpG ODN 2198 (SEQ ID NO:149) was inactive. Upregulation of CD80 and HLA DR was similar to CD86. Increased expression of CD80 and CD86 in response to the weakly IFN-α-inducing ODN 2006 was detectable after 6 hours. In contrast, ODN 1585 and ODN 2216 showed a delayed response, starting later than 12 hours. For both strongly IFN-α-inducing CpG ODN (ODN 1585, ODN 2216) and weakly IFN-α-inducing CpG ODN (ODN 2006), a plateau was reached after 48 hours. At later time points, identification of pDC among PBMC by flow cytometry was hampered by downregulation of CD 123 during cell culture.

### Example 9. Stimulation of IFN-α and IFN-β production by CpG ODN is a direct effect on plasmacytoid dendritic cells and is partially blocked by anti-CD4 magnetic beads.

In order to examine whether CpG ODN induce IFN-α production directly, purified pDC, rather than pDC-enriched PBMC were studied. PBMC were depleted of monocytes, T-cells, NK cells and B cells. CD123⁺⁺ and HLA DR⁺ pDC were sorted by FACS from the remaining cell population to yield purified (97 %) pDC (**Figure 11A**). Purified pDC (160,000 cells/ml) were incubated with or without ODN 2216 (SEQ ID NO:7) in the presence of IL-3. After 48 hours IFN-α and IFN-β were measured in the supernatant by ELISA. As shown in **Figure 11B**, ODN 2216 stimulated the production of high levels of IFN-α (146 ng/ml; 1 pg per single pDC) and IFN-β (1 ng/ml), as compared to IL-3 alone (< 10 pg/ml).

Within PBMC as well as within pDC-enriched PBMC, 4.2 ± 0.8 pg IFN-α (0.8 to 1.4 U; n=4) was produced per single pDC. IFN-α production of pDC was much lower when pDC were enriched by using magnetically labeled anti-CD4 mAb. This was not due to a loss of IFN-α producing cells in the CD4⁻ fraction, since the CD4⁻ fraction did not produce IFN-α. Adding the CD4⁻ fraction back to CD4⁺ DC did not restore the IFN-α response, thereby excluding a secondary effect of CpG ODN via accessory cells in the CD4⁻ fraction. Thus, crosslinking of CD4 on the surface of pDC appeared to be responsible for the reduced activity.

### Example 10. IFN-α-inducing CpG ODN provide superior indirect activation of NK cells compared to non-IFN-α-inducing CpG ODN.

To examine if CpG ODN which induce high amounts of IFN-α also show higher activation ofNK cells, PBMC were incubated with different CpG ODN. NK cell activation was measured in terms of CD69 expression (FACS) and by in vitro NK-cell lytic activity. For determination of NK-cell lytic activity, PBMC were incubated with different ODN at various concentrations. After 18 hours cells were harvested and used as effector cells in a standard 4 hour ⁵¹Cr-release assay against K562 target cells as previously described. Hartmann G et al. Gene Therapy 6:893 (1999). Positive controls included recombinant IL-2 (100 IU/ml) and negative controls included media alone. Results are expressed as % specific lysis: specific lysis (%) = ((experimental counts - spontaneous release counts) / (maximal release counts - spontaneous release counts)) x 100 %.

*Results.* The IFN-α-inducing ODN 2216 and ODN 1585 increased the percentage of CD69-positive (early marker of activation) NK cells within 24 hours (38 ± 12 % with ODN 2216; n=5) as compared to the control without stimulus (8 ± 2 %; n=5). ODN 2006 showed a lower response (19 % ± 6 %). In agreement with increased CD69 expression, NK cell-mediated lysis of K562 cells was markedly enhanced when PBMC were incubated with CpG ODN. Even at the low concentration of 0.6 µg/ml, ODN 2216 (SEQ ID NO:7) was still as effective as IL-2 (100 IU/ml) to stimulate NK cell lytic activity (**Figure 12**). ODN 1585 (SEQ ID NO:1) and ODN 2006 (SEQ ID NO:147) were less effective. Even at higher concentrations (6 µg/ml) the GC control to ODN 1585 (5' ggGGTCAAGCTTGAgggggG 3'; ODN 2118; SEQ ID NO:151) was completely inactive compared to medium alone (**Figure** 12). When purified NK cells were incubated with CpG ODN, CD69 expression and lysis of K562 cells was not increased, demonstrating an indirect effect of CpG ODN on NK cells.

### Example 11. CpG oligonucleotide induces production of high amounts of IL-8 by IPCs.

IL-8 is a chemokine which attracts other immune cells. IPCs grown in IL-3 produce no IL-8, while CpG oligonucleotide stimulates the production by IPCs of large amounts of IL-8 (mean 23 ng/ml, **Figure 13**).

Freshly prepared IPCs (see Example 1, final concentration 2x10⁵-5x10⁵ cells per ml) were cultured for two days in complete medium supplemented with 10 ng/ml IL-3. One set of parallel cultures was supplemented with 10 µg/ml poly IC, and another set of parallel cultures supplemented with 6 µg/ml CpG ODN 2006 (SEQ ID NO: 147). Supernatants were analyzed for IL-8 using an ELISA specific for human IL-8 (R&D Systems, Minneapolis, MN) according to the supplier's instructions.

*Results.* Representative data from three different donors are shown in **Figure 13**. IL-8 secretion by IPCs was strongly induced by the addition of CpG oligonucleotide to complete media containing IL-3. In contrast, addition of poly IC to the media had no effect. This result demonstrates that CpG oligonucleotide induces IPCs to produce high amounts of IL-8.

### Example 12. Type I IFN induces activation and proliferation of γδ T cells.

The γδ T cells (Vγ9/Vδ2) are antigen-specific T cells in a preactivated stage which respond to common non-peptidic phosphoantigens. Exposure of γδ T cells to these antigens stimulates IFN-γ production in the absence of APC. To examine γδ T cell activation, PBMC (2 x10⁶/ml) from healthy donors were stimulated for three days with 6 µg/ml CpG ODN (2006, 1585, or 2216) or medium alone in the presence or absence of 15 µM isopentenyl pyrophosphate (IPP, specific phosphoantigen for Vγ9/Vδ2 cells). Brefeldin A was added for the last 4 hours. After surface staining for Vγ9 TCR and CD3, cells were fixed, permeabilized and stained with mAb against IFN-γ. In three-color flow cytometry γδ T cells were gated using their FSC/SSC profile, CD3 and TCR Vγ9 expression and analyzed for IFN-γ expression. To compare the results from different donors, data were calculated first as x-fold increase compared to medium or IPP controls and then multiplied with the mean of medium and IPP, respectively. Between 14 and 20 donors were analyzed for each CpG ODN. Data are presented as mean + SEM; * (p < 0,01) and ** (p <0,001) indicate p values calculated by Student's t-test for paired samples comparing medium control to CpG ODN and IPP alone to IPP + CpG ODN.

To examine γδ T cell proliferation, PBMC from healthy donors were stimulated with IPP (30 µM) in the presence or absence of different CpG ODN (2006, 1585, or 2216, each at 6 µg/ml). The expansion of γδ TCR positive cells was assessed by flow cytometry with an anti Vγ9 antibody and is shown as % TCR Vγ9 positive cells within viable PBMC. Between 9 and 16 donors were analyzed for each ODN. Data are presented as x-fold increase compared to IPP alone (mean + SEM); * indicates p < 0,05 (IPP versus IPP + CpG ODN.

*Results.* Within PBMCs both γδ T cells and NK cells but not αβ T cells responded to CpG ODN with increased CD69 expression, IFN-γ and TNF-α production, perforin content and lytic activity. CpG ODN in combination with IPP synergistically induced the production of IFN-γ (**Figure 14**) and perforin in γδ T cells. The synergistic effect was more pronounced for ODN 2216 and 1585, i.e., ODN that are strong inducers of type I IFN, than for ODN 2006. In purified γδ T cells or NK cells, CpG ODN showed no activity or even reduced IPP-stimulated activity.

Furthermore CpG ODN synergistically enhanced the proliferative response of γδ T cells to IPP (**Figure 15**). **Figure 15A** shows the kinetics of γδ T cell expansion from one representative experiment. **Figure 15B** shows the expansion of γδ T cells 10 days after stimulation with IPP alone or in combination with different CpG ODN.

The addition of recombinant IFN-α/β or IL-12 mimicked the stimulatory effects of CpG ODN within PBMC. Functional IL12p70 could not be detected in the supernatants of PBMCs stimulated with CpG ODN. The potential of CpG ODN to activate γδ T-cells and NK cells correlated well with their ability to induce IFN-α/β. The blockade of IFN-α/β function by a combination of neutralizing antibody to IFN-α/β protein and the corresponding receptor inhibited CpG ODN-induced activation of γδ T cells and NK cells. A neutralizing antibody to IL-12 or the addition of IL-18 binding protein reduced baseline IFN-γ but not CpG ODN-stimulated IFN-γ. Neutralizing TNF-α, IL-1β or IL-15 showed no effect. In conclusion the results demonstrated that (i) IFN-α/β is a potent activator of γδ T cells; (ii) CpG ODN activates yδ T cells and NK cells via induction of IFN-α/β; (iii) CpG ODN which are strong inducers of type I IFN are more potent than ODN which are not strong inducers of type I IFN to activate γδ T cells and NK cells; (iv) CpG ODN costimulate antigen-specific T cell responses in γδ T cells; and (v) CpG ODN-induced nonspecific activation of γδ T cells and NK cells provides early IFN-γ which promotes Th1 responses.

### Example 13. Type I IFN-inducing ISNA inhibit IL-12 production.

IFN-β has been described to downregulate IL-12 production. The effect of type I IFN-inducing and non-type I IFN-inducing ISNA on IL-12 production was therefore studied as follows. PBMC (2 x 10⁶/ml) from healthy donors were stimulated with 25 µg/ml of a stimulating anti-CD40 antibody in the presence of IL-4 (100 U/ml), GM-CSF (10 U/ml) and IFN-γ (10 ng/ml). Either medium, 6 µg/ml ODN 2006 (SEQ ID NO:147), 6 µg/ml ODN 1585 (SEQ ID NO:1), or a combination of 5000 U/ml recombinant IFN-α and 500 U/ml IFN-β were added. After 48 hours IL12p70 was measured in the supernatant by ELISA. Data are shown as x-fold of IL 12p70 production by anti-CD40 alone (mean = 143 pg/ml) and represent the mean (+ SEM) of three different donors.

*Results.* **Figure 16** shows that ODN 1585 in combination with anti-CD40 inhibited IL12p70 production compared to anti-CD40 control, to an extent similar to the inhibition by addition of recombinant IFN-α and recombinant IFN-β. In contrast, ODN 2006 in combination with anti-CD40 boosted IL12p70 production beyond anti-CD40 positive control. These results show that, in PBMC, ISNA that induce type I IFN can suppress production of IL-12p70, and conversely ISNA that do not induce type I IFN can boost production of IL-12p70.

Analysis of mRNA levels by quantitative real-time PCR revealed the induction of small but equal copy numbers of IL-12p40 and IL-12p35 mRNA by ISNA that do not induce type I IFN. In contrast, ISNA that induce type I IFN induced a higher number of copies of IL-12 p35 mRNA, but IL-12p40 mRNA could not be detected. ISNA that do not induce type I IFN enhanced (170 %), and ISNA that induce type I IFN blocked (25 %) IL-12p70 synthesis. Inhibition of IL-12p70 could be mimicked by recombinant IFN-β. A combination of neutralizing antibodies to IFN-α/β protein and receptor reversed the type I IFN-inducing ISNA-mediated inhibition of IL-12p70. These results demonstrate that CpG ODN which are strong inducers of type I IFN suppress CD40-dependent IL-12p70 production by an IFN-α/β-mediated negative feedback mechanism on IL-12p40 mRNA production. Thus the interaction of T cells and antigen presenting cells via CD40L leads to a cytokine milieu dominated by IL-12 (ISNA that do not induce type I IFN) or IFN-α/β (ISNA that induce type I IFN). Although both promote Th1 responses, ISNA that do not induce type I IFN may be superior for priming naive T cells, and ISNA that induce type IIFN may have higher activity to support preactivated and memory T cells.

### Example 14. Effect of CpG ODN on primary and recall peptide-specific human CTL responses.

CD8+ T cells (1 x 10⁶) from HLA A2 positive healthy donors were stimulated in 24 well plates in the presence or absence of CpG ODN 2006 (SEQ ID NO:147), 1585 (SEQ ID NO:1), or 2216 (SEQ ID NO:7) at 6 µg/ml with either a HLA A2-restricted peptide derived from the influenza matrix protein (GILGFVFTL) or a peptide derived from the melan A/mart-1 protein (ELAGIGILTV). Autologous PBMC (3 x 10⁶) were used as APCs. After 14 days cells were harvested, washed, and restimulated with influenza matrix or melan-A peptides for 6 hours. Brefeldin A was added for the last 4 hours. Cells were stained for CD8 and CD3, subsequently fixed, permeabilized and stained with mAb against IFN-γ. Also after 14 days the percentage of tetramer-positive CD8⁺ T cells (HLA-A2/melan-A-peptide and HLA-A2/influenza matrix-peptide) was determined by flow cytometry. Tetramers are fluorochrome-labeled MHC-peptide tetramers which are designed to bind specifically to a peptide-specific T cell receptor, making it possible to identify peptide-specific T cells using flow cytometry. Altman JD et al. Science 274:94-96 (1996); U.S. Patent No. 5,635,363.

*Results.* In three-color flow cytometry CD8⁺ T cells (CTL) were analyzed for IFN-γ expression. Results are presented in **Figure 17A** and **17C** as % IFN-γ positive cells of all CD8⁺ T cells. Peptide specificity was tested by stimulating with an irrelevant HLA A2 peptide derived from HIV pol and was < 0.2 % for all samples. Data from 7 donors are presented as mean + SEM. These results show that ODN 2006 increased both primary and recall CTL responses to melan A/mart-1 peptide and to influenza peptide, respectively, in contrast to ODN 1585 and ODN 2216, which induced less recall and had no effect or even inhibited the development of primary CTLs.

Results from the quantification of antigen-specific CTLs using MHC-tetramer staining are shown for influenza peptide and melan A/mart-1 peptide in **Figure 17B** and **17D****,** respectively. Data from 7 donors are presented as mean + SEM; * indicate p values < 0,05 calculated by Student's t-test for paired samples (medium compared to stimulation with CpG ODN).

### Example 15. IFN-α secretion in "high responders"

PBMC from 12 different donors were incubated with varying concentrations of ODN selected from a panel ODN including: ODN 2336 (SEQ ID NO:37), ODN 2334 (SEQ ID NO:36), ODN 2295 (SEQ ID NO:20), ODN 2255 (SEQ ID NO:16), ODN 2247 (SEQ ID NO:11), ODN 2216 (SEQ ID NO:7), and ODN 2006 (SEQ ID NO:147). Results from this study indicated that 6 of the donors could be classified as "high responders" in that the cells of these blood donors secreted more than 500 pg/ml (up to 7000 pg/ml) IFN-α after incubation with the selected ODN. A discrimination between "high" and "low" responders could be made because the cells of the 6 remaining donors only secreted amounts of IFN-α between 10 and 500 pg/ml. One reason for these differing results might come about from using buffy coats that are at least 24h old. pDC, as the main cell type secreting IFN-α, survive only about 3 days in cell culture so that PBMC from buffy coats at least 24 hours old may contain very low numbers of this cell type.

IFN-α was measured in the above experiments by using an ELISA kit that recognizes all subtypes of IFN-α. Most other ELISA kits, in contrast, only measure IFN-α2B. Therefore, the amounts of IFN-α2B were compared against all IFN-α subtypes in several experiments to obtain information about possible differences in induction of different IFN-α subtypes. In addition, the amounts of IFN-α were compared against IFN-γ. Based on the results of this study, there was a correlation between the induction of IFN-α2B and all IFN-α subtypes. In contrast, however, ther was no clear correlation between IFN-α and IFN-γ.

### Example 16. Induction of IFN-α secretion by select CpG ODN

Human PBMCs from a single donor were enriched for DC by going through the first step of the Miltenyi DC isolation kit which depletes monocytes, NK cells, and T cells, leaving mostly B cells, RBCs, and DCs. These were then incubated for two days in the presence of IL-3 (10 ng/ml) and various ODN at 6 µg/ml: ODN 1585 (SEQ ID NO:1), ODN 2022 (SEQ ID NO:2), ODN 2118 (SEQ ID NO:151), ODN 2184 (SEQ ID NO:3), ODN 2185 (SEQ ID NO:4), ODN 2192 (SEQ ID NO:5), ODN 2197 (SEQ ID NO:148), ODN 2198 (SEQ ID NO:149), ODN 2204 (SEQ ID NO:6), ODN 2216 (SEQ ID NO:7), or ODN 2217 (SEQ ID NO:8). In parallel samples, IFN-γ was added at 1000 U/ml. Supernatants were collected and analyzed in an ELISA specific for IFN-α.

*Results.* ODN induced IFN-α to varying degrees, with some augmentation by the addition of IFN-γ. Some of the ODN induced IFN-α to an exceptional degree (>50,000 pg/ml) even in the absence of added IFN-γ.

### Example 17. Donor and sequence dependence of IFN-α response to various ODN.

PBMC taken from four different donors were incubated for two day with a variety of ODN at 0.1 µg/ml. The panel of ODN included the following:

| | | |
|---|---|---|
| ggGGTCAACGTTGAgggggG | ODN 1585 | SEQ ID NO:1 |
| ggggtcgtcgttttgggggg | ODN 2184 | SEQ ID NO:3 |
| tcgtcgttttgtcgttttgggggg | ODN 2185 | SEQ ID NO:4 |
| ggggtcgacgtcgagggggg | ODN 2192 | SEQ ID NO:5 |
| gmGGTCAACGTTGAgggmggG | ODN 2197 | SEQ ID NO:148 |
| ggGGAGTTCGTTGAgggggG | ODN 2198 | SEQ ID NO:149 |
| ggggtcatcgatgagggggg | ODN 2204 | SEQ ID NO:6 |
| ggGGGACGATCGTCgggggG | ODN 2216 | SEQ ID NO:7 |
| gggggtcgtacgacgggggg | ODN 2217 | SEQ ID NO:8 |
| ggggacgtcgacgtgggggg | ODN 2229 | SEQ ID NO:152 |
| ggggtcgttcgaacgagggggg | ODN 2237 | SEQ ID NO:153 |
| ggggacgttcgaacgtgggggg | ODN 2238 | SEQ ID NO:154 |
| ggGGGAGCATGCTGgggggG | ODN 2243 | SEQ ID NO:155 |
| ggGGGACGATATCGTCgggggG | ODN 2245 | SEQ ID NO:9 |
| ggGGGACGACGTCGTCgggggG | ODN 2246 | SEQ ID NO: 10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO: 15 |
| ggGGGACGATCGTCGgggggG | ODN 2255 | SEQ ID NO:16 |
| ggGGGTCATCGATGAgggggG | ODN 2260 | SEQ ID NO:17 |
| ggGGGTCAACGTTGAgggggG | ODN 2261 | SEQ ID NO: 156 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTCgggggG | ODN 2298 | SEQ ID NO:22 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGTCGACGTCGagggg | ODN 2304 | SEQ ID NO:28 |
| ggGGAACGTTAACGTTgggggG | ODN 2305 | SEQ ID NO:29 |
| ggGGACGTCGACGTggggG | ODN 2306 | SEQ ID NO:30 |
| ggGGGTCGTTCGTTgggggG | ODN 2311 | SEQ ID NO:31 |
| ggGGGATGATTGTTgggggG | ODN 2312 | SEQ ID NO:157 |
| ggGGGAZGATZGTTgggggG | ODN 2313 | SEQ ID NO:158 |
| ggGGGAGCTAGCTTgggggG | ODN 2314 | SEQ ID NO:159 |
| ggGACGATCGTCGgggggG | ODN 2328 | SEQ ID NO:32 |
| ggGTCGTCGACGAggggggG | ODN 2329 | SEQ ID NO:33 |
| ggTCGTCGACGAGgggggG | ODN 2330 | SEQ ID NO:34 |
| ggGTCGTCGTCGTGgggggG | ODN 2331 | SEQ ID NO: 160 |
| ggGGACGATCGTCGgggggG | ODN 2332 | SEQ ID NO:35 |
| ggGGACGTCGTCGTgggggG | ODN 2333 | SEQ ID NO:161 |
| ggGGTCGACGTCGACGTCGAGgggggG | ODN 2334 | SEQ ID NO:36 |
| ggGGAACCGCGGTTgggggG | ODN 2335 | SEQ ID NO: 162 |

(Z in ODN 2313 represents 5-methyl cytosine)

Supernatants were collected and assayed for IFN-α by ELISA. In a parallel set of experiments PBMC taken from the same four different donors were incubated for two day with the dame panel of ODN at 1 µg/ml.

*Results.* The results for PBMC derived from the four donors and incubated with ODN at 0.1 µg/ml and with ODN at 1 µg/ml again showed dose and donor variation, with several ODN inducing IFN-α to levels of at least 5000 pg/ml and some inducing IFN-α to levels well in excess of 5000 pg/ml.

## Claims

1. An isolated nucleic acid having a sequence selected from the group consisting of:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, and |
| ggGGACGACGTCGTGgggggG | ODN 2336 | SEQ ID NO:37, |
wherein each lower case letter represents phosphorothioate linkage and each upper case letter indicates phosphodiester linkage.

2. A pharmaceutical composition comprising an isolated nucleic acid having a sequence selected from the group consisting of:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| *ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, and |
| ggGGACGACGTCGTGgggggG | ODN 2336 | SEQ ID NO:37, |
wherein each lower case letter represents phosphorothioate linkage and each upper case letter indicates phosphodiester linkage; and
a pharmaceutically acceptable carrier.

3. A pharmaceutical composition as claimed in claim 2, further comprising interferon alpha (IFN-α).

4. An isolated nucleic acid as claimed in claim 1, or a pharmaceutical composition as claimed in claim 2 or 3, for use in a therapeutic application.

5. An isolated nucleic acid as claimed in claim 1, or a pharmaceutical composition as claimed in claim 2 or 3, for use in the treatment of a proliferative disorder or viral infection which calls for IFN-α treatment.

6. Use of an isolated nucleic acid for the manufacture of a medicament for use in the treatment of a proliferative disorder or viral infection which calls for interferon alpha (IFN-α) treatment, wherein said nucleic acid has a sequence selected from the group consisting of:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, and |
| ggGGACGACGTCGTGgggggG | ODN 2336 | SEQ ID NO:37, |
wherein each lower case letter represents phosphorothioate linkage and each upper case letter indicates phosphodiester linkage.

7. A use as claimed in claim 6, wherein said nucleic acid is to be co-administered with IFN-α.

8. A use as claimed in claim 7, wherein the IFN-α is to be administered:
(a) at a dose below the clinically established effective dose for IFN-α alone;
(b) at the maximum tolerated dose for IFN-α in the absence of the nucleic acid; or
(c) at a dose at least 20, at least 30, at least 40 or at least 50 percent below the maximum tolerated dose of IFN-α.

9. A use as claimed in claim 6 or 7, wherein granulocyte-monocyte colony-stimulating factor (GM-CSF) is to be co-administered to the subject.

10. A use as claimed in claim 6 or 7, wherein the treatment involves activating a subject's interferon-producing cells (IPCs) in a method wherein the IPCs are to be isolated from the subject, cultured *in vitro,* contacted *in vitro* with an effective amount of the nucleic acid, and returned to the subject.

11. A use as claimed in claim 10, wherein the IPCs are contacted in vitro with a growth factor, IL-3 and/or GM-GSF.

12. A use as claimed in claim 6 or 7, wherein the medicament is a pharmaceutical composition comprising the nucleic acid and said composition is to be co-administered to a subject, with a pharmaceutical composition comprising IFN-α, in an amount which, together with the co-administered IFN-α, is an effective IFN-α treatment, wherein the efficacy of the IFN-α treatment is greater than the efficacy of administering the same amount of IFN-α in the absence of co-administering the nucleic acid.

13. A use as claimed in claim 6 or 7, wherein the medicament is a pharmaceutical composition comprising the nucleic acid and said composition is to be co-administered to a subject, with a pharmaceutical composition comprising IFN-α, in an amount which, together with the co-administered IFN-α, is an effective IFN-α treatment, wherein the amount of co-administered IFN-α is less than that which would be required in the absence of co-administering the nucleic acid.

14. A use as claimed in claim 13, wherein the amount of administered IFN-α is at least 20, at least 30, at least 40 or at least 50 percent below the amount of IFN-α required in the absence of co-administering the nucleic acid.

15. A use as claimed in claim 6 or 7, wherein the medicament is a pharmaceutical composition comprising the nucleic acid and said composition is to be co-administered to a subject, with a pharmaceutical composition comprising IFN-α, in an amount which, together with the co-administered IFN-α, is an effective IFN-α treatment, wherein an IFN-α treatment-related side effect is reduced in comparison to the side effect when IFN-α is administered in the absence of co-administering the nucleic acid.

16. A use as claimed in any of claims 12, 13 or 15, wherein the pharmaceutical composition is to be administered locally.

17. A use as claimed in claim 15, wherein the IFN-α treatment-related side effect is systemic, and/or is selected from the group consisting of flu-like syndrome, fever, headache, chills, myalgia, fatigue, anorexia, nausea, vomiting, diarrhea and depression.

18. A use as claimed in claim 6 or 7, wherein the treatment involves enhancing the efficacy of IFN-α treatment in a subject in need of such treatment, and comprises *ex vivo* contacting IPCs, isolated from a donor, with an amount of the nucleic acid effective for inducing the IPCs to release IFN-α; said cells thereafter to be administered to a subject treated with a pharmaceutical composition comprising IFN-α.

19. A use as claimed in claim 18, wherein the donor is the subject and/or the isolated IPCs are further contacted with an antigen.

20. A use as claimed in claim 18, wherein the contacted cells are to be administered by local injection, preferably via a blood vessel supplying a target tissue, more preferably wherein the blood vessel is selected from the group consisting of a hepatic artery, a portal vein, a celiac artery, and a splenic artery.

21. A use as claimed in any one of claims 1-20, wherein said proliferative disorder is selected from the group consisting of hairy cell leukemia, chronic myelogenous leukaemia, cutaneous T-cell leukaemia, multiple myeloma, follicular lymphoma, malignant melanoma, squamous cell carcinoma, AIDS-related Kaposi's sarcoma, renal cell carcinoma, prostate carcinoma, bladder cell carcinoma, cervical dysplasia, and colon carcinoma.

22. A use as claimed in any one of claims 1-20, wherein said viral infection is selected from the group consisting of hepatitis B, hepatitis C, condyloma acuminatum, human immunodeficiency virus, herpes, cytomegalovirus, Epstein-Barr virus, and papillomavirus.

23. A use as claimed in claim 10, wherein the IPCs are to be cultured in the absence of IL-3, and/or in the absence of GM-CSF.

24. A use as claimed in claim 6 or 7, wherein the treatment involves stimulating production of a plurality of type I IFN subtypes, and comprises contacting IPCs *in vivo* with an amount of the nucleic acid effective to induce secretion of at least two type I interferons.

25. A use as claimed in claim 24, wherein the IPCs are precursor type 2 dendritic cells (pDC2s).

26. A use as claimed in claim 24, wherein the IPCs are induced to secrete at least three, at least four, at least five, at least six, at least seven or at least eight type I interferons.

27. A use as claimed in claim 6 or 7, where the treatment involves inhibiting IL-12 production, wherein interferon producing cells are contacted with the nucleic acid in an amount effective for inducing secretion of type I interferon in the presence of IL-12 producing cells, under conditions in which the IL-12 producing cells normally produce IL-12.

28. Natural interferon producing cells (IPCs) prepared by *ex vivo* contacting IPCs isolated from a donor with an amount of a pharmaceutical composition comprising a nucleic acid having a sequence selected from the group consisting of:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, and |
| ggGGACGACGTCGTGgggggG | ODN 2336 | SEQ ID NO:37, |
wherein each lower case letter represents phosphorothioate linkage and each upper case letter indicates phosphodiester linkage, for administration in a therapeutic method for enhancing the efficacy of co-administered IFN-α in the treatment of a proliferative disorder or viral infection.

## Patentansprüche

1. Isolierte Nukleinsäure mit einer Sequenz ausgewählt aus der Gruppe bestehend aus:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTICGAACGTgggggG | ODN 2295 | SEQ ID NO:20 |
| ggGGAACGACGTCGTTgggggG | ODN 2297 | SEQ ID NO:21 |
| ggGGAACGTACGTACGTIgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACGGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGA TCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGggg | ODN 2334 | SEQ ID NO:36, und |
| ggG ggGGACGACGTCGTGgggggG | ODN 2336 | SEQ ID NO:37, |
wobei ein jeder Kleinbuchstabe eine Phosphothioatbindung und ein jeder Großbuchstabe eine Phosphodiesterbindung anzeigt.

2. Pharmazeutische Zusammensetzung umfassend eine isolierte Nukleinsäure mit einer Sequenz ausgewählt aus der Gruppe bestehend aus:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGA TCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, und |
| ggGGACGACGTCGTGgggggG | ODN 2336 | SEQ ID NO:37, |
wobei ein jeder Kleinbuchstabe eine Phosphothioatbindung und ein jeder Großbuchstabe eine Phosphodiesterbindung anzeigt; und
einen pharmazeutisch akzeptablen Träger.

3. Pharmazeutische Zusammensetzung nach Anspruch 2, weiter umfassend Interferon alpha (IFN-α).

4. Isolierte Nukleinsäure nach Anspruch 1 oder eine pharmazeutische Zusammensetzung nach Anspruch 2 oder 3, zur Verwendung in einer therapeutischen Anwendung.

5. Isolierte Nukleinsäure nach Anspruch 1 oder eine pharmazeutische Zusammensetzung nach Anspruch 2 oder 3, zur Verwendung bei der Behandlung einer proliferativen Erkrankung oder viralen Infektion, die eine IFN-α-Behandlung erfordert.

6. Verwendung einer Isolierten Nukleinsäure für die Herstellung eines Medikaments zur Verwendung bei der Behandlung einer proliferativen Erkrankung oder einer viralen Infektion, die eine Interferon alpha (IFN-α)-Behandlung erfordert, wobei die Nukleinsäure eine Sequenz aufweist, die ausgewählt ist aus der Gruppe bestehend aus:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTICGAACGTgggggG | ODN 2295 | SEQ ID NO:20 |
| ggGGAACGACGTCGTTgggggG | ODN 2297 | SEQ ID NO:21 |
| ggGGAACGTACGTACGTIgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACGGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGA TCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGggg | ODN 2334 | SEQ ID NO:36, und |
| ggG ggGGACGACGTCGTGgggggG | ODN 2336 | SEQ ID NO:37, |
wobei ein jeder Kleinbuchstabe eine Phosphothioatbindung und ein jeder Großbuchstabe eine Phosphodiesterbindung anzeigt.

7. Verwendung nach Anspruch 6, wobei die Nukleinsäure zusammen mit IFN-α verabreicht werden soll.

8. Verwendung nach Anspruch 7, wobei das IFN-α verabreicht werden soll:
(a) mit einer Dosis unterhalb der klinisch etablierten wirksamen Dosis für IFN-α allein;
(b) mit einer tolerierten Maximaldosis für IFN-α bei Abwesenheit der Nukleinsäure; oder
(c) bei einer Dosierung von wenigstens 20, wenigstens 30, wenigstens 40 oder wenigstens 50 % unterhalb der tolerierten Maximaldosis von IFN-α.

9. Verwendung nach Anspruch 6 oder 7, wobei dem Lebewesen Granulozyten-Monozyten-Kolonie-stimulierender Faktor (GM-CSF) co-verabreicht werden soll.

10. Verwendung nach Anspruch 6 oder 7, wobei die Behandlung die Aktivierung der Interferon-produzierenden Zellen (IPCs) eines Lebewesens in einem Verfahren umfasst, in dem die IPCs aus dem Lebewesen isoliert, *in vitro kultiviert,* in vitro mit einer wirksamen Menge der Nukleinsäure kontaktiert und in das Lebewesen zurückgeführt werden sollen.

11. Verwendung nach Anspruch 10, wobei die IPCs in vitro mit einem Wachstumsfaktor, IL-3 und/oder GM-CSF kontaktiert werden.

12. Verwendung nach Anspruch 6 oder 7, wobei das Medikament eine pharmazeutische Zusammensetzung ist, die die Nukleinsäure umfasst, und die Zusammensetzung einem Lebewesen zusammen mit einer pharmazeutischen Zusammensetzung, die IFN-α umfasst, in einer Menge verabreicht werden soll, die, zusammen mit dem co-verabreichten IFN-α, eine wirksame IFN-α-Behandlung darstellt, wobei die Wirksamkeit der IFN-α-Behandlung besser ist als die Wirksamkeit der Verabreichung der gleichen Menge an IFN-α bei Abwesenheit der co-verabreichten Nukleinsäure.

13. Verwendung nach Anspruch 6 oder 7, wobei das Medikament eine pharmazeutische Zusammensetzung ist, die die Nukleinsäure umfasst, und die Zusammensetzung einem Lebewesen zusammen mit einer pharmazeutischen Zusammensetzung, die IFN-α umfasst, in einer Menge verabreicht werden soll, die, zusammen mit dem co-verabreichten IFN-α, eine wirksame IFN-α-Behandlung darstellt, wobei die Menge an co-verabreichtem IFN-α geringer ist als diejenige, die bei Abwesenheit der co-verabreichten Nukleinsäure erforderlich wäre.

14. Verwendung nach Anspruch 13, wobei die Menge an verabreichtem IFN-α wenigstens 20, wenigstens 30, wenigstens 40 oder wenigstens 50 % unterhalb der Menge von IFN-α liegt, die bei Abwesenheit der co-verabreichten Nukleinsäure erforderlich ist.

15. Verwendung nach Anspruch 6 oder 7, wobei das Medikament eine pharmazeutische Zusammensetzung ist, die die Nukleinsäure umfasst, und die Zusammensetzung einem Lebewesen zusammen mit einer pharmazeutischen Zusammensetzung, die IFN-α umfasst, In einer Menge verabreicht werden soll, die, zusammen mit dem co-verabreichten IFN-α, eine wirksame IFN-α-Behandlungdarstellt, wobei eine Nebenwirkung, die mit der IFN-α-Behandlung verbunden ist, verglichen mit der Nebenwirkung verringert Ist, wenn IFN-α bei Abwesenheit der co-verabreichten Nukleinsäure verabreicht wird.

16. Verwendung nach einem der Ansprüche 12, 13 oder 15, wobei die pharmazeutische Zusammensetzung lokal verabreicht werden soll.

17. Verwendung nach Anspruch 15, wobei die mit der IFN-α-Behandlung verbundene Nebenwirkung systemisch ist und/oder ausgewählt ist aus der Gruppe bestehend aus erkältungsähnllchem Syndrom, Fieber, Kopfschmerz, Schüttelfrost, Muskelschmerz, Müdigkeit, Anorexie, Übelkeit, Erbrechen, Diarrhö und Depression.

18. Verwendung nach Anspruch 6 oder 7, wobei die Behandlung das Verstärken der Wirksamkeit einer IFN-α-Behandlung in einem Lebewesen umfasst, das einer derartigen Behandlung bedarf, und das ex vivo Kontaktieren von IPCs, die von einem Spender isoliert sind, mit einer Menge der Nukleinsäure umfasst, die wirksam ist, um die IPCs zu induzieren, IFN-α freizusetzen; wobei die Zellen danach einem Lebewesen verabreicht werden sollen, das mit einer pharmazeutischen Zusammensetzung umfassend IFN-α behandelt wird.

19. Verwendung nach Anspruch 18, wobei der Spender das Lebewesen ist und/oder die isolierten IPCs weiter mit einem Antigen kontaktiert werden.

20. Verwendung nach Anspruch 18, wobei die kontaktierten Zellen durch lokale Injektion verabreicht werden sollen, bevorzugterweise durch ein Blutgefäß, das ein Zielgewebe versorgt, wobei bevorzugtererweise das Blutgefäß ausgewählt ist aus der Gruppe bestehend aus einer Leberarterie, einer Pfortader, einer Bauchhöhlenarterie und einer Milzarterie.

21. Verwendung nach einem der Ansprüche 1 - 20, wobei die proliferative Erkrankung ausgewählt ist aus der Gruppe bestehend aus Haarzellleukämie, chronischer myelogenen Leukämie, Haut-T-Zell-Leukämie, multiplem Myelom, follikulärem Lymphom, malignem Melanom, Plattenepithelkarzinom, mit AIDS im Zusammenhang stehendem Kaposi-Sarkom, Hypemephrom, Prostatakarzinom, Blasenzellkarzinom, zervikale Dysplasie und Kolonkarzinom.

22. Verwendung nach einem der Ansprüche 1 - 20, wobei die virale Infektion ausgewählt ist aus der Gruppe bestehend aus Hepatitis B, Hepatitis C, Condyloma acuminatum, menschlichem Immundefizienzvirus, Herpes, Zytomegalievirus, Epstein-Barr-Virus und Papillomavirus.

23. Verwendung nach Anspruch 10, wobei die IPCs in Abwesenheit von IL-3 und/oder in Abwesenheit von GM-CSF kultiviert werden sollen.

24. Verwendung nach Anspruch 6 oder 7, wobei die Behandlung das Stimulieren der Produktion einer Vielzahl von Typ I-IFN-Subtypen und das Kontaktieren von IPCs *In vivo* mit einer Menge der Nukleinsäure umfasst, die wirksam ist, um die Sekretion von wenigstens zwei Typ 1-Interferonen zu induzieren.

25. Verwendung nach Anspruch 24, wobei die IPCs dendritische Zellen vom Precursor-Typ II (pDC2s) sind.

26. Verwendung nach Anspruch 24, wobei die IPCs induziert werden, wenigstens drei, wenigstens vier, wenigstens fünf, wenigstens sechs, wenigstens sieben oder wenigstens acht Typ I-Interferone zu sekretieren.

27. Verwendung nach Anspruch 6 oder 7, wobei die Behandlung die Inhibierung von IL-12-Produktion umfasst, wobei Interferon-produzierende Zellen mit der Nukleinsäure in einer Menge kontaktiert werden, die wirksam ist, um eine Sekretion von Typ I-Interferon in Gegenwart von IL-12-produzierenden Zellen zu induzieren, unter Bedingungen, bei denen IL-12-produzierende Zellen normalerweise IL-12 produzieren.

28. Natürliche Interferon-produzierende Zellen (IPCs), hergestellt durch *ex vivo* Kontaktieren von IPCs, die aus einem Spender isoliert sind, mit einer Menge einer pharmazeutischen Zusammensetzung umfassend eine Nukleinsäure mit einer Sequenz, die ausgewählt ist aus der Gruppe bestehend aus:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTICGAACGTgggggG | ODN 2295 | SEQ ID NO:20 |
| ggGGAACGACGTCGTTgggggG | ODN 2297 | SEQ ID NO:21 |
| ggGGAACGTACGTACGTIgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACGGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGA TCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, und |
| ggGGACGACGTCGTGgggggG | ODN 2336 | SEQ ID NO:37, |
wobei ein jeder Kleinbuchstabe eine Phosphothioatbindung anzeigt und ein jeder Großbuchstabe eine Phosphodiesterbindung anzeigt, für die Verabreichung in einem therapeutischen Verfahren zur Erhöhung der Wirksamkeit von co-verabreichtem IFN-α bei der Behandlung einer proliferativen Erkrankung oder viralen Infektion.

## Revendications

1. Acide nucléique isolé ayant pour séquence une séquence choisie dans le groupe formé par :
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTrCGAACGAgggggG | ODN 2294 | SEQ ID NO:19 |
| ggGGACGTTCGAACGTgggggG | ODN 2295 | SEQ ID NO:20 |
| ggGGAACGACGTCOTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCQrAgggggG | ODN 2301 | SEQ ID NO:25 |
| ggGGACCGGT ACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggCGTCGACGTCGACGTCCACgggggG | ODN 2334 | SEQ ID NO:36, et |
| ggGGACGACGTCGTGgggggG | ODN 2336 | SEQ ID NO:37, |
dans lequel chaque lettre minuscule représente une liaison phosphorothioate et chaque lettre majuscule Indique une liaison phosphodiester.

2. Composition pharmaceutique comprenant un acide nucléique isolé ayant pour séquence une séquence choisie dans le groupe formé par:
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | -ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, et |
| ggGGACGACGTCGTGgggggG | ODN 2336 | SEQ ID NO:37, |
dans lequel chaque lettre minuscule représente une liaison phosphorothioate et chaque lettre majuscule indique une liaison phosphodiester ; et
un support pharmaceutiquement acceptable.

3. Composition pharmaceutique telle que revendiquée dans la revendication 2, comprenant en outre l'interféron alpha (IFN _).

4. Acide nucléique isolé tel que revendiqué dans la revendication 1, ou composition pharmaceutique telle que revendiquée dans la revendication 2 ou 3, destinés à être utilisés dans une application thérapeutique.

5. Acide nucléique isolé tel que revendiqué dans la revendication 1, ou composition pharmaceutique telle que revendiquée dans la revendication 2 ou 3, destinés à être utilisés dans le traitement d'un trouble de la prolifération ou d'une infection virale exigeant un traitement à l'aide d'IFN_.

6. Utilisation d'un acide nucléique isolé pour la fabrication d'un médicament destiné à être utilisé dans le traitement d'un trouble de la prolifération ou d'une infection virale exigeant un traitement à l'aide d'interféron alpha (IFN _), où ledit acide nucléique a pour séquence une séquence choisie dans le groupe formé par :
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACGTACGTACGTTgggggG | ODN 2299 | SEQ 10 NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCCACGTCGAgggggG | ODN 2303 | SEQ ID NO:27 |
| ggGGACGTCGACGTggggG | ODN 2306 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| ggGGTCGACGTCGACGTCGAGgggggG | ODN 2334 | SEQ ID NO:36, et |
| ggGGACGACGTCGTGgggggG | ODN 2336 | SEQ ID NO:37, |
dans lequel chaque lettre minuscule représente une liaison phosphorothioate et chaque lettre majuscule Indique une liaison phosphodiester.

7. Utilisation telle que revendiquée dans la revendication 6, dans laquelle ledit acide nucléique sera co-administré avec de l'IFN_.

8. Utilisation telle que revendiquée dans la revendication 7, dans laquelle l'IFN_ sera administré :
(a) à une dose inférieure à la dose efficace cliniquement établie pour l'IFN_ seul ;
(b) à la dose maximale tolérée pour l'IFN_ en l'absence de l'acide nucléique; ou
(c) à une dose au moins 20, au moins 30, au moins 40 ou au moins 50 pour cent inférieure à la dose maximale tolérée d'IFN_.

9. Utilisation telle que revendiquée dans la revendication 6 ou 7, dans laquelle du facteur de croissance des granulocytes et des monocytes (GM-CSF) sera co-administré au sujet.

10. Utilisation telle que revendiquée dans la revendication 6 ou 7, dans laquelle le traitement implique l'activation des cellules productrices d'interféron (IPC) d'un sujet dans un procédé dans lequel les IPC seront isolées du sujet, cultivées *in vitro,* mises en contact *in vitro* avec une quantité efficace de l'acide nucléique, et rendues au sujet.

11. Utilisation telle que revendiquée dans la revendication 10, dans laquelle les IPC sont mises en contact *in vitro* avec un facteur de croissance, de l'IL-3 et/ou du GM-CSF.

12. Utilisation telle que revendiquée dans la revendication 6 ou 7, dans laquelle le médicament est une composition pharmaceutique comprenant l'acide nucléique et ladite composition sera co-administrée à un sujet, avec une composition pharmaceutique comprenant de l'IFN _, en une quantité qui, conjointement avec l'IFN_ co-administré, constitue un traitement à l'IFN _ efficace, où l'efficacité du traitement à l'IFN _ est supérieure à l'efficacité de l'administration de la même quantité d'IFN_ en l'absence de co-administration de l'acide nucléique.

13. Utilisation telle que revendiquée dans la revendication 6 ou 7, dans laquelle le médicament est une composition pharmaceutique comprenant l'acide nucléique et ladite composition sera co-administrée à un sujet, avec une composition pharmaceutique comprenant de l'IFN _, en une quantité qui, conjointement avec l'IFN _ co-administré, constitue un traitement à l'IFN _ efficace, où la quantité d'IFN _ co-administré est inférieure à celle qui serait nécessaire en l'absence de co-administration de l'acide nucléique.

14. Utilisation telle que revendiquée dans la revendication 13, dans laquelle la quantité d'IFN _ administré est au moins 20, au moins 30, au moins 40 ou au moins 50 pour cent inférieure à la quantité d'IFN _ nécessaire en l'absence de co-administration de l'acide nucléique.

15. Utilisation telle que revendiquée dans la revendication 6 ou 7, dans laquelle le médicament est une composition pharmaceutique comprenant l'acide nucléique et ladite composition sera co-administrée à un sujet, avec une composition pharmaceutique comprenant de l'IFN _, en une quantité qui, conjointement avec l'IFN_ co-administré, constitue un traitement à l'IFN _ efficace, où un effet secondaire lié au traitement à I'IFN _ est réduit par rapport à l'effet secondaire lorsque l'IFN_ est administré en l'absence de co-administration de l'acide nucléique.

16. Utilisation telle que revendiquée dans l'une quelconque des revendications 12,13 ou 15, dans laquelle la composition pharmaceutique sera administrée à niveau local.

17. Utilisation telle que revendiquée dans la revendication 15, dans laquelle l'effet secondaire lié au traitement à l'IFN _ est systémique, et/ou est choisi dans le groupe formé par un syndrome ressemblant à la grippe, une fièvre, une céphalée, des frissons, une myalgie, une fatigue, une anorexie, des nausées, des vomissements, des diarrhées et une dépression.

18. Utilisation telle que revendiquée dans la revendication 6 ou 7, dans laquelle le traitement implique l'amélioration de l'efficacité du traitement à l'IFN_ chez un sujet ayant besoin d'un tel traitement et comprend la mise en contact *ex vivo* d'IPC, isolées d'un donneur, avec une quantité de l'acide nucléique efficace pour stimuler les IPC à libérer de l'IFN _ ; lesdites cellules devant être administrées par la suite à un sujet traité à l'aide d'une composition pharmaceutique comprenant de l'IFN _.

19. Utilisation telle que revendiquée dans la revendication 18, dans laquelle le donneur est le sujet et/ou les IPC isolées sont en outre mises en contact avec un antigène.

20. Utilisation telle que revendiquée dans la revendication 18, où les cellules mises en contact seront administrées par injection locale, de préférence par un vaisseau sanguin irriguant un tissu cible, de préférence encore où le vaisseau sanguin est choisie dans le groupe formé par une artère hépatique, une veine porte, une artère coeliaque, et une artère splénique.

21. Utilisation telle que revendiquée dans l'une quelconque des revendications 1 à 20, où ledit trouble de la prolifération est choisi dans le groupe formé par la leucémie à tricholeucocytes, la leucémie myéloïde chronique, le lymphome à cellules T cutanées, le myélome multiple, le lymphome folliculaire, le mélanome malin, l'épithélioma pavimenteux, le sarcome de Kaposi lié au SIDA, le carcinome des cellules rénales, le cancer de la prostate, le carcinome de cellules vésicales, la dysplasie cervicale, et le carcinome du côlon.

22. Utilisation telle que revendiquée dans l'une quelconque des revendications 1 à 20, où ladite infection virale est choisie dans le groupe formé par l'hépatite B, l'hépatite C, le condylome acuminé, le virus de l'immunodéficience humaine, l'herpès, le cytomégalovirus, le virus d'Epstein-Barr, et le papillomavirus.

23. Utilisation telle que revendiquée dans la revendication 10, dans laquelle les IPC seront cultivées en l'absence d'IL-3, et/ou en l'absence de GM-CSF.

24. Utilisation telle que revendiquée dans la revendication 6 ou 7, dans laquelle le traitement implique la stimulation de la production d'une pluralité de sous-types d'IFN de type I, et comprend la mise en contact d'IPC *in vivo* avec une quantité de l'acide nucléique efficace pour induire la sécrétion d'au moins deux interférons de type I.

25. Utilisation telle que revendiquée dans la revendication 24, dans laquelle les IPC sont des cellules dendritiques de type 2 précurseurs (pDC2).

26. Utilisation telle que revendiquée dans la revendication 24, dans laquelle les IPC sont stimulées à sécréter au moins trois, au moins quatre, au moins cinq, au moins six, au moins sept ou au moins huit interférons de type I.

27. Utilisation telle que revendiquée dans la revendication 6 ou 7, où le traitement implique l'inhibition de la production d'IL-12, dans laquelle des cellules productrices d'interféron sont mises en contact avec l'acide nucléique en une quantité efficace pour l'induction de la sécrétion d'interféron de type I en présence de cellules productrices d'IL-12, dans des conditions dans lesquelles les cellules productrices d'IL-12 produisent normalement de l'IL-12.

28. Cellules productrices d'interféron (IPC) naturelles préparées en mettant des IPC isolées à partir d'un donneur en contact *ex vivo* avec une quantité d'une composition pharmaceutique comprenant un acide nucléique ayant pour séquence une séquence choisie dans le groupe formé par :
| | | |
|---|---|---|
| ggGGGACGATCGTCgggggG | ODN 2216 | SEQ ID NO:7 |
| ggGGGACGATATCGTCgggggG | ODN 2245 | SEQ ID NO:9 |
| ggGGGACGACGTCGTCgggggG | ODN 2246 | SEQ ID NO:10 |
| ggGGGACGAGCTCGTCgggggG | ODN 2247 | SEQ ID NO:11 |
| ggGGGACGTACGTCgggggG | ODN 2248 | SEQ ID NO:12 |
| ggGGGACGATCGTTGggggG | ODN 2252 | SEQ ID NO:13 |
| ggGGAACGATCGTCgggggG | ODN 2253 | SEQ ID NO:14 |
| ggGGGGACGATCGTCgggggG | ODN 2254 | SEQ ID NO:15 |
| ggGGGACGATCGTCGgggggG | ODN 2255 | SEQ ID NO:16 |
| ggGGGTCATCGATGAgggggG | ODN 2260 | SEQ ID NO:17 |
| ggGGTCGTCGACGAgggggG | ODN 2293 | SEQ ID NO:18 |
| ggGGTCGTTCGAACGAgggggG | ODN 2294 | SEQ ID NO:19 |
| ggGGACGTTCGAACGTgggggG | ODN 2295 | SEQ ID NO:20 |
| ggGGAACGACGTCGTTgggggG | ODN 2297 | SEQ ID NO:21 |
| ggGGAACOTACGTACGTTgggggG | ODN 2299 | SEQ ID NO:23 |
| ggGGTCACCGGTGAgggggG | ODN 2300 | SEQ ID NO:24 |
| ggGGTCGACGTACGTCGAgggggG | ODN 2301 | SEQ ID NO:25 |
| ggGGACCGGTACCGGTgggggG | ODN 2302 | SEQ ID NO:26 |
| ggGTCGACGTCGAgggggG | ODN 2303 | SEQ ID NO:27 |
| ggGGACGTCGACGTggggG | ODN 2386 | SEQ ID NO:30 |
| ggGACGATCGTCGgggggG | ODN 2328 | SEQ ID NO:32 |
| ggGTCGTCGACGAggggggG | ODN 2329 | SEQ ID NO:33 |
| ggGGACGATCGTCGgggggG | ODN 2332 | SEQ ID NO:35 |
| gg,GGTCGACGTCGACGTCGACrgggggG | ODN 2334 | SEQ ID NO:36, et |
| ggGGACGACGTCGTGgggggG | ODN 2336 | SEQ ID NO:37, |
dans lequel chaque lettre minuscule représente une liaison phosphorothioate et chaque lettre majuscule indique une liaison phosphodiester, destinées à être administrées dans un procédé thérapeutique visant à améliorer l'efficacité de l'IFN_ co-administré dans le traitement d'un trouble de la prolifération ou d'une infection virale.
